(19) Europäisches Patentamt
European Patent Office
Office européen des brevets

(11) EP 1 888 783 B1

(12) **EUROPEAN PATENT SPECIFICATION**

(45) Date of publication and mention of the grant of the patent:
**26.10.2011 Bulletin 2011/43**

(21) Application number: **06763326.3**

(22) Date of filing: **29.05.2006**

(51) Int Cl.:
***C12Q 1/68*** *(2006.01)*

(86) International application number:
**PCT/EP2006/062667**

(87) International publication number:
**WO 2006/125830 (30.11.2006 Gazette 2006/48)**

(54) **KCNN3 AS DIAGNOSTIC AND THERAPEUTIC TARGET FOR ALZHEIMER'S DISEASE**

KCNN3 ALS DIAGNOSTISCHES UND THERAPEUTISCHES TARGET FÜR ALZHEIMER-KRANKHEIT

KCNN3 SERVANT DE CIBLE DIAGNOSTIQUE ET THERAPEUTIQUE POUR LA MALADIE D'ALZHEIMER

(84) Designated Contracting States:
**AT BE BG CH CY CZ DE DK EE ES FI FR GB GR HU IE IS IT LI LT LU LV MC NL PL PT RO SE SI SK TR**

(30) Priority: **27.05.2005 EP 05104552**
**27.05.2005 US 685072 P**

(43) Date of publication of application:
**20.02.2008 Bulletin 2008/08**

(73) Proprietor: **EVOTEC Neurosciences GmbH**
**22525 Hamburg (DE)**

(72) Inventors:
- **POHLNER, Johannes**
  **22175 Hamburg (DE)**
- **VON DER KAMMER, Heinz**
  **22607 Hamburg (DE)**

(74) Representative: **von Kreisler Selting Werner**
**Deichmannhaus am Dom**
**Bahnhofsvorplatz 1**
**50667 Köln (DE)**

(56) References cited:
**WO-A-99/03889** **WO-A-2004/020665**
**US-A1- 2005 010 967**

- **KOHLER M ET AL: "Small-conductance, calcium-activated potassium channels from mammalian brain [see comments]" SCIENCE, AMERICAN ASSOCIATION FOR THE ADVANCEMENT OF SCIENCE,, US, vol. 273, no. 5282, 20 September 1996 (1996-09-20), pages 1709-1714, XP002091031 ISSN: 0036-8075**
- **SHIEH CHAR-CHANG ET AL: "Potassium channels: Molecular defects, diseases, and therapeutic opportunities" PHARMACOLOGICAL REVIEWS, WILLIAMS AND WILKINS INC., BALTIMORE, MD,, US, vol. 52, no. 4, December 2000 (2000-12), pages 557-593, XP002295931 ISSN: 0031-6997**
- **BLANK THOMAS ET AL: "Small-conductance, Ca2+-activated K+ channel SK3 generates age-related memory and LTP deficits." NATURE NEUROSCIENCE. SEP 2003, vol. 6, no. 9, September 2003 (2003-09), pages 911-912, XP002361761 ISSN: 1097-6256**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 888 783 B1

Printed by Jouve, 75001 PARIS (FR)

## Description

The present invention relates to methods of diagnosing, Alzheimer's disease in a subject. Furthermore, methods of therapy control and screening for modulating agents of Alzheimer's disease are provided.

Neurodegenerative diseases, in particular Alzheimer's disease (AD), have a strongly debilitating impact on a patient's life. Furthermore, these diseases constitute an enormous health, social, and economic burden. AD is the most common neurodegenerative disease, accounting for about 70 % of all dementia cases, and it is probably the most devastating age-related neurodegenerative condition affecting about 10 % of the population over 65 years of age and up to 45 % over age 85 (Vickers et al., Progress in Neurobiology 2000, 60: 139-165; Walsh and Selkoe, Neuron 2004, 44:181-193). Presently, this amounts to an estimated 12 million cases in the US, Europe, and Japan. This situation will inevitably worsen with the demographic increase in the number of old people in developed countries. The neuropathological hallmarks that occur in the brains of individuals with AD are senile plaques, composed of amyloid-β protein, and profound cytoskeletal changes coinciding with the appearance of abnormal filamentous structures and the formation of neurofibrillary tangles.
The amyloid-β protein evolves from the cleavage of the amyloid precursor protein (APP) by different kinds of proteases (Selkoe and Kopan, Annu Rev Neurosci 2003, 26:565-597; Ling et al., Int J Biochem Cell Biol 2003, 35:1505-1535). Two types of plaques, diffuse plaques and neuritic plaques can be detected in the brain of AD patients. They are primarily found in the cerebral cortex and hippocampus. The generation of toxic Aβ deposits in the brain starts very early in the course of AD, and it is discussed to be a key player for the subsequent destructive processes leading to AD pathology. The other pathological hallmarks of AD are neurofibrillary tangles (NFTs) and abnormal neurites, described as neuropil threads (Braak and Braak, J Neural Transm 1998, 53: 127-140). NFTs emerge inside neurons and consist of chemically altered tau, which forms paired helical filaments twisted around each other. Along the formation of NFTs, a loss of neurons can be observed (Johnson and Jenkins, J Alzheimers Dis 1996, 1: 38-58; Johnson and Hartigan, J Alzheimers Dis 1999, 1: 329-351). The appearance of neurofibrillary tangles and their increasing number correlates well with the clinical severity of AD (Schmitt et al., Neurology 2000, 55: 370-376). AD is a progressive disease that is associated with early deficits in memory formation and ultimately leads to the complete erosion of higher cognitive function. The cognitive disturbances include among other things memory impairment, aphasia, agnosia and the loss of executive functioning. A characteristic feature of the pathogenesis of AD is the selective vulnerability of particular brain regions and subpopulations of nerve cells to the degenerative process. Specifically, the inferior temporal lobe region and the hippocampus are affected early and more severely during the progression of the disease. On the other hand, neurons within the frontal cortex, occipital cortex, and the cerebellum remain largely intact and are protected from neurodegeneration (Terry et al., Annals of Neurology 1981, 10: 184-92). Currently, there is no cure for AD, nor is there an effective treatment to halt the progression of AD or even to diagnose AD ante-mortem with high probability. Several risk factors have been identified that predispose an individual to develop AD, among them most prominently the epsilon 4 allele of the three different existing alleles (epsilon 2, 3, and 4) of the apolipoprotein E gene (ApoE) (Strittmatter et al., Proc Natl Acad Sci USA 1993, 90: 1977-81; Roses, Ann NY Acad Sci 1998, 855: 738-43). Although there are rare examples of early-onset AD which have been attributed to genetic defects in the genes for amyloid precursor protein (APP) on chromosome 21, presenilin-1 on chromosome 14, and presenilin-2 on chromosome 1, the prevalent form of late-onset sporadic AD is of hitherto unknown etiologic origin. The late onset and complex pathogenesis of neurodegenerative disorders pose a formidable challenge to the development of therapeutic and diagnostic agents. It is crucial to expand the pool of potential drug targets and diagnostic markers. It is therefore an object of the present disclosure to provide insight into the pathogenesis of neurological diseases and to provide methods, materials, agents, compositions, and animal models which are suited inter alia for the diagnosis and development of a treatment of these diseases. This object has been solved by the features of the independent claims. The subclaims define preferred embodiments of the present invention.

WO-A-2004/020665 discloses a method of diagnosing AD in a subject by determining the foap-13 gene expression level in post-mortem brain tissues and comparing said level to a reference value representing a known disease status and/or representing a known health status and/or representing a known Braak stage and analyzing whether said level is altered compared to a reference value representing a known health status, and/or is similar or equal to a reference value representing a known disease status or representing a known Braak stage.

The present invention is based on the detection of dysregulated, differential expression of a gene coding for a potassium ion channel, a small conductance calcium activated potassium channel, KCNN3, also named SK3, and of the protein products of KCNN3 in human Alzheimer's disease brain samples.
Potassium ion ($K^+$) channels are transmembrane proteins which are responsible for a wide variety of physiological processes, including cell excitability (heart beat, muscle contraction, and neuronal signalling) as well as insulin secretion, cell proliferation, cell volume regulation and others. Small conductance calcium activated potassium channels (SK channels) together with BK- and IK-channels display a special feature among the potassium ion channels in that their activity is regulated by intracellular calcium ions. SK-channels, in particular, play important roles in the afterhyperpolarization that follows an action potential in neurons. As such they are crucial in setting the firing frequency of neurons.

Three SK-channels were cloned in 1996 from rat and human brain (Kohler et al., Science 1996, 273:1709-1714) and termed Sk1-3. A fourth member has been identified later on by several groups (e.g. Ishii et al., Proc. Natl. Acad. Sci. USA 1997, 94:11651).

The SK1-4-channels, also termed KCNN1-4, consist of 6 transmembrane helices and are active mostly as homomeric complexes although the formation of heterotetrameric complexes has also been suggested (Ishii et al. 1997, Proc. Natl. Acad. Sci. USA 94:11651). KCNN3 differs from KCNN1 and KCNN2 in that this channel has an extended N-terminus which harbours two polyglutamine repeats that have been discussed to be involved in bipolar disorders (Chandy et al., Molec. Psychiat. 1998, 3:32-37). However, this finding could not be confirmed by other research groups (e.g. Frebourg et al., Am. J. Hum. Genet. (suppl.) 1998, 63: A326 only; Austin et al., Molec. Psychiat. 1999, 4:261-266; Wittekindt et al., Neurogenetics 1998, 1:259-265).

The coding sequence of KCNN3 consists of 2211 base pairs encoding a protein with 736 amino acids and a calculated molecular weight of 82 kDa. The gene has been mapped to chromosome 1q22 where its 8 exons span approximately 163 kbp (Sun et al., J. Hum. Genet. 2001, 46:463-470). KCNN3 is highly expressed in human and mouse hippocampus (Blank et al., Nature Neurosci. 6: 2003, 911-912; Tacconi et al., Neuroscience 2001, 102:209-215) and its expression has been reported to increase with aging in the hippocampus (Blank et al., Nature Neurosci. 2003, 6:911-912). A precise expression analysis has located KCNN3 to be expressed inter alia in hippocampus dentate gyrus and CA1-4 regions as well as in the entorhinal cortex and also in basal ganglia, thalamus and various brain stem nuclei (Sailer et al., Mol. Cell. Neurosci. 2004, 26:458-469). KCNN3 has been reported to be alternatively spliced giving rise to a dominant negative isoform that suppresses the function of KCNN1-3 (Kolski-Andreaco et al., J. Biol. Chem. 2004, 279:6893-6904).

Additional functions have been ascribed to KCNN3 among them being roles in respiration and parturition (Bond et al., Science 2000, 289:1942-1946) and the regulation of blood pressure (Taylor et al., Circ. Res. 2003, 93:124-131). Interestingly, expression levels of KCNN3 have been linked to synaptic plasticity because an age-related up-regulation of KCNN3 led to some cognitive deficits in mice that could be overcome by the selective down-regulation of KCNN3-mRNA by means of antisense-oligonucleotide treatment (Blank et al., Nature Neurosci. 2003, 6:911-912). Moreover, KCNN3 has been shown to be involved in the modulation of membrane excitability and the determination of firing properties of central neurons (Pedarzani et al., J. Biol. Chem. 2001, 276:9762-9769). Transgenic animals have been established (Bond et al., Science 2000, 289:1942-1946). Here the expression of the KCNN3 gene could be regulated by introduction of a gene switch while retaining normal KCNN3 promoter function. The authors hypothesize that KCNN3 might be a target for e.g. sleep apnea or sudden infant death.

Several blockers of SK-channels have been described: among them being the bee toxin apamin and the scorpion toxin scyllatoxin that block the channels in the low nanomolar range. However, the toxins block all SK-channels rather unspecifically. Small molecular weight compounds have also been described to interfere with SK-channel activity although to a lesser potency compared to the toxins (e.g. tubocurarine, UCL-1684, gallamine).

Brief description of the drawings

Figure 1 discloses the identification of differences in the levels of KCNN3 gene derived mRNA in human brain tissue samples from individuals corresponding to different Braak stages as measured and compared by GeneChip analyses. It indicates that the levels of the respective mRNA species correlate quantitatively with AD progression and thus are indicative for AD as measured by the neuropathological staging of brain tissue samples according to Braak and Braak (Braak staging).

Figure 2 lists the data for the verification of differences in the levels of KCNN3 gene derived mRNA in human brain tissue samples from individuals corresponding to different Braak stages indicative for AD as measured by quantitative RT-PCR analysis.

Figure 3 shows the analysis of absolute levels of KCNN3 gene derived mRNA in human brain tissue samples from individuals corresponding to different Braak stages indicative for AD as measured by quantitative RT-PCR and using statistical method of the median at 98%-confidence level.

Figure 4A discloses SEQ ID NO: 1, the amino acid sequence of the human KCNN3 splice variant 1 protein.

Figure 4B discloses SEQ ID NO: 2, the amino acid sequence of the human KCNN3 splice variant 2 protein.

Figure 4C discloses SEQ ID NO: 3, the amino acid sequence of the human KCNN3 splice variant 3 protein.

Figure 4D discloses SEQ ID NO: 4, the amino acid sequence of the human KCNN3 splice variant 4 protein.

Figure 5A shows SEQ ID NO: 5, the nucleotide sequence of the human KCNN3 splice variant 1 cDNA.

Figure 5B shows SEQ ID NO: 6, the nucleotide sequence of the human KCNN3 splice variant 2 cDNA.

Figure 5C shows SEQ ID NO: 7, the nucleotide sequence of the human KCNN3 splice variant 3 cDNA.

Figure 5D shows SEQ ID NO: 8, the nucleotide sequence of the human KCNN3 splice variant 4 cDNA.

Figure 6A depicts SEQ ID NO: 9, the coding sequence (cds) of the human KCNN3 splice variant 1.

Figure 6B depicts SEQ ID NO: 10, the coding sequence (cds) of the human KCNN3 splice variant 2.
Figure 6C depicts SEQ ID NO: 11, the coding sequence (cds) of the human KCNN3 splice variant 3.
Figure 6D depicts SEQ ID NO: 12, the coding sequence (cds) of the human KCNN3 splice variant 4.
Figure 7 depicts the sequence alignment of the primers used for measuring levels of KCNN3 gene derived mRNA by quantitative RT-PCR with the corresponding clippings of KCNN3 cDNA.
Figure 8 schematically charts the alignment of the KCNN3 cDNA sequence, the coding sequence and both primer sequences used for KCNN3 transcription level profiling.
Figure 9 exemplifies the co-deposition of KCNN3 protein with cortical beta-amyloid plaques in human brain specimens from AD patients. In contrast no such deposition of KCNN3 protein is observed in brain specimens from age-matched controls which have not been diagnosed to suffer from AD signs and symptoms.
Figure 10 exemplifies the co-deposition of KCNN3 protein with cortical beta-amyloid plaques as observed in human brain specimen from AD patients in a magnificated picture.
Figure 11 exemplifies that reactive astrocytes in the cortex of AD patients contain the KCNN3 protein at high levels. In contrast no KCNN3 protein can be found in astrocytes in the cortex of age-matched controls which have not been diagnosed to suffer from AD signs and symptoms.
Figure 12 shows a Western blot analysis of KCNN3 protein production in a CHO cell line stably transfected with a KNN3 expression plasmid.
Figure 13 shows the immunofluorescence based analysis of KCNN3 overproduction and subcellular localization in a stably transfected CHO cell line used for assay development and compound screening.
Figure 14 shows the development of a cellular screening assay for the identification of KCNN3 ion channel modulating compounds.
Figure 15 shows the validation of the cellular KCNN3 screening assay by means of the IC50 determination of the KCNN3 antagonists apamin and trifluorperatine.
Figure 16 shows the Z'-value assessment of the cellular KCNN3 screening assay demonstrating the use of the cellular system for the identification of ion channel modulators for use in AD.

The singular forms "a", "an", and "the" as used herein and in the claims include plural reference unless the context dictates otherwise. For example, "a cell" means as well a plurality of cells, and so forth.
The term "and/or" as used in the present specification and in the claims implies that the phrases before and after this term are to be considered either as alternatives or in combination. For instance, the wording "determination of a level and/or an activity" means that either only a level, or only an activity, or both a level and an activity are determined.
The term "level" as used herein is meant to comprise a gage of, or a measure of the amount of, or a concentration of a transcription product, for instance an mRNA, or a translation product, for instance a protein or polypeptide.
The term "activity" as used herein shall be understood as a measure for the ability of a transcription product or a translation product to produce a biological effect or a measure for a level of biologically active molecules.
The term "activity" also refers to biological activity and/or pharmacological activity which refers to binding, antagonization, repression, blocking, neutralization or sequestration of a potassium channel or potassium channel subunit and which refers to activation, agonization, up-regulation of a potassium channel or potassium channel subunit including but not limited to the novel potassium channel polypeptide of SEQ ID NO: 1. "Biological activity" includes but is not limited to the transmembrane transport of potassium ions and/or transmembrane potassium ion flow and/or the regulation thereof. "Pharmacological activity" includes but is not limited to the ability of a potassium channel or a potassium channel subunit to bind a ligand, a compound, an agent, a modulator and/or another potassium channel subunit.
The terms "level" and/or "activity" as used herein further refer to gene expression levels or gene activity. Gene expression can be defined as the utilization of the information contained in a gene by transcription and translation leading to the production of a gene product.
"Dysregulation" shall mean an up-regulation or down-regulation of gene expression and/or an increase or decrease in the stability of the gene products. A gene product comprises either RNA or protein and is the result of expression of a gene. The amount of a gene product can be used to measure how active a gene is and how stable their gene products are.
The term "gene" as used in the present specification and in the claims comprises both coding regions (exons) as well as non-coding regions (e.g. non-coding regulatory elements such as promoters or enhancers, introns, leader and trailer sequences).
The term "ORF" is an acronym for "open reading frame" and refers to a nucleic acid sequence that does not possess a stop codon in at least one reading frame and therefore can potentially be translated into a sequence of amino acids. "Regulatory elements" shall comprise inducible and non-inducible promoters, enhancers, operators, and other elements that drive and regulate gene expression.
The term "fragment" as used herein is meant to comprise e.g. an alternatively spliced, or truncated, or otherwise cleaved transcription product or translation product.
The term "derivative" as used herein refers to a mutant, or an RNA-edited, or a chemically modified, or otherwise altered transcription product, or to a mutant, or chemically modified, or otherwise altered translation product. For the purpose of clarity, a derivative transcript, for instance, refers to a transcript having alterations in the nucleic acid sequence such as single or multiple nucleotide deletions, insertions, or exchanges. A derivative translation product, for instance, may be generated by processes such as altered phosphorylation, or glycosylation, or acetylation, or lipidation, or by altered signal peptide cleavage or other types of maturation cleavage. These processes may occur post-translationally.

The term "modulator" as used in the present invention and in the claims refers to a molecule capable of changing or altering the level and/or the activity of a gene, or a transcription product of a gene, or a translation product of a gene. A "modulator" refers to a molecule which has the capacity to either enhance or inhibit, thus to "modulate" a functional property of a potassium channel subunit or potassium channel, to "modulate" binding, antagonization, repression, blocking, neutralization or sequestration of a potassium channel or potassium channel subunit and to "modulate" activation, agonization and up-regulation. "Modulation" will be also used to refer to the capacity to affect the biological activity of a cell. Preferably, a "modulator" is capable of changing or altering the biological activity of a transcription product or a translation product of a gene. Said modulation, for instance, may be an increase or a decrease in the biological activity and/or pharmacological activity, a change in binding characteristics, or any other change or alteration in the biological, functional, or immunological properties of said translation product of a gene.

The terms "agent", "reagent", or "compound" refer to any substance, chemical, composition, or extract that have a positive or negative biological effect on a cell, tissue, body fluid, or within the context of any biological system, or any assay system examined. They can be agonists, antagonists, partial agonists or inverse agonists of a target. Such agents, reagents, or compounds may be nucleic acids, natural or synthetic peptides or protein complexes, or fusion proteins. They may also be antibodies, organic or anorganic molecules or compositions, small molecules, drugs and any combinations of any of said agents above. They may be used for testing, for diagnostic or for therapeutic purposes.

The terms "oligonucleotide primer" or "primer" refer to short nucleic acid sequences which can anneal to a given target polynucleotide by hybridization of the complementary base pairs and can be extended by a polymerase. They may be chosen to be specific to a particular sequence or they may be randomly selected, e.g. they will prime all possible sequences in a mix. The length of primers used herein may vary from 10 nucleotides to 80 nucleotides. "Probes" are short nucleic acid sequences of the nucleic acid sequences described and disclosed herein or sequences complementary therewith. They may comprise full length sequences, or fragments, derivatives, isoforms, or variants of a given sequence. The identification of hybridization complexes between a "probe" and an assayed sample allows the detection of the presence of other similar sequences within that sample.

As used herein, "homolog or homology" is a term used in the art to describe the relatedness of a nucleotide or peptide sequence to another nucleotide or peptide sequence, which is determined by the degree of identity and/or similarity between said sequences compared. In the art, the terms "identity" and "similarity" mean the degree of polypeptide or polynucleotide sequence relatedness which are determined by matching a query sequence and other sequences of preferably the same type (nucleic acid or protein sequence) with each other. Preferred computer program methods to calculate and determine "identity" and "similarity" include, but are not limited to GCG BLAST (Basic Local Alignment Search Tool) (Altschul et al., J. Mol. Biol. 1990, 215: 403-410; Altschul et al., Nucleic Acids Res. 1997, 25: 3389-3402; Devereux et al., Nucleic Acids Res. 1984, 12: 387), BLASTN 2.0 (Gish W., http://blast.wustl.edu, 1996-2002), FASTA (Pearson and Lipman, Proc. Natl. Acad. Sci. USA 1988, 85: 2444-2448), and GCG GelMerge which determines and aligns a pair of contigs with the longest overlap (Wilbur and Lipman, SIAM J. Appl. Math. 1984, 44: 557-567; Needleman and Wunsch, J. Mol. Biol. 1970, 48: 443-453).

The term "variant" as used herein refers to any polypeptide or protein, in reference to polypeptides and proteins disclosed in the present invention, in which one or more amino acids are added and/or substituted and/or deleted and/or inserted at the N-terminus, and/or the C-terminus, and/or within the native amino acid sequences of the native polypeptides or proteins of the present invention, but retains its essential properties. Furthermore, the term "variant" shall include any shorter or longer version of a polypeptide or protein. "Variants" shall also comprise a sequence that has at least about 80% sequence identity, more preferably at least about 90% sequence identity, and most preferably at least about 95% sequence identity with the amino acid sequences of KCNN3 protein, in particular SEQ ID NO: 1. "Variants" include, for example, proteins with conservative amino acid substitutions in highly conservative regions. "Proteins and polypeptides" of the present invention include variants, fragments and chemical derivatives of the protein comprising the amino acid sequences of KCNN3 protein, in particular SEQ ID NO: 1. Sequence variations shall be included wherein a codon are replaced with another codon due to alternative base sequences, but the amino acid sequence translated by the DNA sequence remains unchanged. This known in the art phenomenon is called redundancy of the set of codons which translate specific amino acids. Included shall be such exchange of amino acids which would have no effect on functionality, such as arginine for lysine, valine for leucine, asparagine for glutamine. Proteins and polypeptides can be included which can be isolated from nature or be produced by recombinant and/or synthetic means. Native proteins or polypeptides refer to naturally-occurring truncated or secreted forms, naturally occurring variant forms (e.g. splice-variants) and naturally occurring allelic variants. The term "isolated" as used herein is considered to refer to molecules or substances which have been changed and/or that are removed from their natural environment, i.e. isolated from a cell or from a living organism in which they normally occur, and that are separated or essentially purified from the coexisting components with which they are found to be associated in nature. This notion further means that the sequences encoding such molecules can be linked by the hand of man to polynucleotides, to which they are not linked in their natural state and such molecules can be produced by recombinant and/or synthetic means, it is also said that they are "non-native". Even if for said purposes those sequences may be introduced into living or non-living organisms by methods known to those skilled in the art, and even if those sequences are still present in said organisms, they are still considered to be isolated.
In the present invention, the terms "risk", "susceptibility", and "predisposition" are tantamount and are used with respect to the probability of developing a neurodegenerative disease, preferably Alzheimer's disease.
The term "AD" shall mean Alzheimer's disease.
"AD-type neuropathology", "AD pathology" as used herein refers to neuropathological, neurophysiological, histopathological and clinical hallmarks, signs and symptoms as described in the instant invention and as commonly known from state-of-the-art literature (see: Iqbal, Swaab, Winblad and Wisniewski, Alzheimer's Disease and Related Disorders (Etiology, Pathogenesis and Therapeutics), Wiley & Sons, New York, Weinheim, Toronto, 1999; Scinto and Daffner, Early Diagnosis of Alzheimer's Disease, Humana Press, Totowa, New Jersey, 2000; Mayeux and Christen, Epidemiology of Alzheimer's Disease: From Gene to Prevention, Springer Press, Berlin, Heidelberg, New York, 1999; Younkin, Tanzi and Christen, Presenilins and Alzheimer's Disease, Springer Press, Berlin, Heidelberg, New York, 1998).
The term "Braak stage" or "Braak staging" refers to the classification of brains according to the criteria proposed by Braak and Braak (Braak and Braak, Acta Neuropathology 1991, 82: 239-259; Braak and Braak, J Neural Transm 1998, 53: 127-140). Braak staging of AD rates the extent and distribution of neurofibrillary pathology in determined regions of the forebrain and divides the neuropathologic progression of AD into six stages (stage 0 to 6). It is a well established and universally accepted procedure in post-mortem neuropathological staging of AD. It has convincingly been shown that there is a significant correlation between an AD patient's clinical condition with respect to mental status and cognitive function/impairment and the corresponding Braak stage obtained after autopsy (Bancher et al., Neuroscience Letters 1993, 162:179-182; Gold et al., Acta Neuropathol 2000, 99: 579-582). Likewise, a correlation between neurofibrillary changes and neuronal cellular pathology has been found (Rössler et al., Acta Neuropathol 2002, 103:363-369), and both have been reported to predict cognitive function (Giannakopoulos et al., Neurology 2003, 60:1495-1500; Bennett et al., Arch Neurol 2004, 61:378-384). Moreover, a pathogenic cascade has been proposed that involves the deposition of beta-amyloid peptide and finally cumulates in the formation of neurofibrillary tangles, the latter thus witnessing the precedence of earlier AD-specific events at the molecular/cellular level (Metsaars et al., Neurobiol Aging 2003, 24: 563-572).
In the instant invention, Braak stages are therefore used as a surrogate marker of disease progression independent of the clinical presentation/condition of the individual donor, i.e. independent of the presence or absence of reported mental illness, cognitive deficits, decline in other neuropsychiatric parameters, or the overt clinical diagnosis of AD. I.e. it is presumed that the neurofibrillary changes on which the Braak staging is based reflect the underlying molecular and cellular pathomechanisms in general and hence define a (pre-)morbid condition of the brain, meaning that e.g. a donor staged Braak 1 represents by definition an earlier stage of molecular/cellular pathogenesis than a donor staged 2 (or higher), and that therefore a donor of Braak stage 1 can e.g. be regarded as a control individual when compared to donors of any higher Braak stage. In this regard, the differentiation between control individual and affected individual may not necessarily be the same as the clinical diagnosis based differentiation between "healthy control donor" and "AD patient", but it rather refers to a presumed difference in the (pre-) morbid status as deduced from and mirrored by a surrogate marker, the Braak stage.
The values obtained from "controls" are the "reference values" representing a "known health status" and the values obtained from "AD patients" are the "reference values" representing a "known disease status". In the instant invention Braak stage 0 may represent persons which are not considered to suffer from Alzheimer's disease signs and symptoms, and Braak stages 1 to 4 may represent either healthy control persons or AD patients depending on whether said persons are suffering already from clinical signs and symptoms of AD. The higher the Braak stage the more likely is the possibility to display signs and symptoms of AD or the risk to develop signs and symptoms of AD. For a neuropathological assessment, i.e. an estimation of the probability that pathological changes of AD are the underlying cause of dementia, a recommendation is given by Braak H. (www. alzforum.org).
Neurodegenerative diseases or disorders according to the present invention comprise Alzheimer's disease, Parkinson's disease, Huntington's disease, amyotrophic lateral sclerosis, Pick's disease, fronto-temporal dementia, progressive nuclear palsy, corticobasal degeneration, cerebro-vascular dementia, multiple system atrophy, argyrophilic grain dementia and other tauopathies, and mild-cognitive impairment. Further conditions involving neurodegenerative processes are, for instance, ischemic stroke, age-related macular degeneration, narcolepsy, motor neuron diseases, prion diseases, traumatic nerve injury and repair, and multiple sclerosis.

The present invention discloses the identification, the differential expression, the differential regulation, a dysregulation of a gene coding for a potassium channel, a small conductance calcium activated potassium channel, the small conductance calcium activated potassium channel protein 3, alias KCNN3 or K3, also named SK3 or SKCa3, and of the protein products of said gene KCNN3 (alias SK3), in specific samples, in specific brain regions of AD patients, in specific brain regions of persons with different Braak stages, in comparison with each other and/or in comparison to age-matched control persons. The present invention discloses that the gene expression for KCNN3 (SK3) is varied, is dysregulated in brains of AD patients as compared to the respective brain regions of control persons, in that KCNN3 (SK3) mRNA levels are increased, are up-regulated in the inferior temporal cortex and in the frontal cortex of AD patients. Further, the present invention discloses that the KCNN3 (SK3) expression differs in different Braak stages with an increase in expression level starting already at early Braak stages and with a progressive increase with the course of pathological Braak stages predominantly in the inferior temporal cortex.

This dysregulation of KCNN3 (SK3) which parallels the development of AD-type pathology clearly reflects a link between KCNN3 and AD and is indicative for the progressive pathological events in the course of the disease. The differences observed at the KCNN3 (SK3) gene transcriptional level, when compared between AD patients and controls but also between the different Braak stages, are further supported by substantial differences that can be found at the KCNN3 (SK3) protein level. In contrast to the controls, in brain specimens from AD patients the KCNN3 (SK3) protein is contained at high levels in reactive astrocytes, accumulates and co-deposits with cortical beta-amyloid plaques. This dysregulation of the KCNN3 (SK3) gene expression and the changes in levels and localization of the corresponding gene products which parallels the development of AD-type pathology clearly reflects a link between KCNN3 (SK3) and AD and is indicative for the progressive pathological events in the course of the disease. To date, no experiments have been described that demonstrate a relationship between the dysregulation of the KCNN3 (SK3) gene expression and the changes in levels and localization of the corresponding gene products and the pathology of neurodegenerative diseases, in particular AD. Likewise, no mutations in the KCNN3 (SK3) gene have been described to be associated with said diseases. Linking the KCNN3 (SK3) gene to such diseases offers new ways, inter alia, for the diagnosis and treatment of said diseases. Additionally, linking KCNN3 to pathological events occurring already early in the course of AD provides the possibility of a treatment which will prevent the initiation of AD pathology, a treatment which will be applied before non-repairable damages of the brain occur. Consequently, the present invention has utility for diagnostic evaluation, for diagnostic monitoring of persons undergoing a treatment, for prognosis as well as for the identification of a predisposition to a neurodegenerative disease, in particular AD.

The present invention discloses a dysregulation of a gene coding for KCNN3 (SK3) and of its gene products in specific brain regions of AD patients. Neurons within the inferior temporal lobe, the entorhinal cortex, the hippocampus, and the amygdala are subject to degenerative processes in AD (Terry et al., Annals of Neurology 1981, 10:184-192). These brain regions are mostly involved in the processing of learning and memory functions and display a selective vulnerability to neuronal loss and degeneration in AD. In contrast, neurons within the frontal cortex, the occipital cortex, and the cerebellum remain largely intact and preserved from neurodegenerative processes. Brain tissues from the frontal cortex (F) and the inferior temporal cortex (T) of AD patients and of age-matched control individuals were used for the herein disclosed examples. Consequently, the KCNN3 (SK3) gene and its corresponding transcription and/or translation products play a causative role, have an influence on the selective neuronal degeneration and/or neuroprotection.

In one aspect, the invention features a method of diagnosing Alzheimer's disease in a subject, or of monitoring the effect of a treatment administered to a subject having said disease. The method comprises: determining a level, or an activity, or both said level and said activity of (i) a transcription product of the gene coding for KCNN3 proteins having SEQ ID NO:1, SEQ ID NO:2, SEQ ID NO:3 or SEQ ID NO:4, and/or of (ii) a translation product of the gene coding for KCNN3 proteins having SEQ ID NO:1, SEQ ID NO:2, SEQ ID NO:3 or SEQ ID NO:4, and/or of (iii) a variant of said transcription or translation product comprising a sequence that has at least about 80% sequence identity with the amino acid sequences of said KCNN3 proteins in a brain tissue sample obtained from said subject and comparing said level, and/or said activity of said transcription product and/or said translation product to a reference value representing a known disease status (patient), and/or to a reference value representing a known health status (control), and/or to a reference value representing a known Braak stage and analysing whether said level and/or said activity is varied, is altered compared to a reference value representing a known health status, and/or is similar or equal to a reference value representing a known disease status and/or is similar compared to a reference value representing a known Braak stage which is an indication that said subject has Alzheimer's disease, or that said subject is at increased risk of developing said disease. The wording "in a subject" refers to results of the methods disclosed as far as they relate to a disease afflicting a subject, that is to say, said disease being "in" a subject.

Further disclosed herein is a method of monitoring the progression of a neurodegenerative disease in a subject. A level, expression or an activity, or both said level, expression and said activity, of (i) a transcription product of the gene coding for KCNN3 protein, and/or of (ii) a translation product of the gene coding for KCNN3 protein, and/or of (iii) a fragment, or derivative, or variant of said transcription or translation product in a sample obtained from said subject is determined. Said level, expression and/or said activity are compared to a reference value representing a known disease or health status or a known Braak stage. Thereby, the progression of said neurodegenerative disease in said subject is monitored.

Further disclosed is a method of evaluating a treatment of monitoring the effect of a treatment for a neurodegenerative disease, comprising determining a level, expression or an activity, or both said level, expression and said activity of (i) a transcription product of the gene coding for KCNN3 protein, and/or of (ii) a translation product of the gene coding for KCNN3 protein, and/or of (iii) a fragment, or derivative, or variant of said transcription or translation product in a sample obtained from a subject being treated for said disease. Said level, expression or said activity, or both said level and said activity are compared to a reference value representing a known disease or health status or a known Braak stage, thereby evaluating the treatment for said neurodegenerative disease.

In a preferred embodiment, the level, expression or the activity, or both said level and said activity of (i) a transcription product of the gene coding for KCNN3 protein, and/or of (ii) a translation product of the gene coding KCNN3 protein, and/or of (iii) a fragment, or derivative, or variant of said transcription or translation product in a series of samples taken from said subject over a period of time is compared, in order to monitor the progression of said disease. In further preferred embodiments, said subject receives a treatment prior to one or more of said sample gatherings. In yet another preferred embodiment, said level and/or activity is determined before and after said treatment of said subject.

In a preferred embodiment of the herein claimed methods, assays, and uses of the instant invention, said KCNN3 gene and proteins, also referred to as small conductance calcium activated potassium channel protein 3, alias KCNN3 or K3, also named SK3 or SKCa3, is represented by the KCNN3 gene coding in particular for the protein of SEQ ID NO: 1 (Genbank accession number Q9UG16). The amino acid sequence of said protein is deduced from the mRNA sequence corresponding to SEQ ID NO: 5 which corresponds to the cDNA sequence of Genbank accession number AJ251016 (KCNN3, SK3, K3). In the instant invention KCNN3 also refers to the nucleic acid sequence SEQ ID NO: 9 representing the coding sequence (cds) of human KCNN3. In the instant invention said sequences are "isolated" as the term is employed herein. Further, in the instant invention, the gene coding for said KCNN3 protein is also generally referred to as the KCNN3 gene or the SK3 gene, or simply KCNN3 or SK3. The protein of KCNN3 or SK3 is also generally referred to as the KCNN3 protein or SK3 protein.

In the herein claimed methods, assays, and uses of the instant invention, said neurodegenerative disease or disorder is Alzheimer's disease, and said subjects suffer from signs and symptoms of Alzheimer's disease. The sample to be analyzed and determined is brain tissue. Preferably, the methods of diagnosis, or evaluating a treatment for the neurodegenerative disease, according to the instant invention, can be practiced ex corpore, and such methods preferably relate to samples, for instance, body fluids or cells, removed, collected, or isolated from a subject or patient or a control person.

In further preferred embodiments, said reference value is that of a level, of expression, or of an activity, or both of said level and said activity of (i) a transcription product of the gene coding for KCNN3 protein, and/or of (ii) a translation product of the gene coding for KCNN3 protein, and/or of (iii) a fragment, or derivative, or variant of said transcription or translation product in a sample obtained from a subject not suffering from Alzheimer's disease (control sample, control, healthy control person) or in a sample obtained from a subject suffering from Alzheimer's disease (patient sample, patient, AD sample) or from a person with a defined Braak stage which may suffer or may not suffer from signs and symptoms of AD.

In preferred embodiments, an alteration in the level and/or activity and/or expression of a transcription product of the gene coding for KCNN3 protein and/or of a translation product of the gene coding for KCNN3 protein and/or of a fragment, or derivative, or variant thereof in a brain sample cell or tissue, taken from said subject relative to a reference value representing a known health status (control sample) indicates a diagnosis AD.

In a further preferred embodiment, an equal or similar level and/or activity of a transcription product of the gene coding for a KCNN3 protein and/or of a translation product of the gene coding for a KCNN3 protein and/or of a fragment, or derivative, or variant thereof in a sample cell, or tissue, or body fluid obtained from a subject relative to a reference value representing a known disease status of Alzheimer's disease (AD patient sample), indicates a diagnosis, or prognosis, or increased risk of becoming diseased with said neurodegenerative disease.

In another further preferred embodiment, an equal or similar level and/or activity of a transcription product of the gene coding for a KCNN3 protein and/or of a translation product of the gene coding for a KCNN3 protein and/or of a fragment, or derivative, or variant thereof in a brain sample cell or tissue, from a subject relative to a reference value representing a known Braak stage which Braak stage reflects a high risk of developing signs and symptoms of AD, indicates a diagnosis, or prognosis, or an increased risk of becoming diseased with AD.

It is preferred that said varied, altered level, altered expression and/or said altered activity of said transcription product and/or said translation product of KCNN3 and of its fragments, derivatives, or variants, is an increase, an up-regulation.

In preferred embodiments, measurement of the level of transcription products and/or of expression of the gene coding for KCNN3 protein is performed in a sample obtained from a subject using a quantitative PCR-analysis with primer combinations to amplify said gene specific sequences from cDNA obtained by reverse transcription of RNA extracted from a sample of a subject. Primer combinations (SEQ ID NO: 13, SEQ ID NO: 14) are given in Example (vi) of the instant invention, but also other primers generated from the sequences as disclosed in the instant invention can be used. A Northern blot or a ribonuclease protection assay (RPA) with probes specific for said gene can also be applied.

It might further be preferred to measure transcription products by means of chip-based microarray technologies. These techniques are known to those of ordinary skill in the art (see Sambrook and Russell, Molecular Cloning: A Laboratory Manual, Cold Spring Harbor Laboratory Press, Cold Spring Harbor, New York, 2001; Schena M., Microarray Biochip Technology, Eaton Publishing, Natick, MA, 2000). An example of an immunoassay is the detection and measurement of enzyme activity as disclosed and described in the patent application WO 02/14543.

Further disclosed herein is the construction and the use of primers and probes which are unique to the nucleic acid sequences, or fragments, or variants thereof, as disclosed in the present invention. The oligonucleotide primers and/or probes can be labeled specifically with fluorescent, bioluminescent, magnetic, or radioactive substances. The invention further relates to the detection and the production of said nucleic acid sequences, or fragments and variants thereof, using said specific oligonucleotide primers in appropriate combinations. PCR-analysis, a method well known to those skilled in the art, can be performed with said primer combinations to amplify said gene specific nucleic acid sequences from a sample containing nucleic acids. Such sample may be derived either from healthy or diseased subjects or subjects with defined Braak stages. Whether an amplification results in a specific nucleic acid product or not, and whether a fragment of different length can be obtained or not, may be indicative for Alzheimer's disease. Thus, provided are nucleic acid sequences, oligonucleotide primers, and probes of at least 10 bases in length up to the entire coding and gene sequences, useful for the detection of gene mutations and single nucleotide polymorphisms in a given sample comprising nucleic acid sequences to be examined, which may be associated with neurodegenerative diseases, in particular Alzheimer's disease. This feature has utility for developing rapid DNA-based diagnostic tests, preferably also in the format of a kit. Primers for KCNN3 are exemplarily described in Example 1 (vi).

Furthermore, a level and/or an activity and/or expression of a translation product of the gene coding for KCNN3 protein and/or of a fragment, or derivative, or variant of said translation product, and/or the level of activity of said translation product, and/or of a fragment, or derivative, or variant thereof, can be detected using an immunoassay, an activity assay, and/or a binding assay. These assays can measure the amount of binding between said protein molecule and an anti-protein antibody by the use of enzymatic, chromodynamic, radioactive, magnetic, or luminescent labels which are attached to either the anti-protein antibody or a secondary antibody which binds the anti-protein antibody. In addition, other high affinity ligands may be used. Immunoassays which can be used include e.g. ELISAs, Western blots and other techniques known to those of ordinary skill in the art (see Harlow and Lane, Antibodies: A Laboratory Manual, Cold Spring Harbor Laboratory Press, Cold Spring Harbor, New York, 1999 and Edwards R, Immunodiagnostics: A Practical Approach, Oxford University Press, Oxford; England, 1999). All these detection techniques may also be employed in the format of microarrays, protein-arrays, antibody microarrays, tissue microarrays, electronic biochip or protein-chip based technologies (see Schena M., Microarray Biochip Technology, Eaton Publishing, Natick, MA, 2000).

Disclosed is a kit for diagnosing or prognosticating neurodegenerative diseases, in particular AD, in a subject, or determining the propensity or predisposition of a subject to develop a neurodegenerative disease, in particular AD, or of monitoring the effect of a treatment administered to a subject having a neurodegenerative disease, particularly AD, said kit comprising:

(a) at least one reagent which is selected from the group consisting of (i) reagents that selectively detect a transcription product of the gene coding for KCNN3 protein (ii) reagents that selectively detect a translation product of the gene coding for KCNN3 protein; and

(b) instructions for diagnosing, or prognosticating a neurodegenerative disease, in particular AD, or determining the propensity or predisposition of a subject to develop such a disease or of monitoring the effect of a treatment by

- determine a level, or an activity, or both said level and said activity, and/or expression of said transcription product and/or said translation product and/or of fragments, derivatives or variants of the gene coding for KCNN3 protein, in a sample obtained from said subject; and
- comparing said level and/or said activity and/or expression of said transcription product and/or said translation product to a reference value representing a known disease status (patient) and/or to a reference value representing a known health status (control) and/or to a reference value representing a known Braak stage; and
- analysing whether said level and/or said activity and/or expression is varied compared to a reference value representing a known health status, and/or is similar or equal to a reference value representing a known disease status or a reference value representing a known Braak stage; and
- diagnosing or prognosticating a neurodegenerative disease, in particular AD, or determining the propensity or predisposition of said subject to develop such a disease, wherein a varied or altered level, expression or activity, or both said level and said activity, of said transcription product and/or said translation product compared to a reference value representing a known health status (control) and/or wherein a level, expression or activity, or both said level and said activity, of said transcription product and/or said translation product is similar or equal to a reference value representing a known disease status (patient sample), preferably a disease status of AD (AD patient), and/or to a reference value representing a known Braak stage, indicates a diagnosis or prognosis of a neurodegenerative disease, in particular AD, or an increased propensity or predisposition of developing such a disease, a high risk of developing signs and symptoms of AD. The disclosed kit, may be particularly useful for the identification of individuals that are at risk of developing a neurodegenerative disease, in particular AD.

Reagents that selectively detect a transcription product and/or a translation product of the gene coding for KCNN3 protein can be sequences of various length, fragments of sequences, antibodies, aptamers, siRNA, microRNA, and ribozymes. In a further aspect the invention features the use of a kit in a method of diagnosing Alzheimer's disease, in a subject, and in a method of monitoring the effect of a treatment administered to a subject having AD according to the method of claim 1, wherein the kit comprises at least one reagent which is selected from the group consisting of (i) a transcription product of the gene coding for KCNN3 proteins having SEQ ID NO:1, SEQ ID NO:2, SEQ ID NO:3 or SEQ ID NO:4, and/or (ii) a translation product of the gene coding for KCNN3 proteins having SEQ ID NO:1, SEQ ID NO:2, SEQ ID NO:3 or SEQ ID NO:4, and/or (iii) a variant of said transcription or translation product comprising a sequence that has at least about 80% sequence identity with the amino acid sequences of said KCNN3 proteins.
Consequently, the disclosed kit, may serve as a means for targeting identified individuals for early preventive measures or therapeutic intervention prior to disease onset, before irreversible damage in the course of the disease has been inflicted. Furthermore, the kit is useful for monitoring a progression of a neurodegenerative disease, in particular AD in a subject, as well as monitoring success or failure of therapeutic treatment for such a disease of said subject.

Also disclosed is a method of treating or preventing a neurodegenerative disease, in particular AD, in a subject comprising the administration to said subject in need of such a treatment in a therapeutically or prophylactically effective amount and formulation an agent, agents, modulators or selective antagonist, agonists or antibodies which directly or indirectly affect a level, or an activity, or both said level and said activity, of (i) the gene coding for KCNN3 protein, and/or (ii) a transcription product of the gene coding for KCNN3 protein, and/or (iii) a translation product of the gene coding for KCNN3 protein, and/or (iv) a fragment, or derivative, or variant of (i) to (iii). Said agent may comprise a small molecule, or it may also comprise a peptide, an oligopeptide, or a polypeptide. Said peptide, oligopeptide, or polypeptide may comprise an amino acid sequence of a translation product of the gene coding for KCNN3 protein, or a fragment, or derivative, or a variant thereof. An agent for treating or preventing a neurodegenerative disease, in particular AD, may also consist of a nucleotide, an oligonucleotide, or a polynucleotide. Said oligonucleotide or polynucleotide may comprise a nucleotide sequence of the gene coding for KCNN3 protein, either in sense orientation or in antisense orientation.

Preferably the method comprises the application of per se known methods of gene therapy and/or antisense nucleic acid technology to administer said agent or agents. In general, gene therapy includes several approaches: molecular replacement of a mutated gene, addition of a new gene resulting in the synthesis of a therapeutic protein, and modulation of endogenous cellular gene expression by recombinant expression methods or by drugs. Gene-transfer techniques are described in detail (see e.g. Behr, Acc Chem Res 1993, 26: 274-278 and Mulligan, Science 1993, 260: 926-931) and include direct gene-transfer techniques such as mechanical microinjection of DNA into a cell as well as indirect techniques employing biological vectors (like recombinant viruses, especially retroviruses) or model liposomes, or techniques based on transfection with DNA co-precipitation with polycations, cell membrane pertubation by chemical (solvents, detergents, polymers, enzymes) or physical means (mechanic, osmotic, thermic, electric shocks). The postnatal gene transfer into the central nervous system has been described in detail (see e.g. Wolff, Curr Opin Neurobiol 1993, 3: 743-748).

In particular, disclosed is a method of treating or preventing a neurodegenerative disease by means of antisense nucleic acid therapy, i.e. the down-regulation of an inappropriately expressed or defective gene by the introduction of antisense nucleic acids or derivatives thereof into certain critical cells (see e.g. Gillespie, DN&P 1992, 5: 389-395; Agrawal and Akhtar, Trends Biotechnol 1995, 13: 197-199; Crooke, Biotechnology 1992, 10: 882-6). Apart from hybridization strategies, the application of ribozymes, i.e. RNA molecules that act as enzymes, destroying RNA that carries the message of disease has also been described (see e.g. Barinaga, Science 1993, 262: 1512-1514). In preferred embodiments, the subject to be treated is a human, and therapeutic antisense nucleic acids or derivatives thereof are directed against transcription products of the gene coding for KCNN3 protein. It is preferred that cells of the central nervous system, preferably the brain, of a subject are treated in such a way. Cell penetration can be performed by known strategies such as coupling of antisense nucleic acids and derivatives thereof to carrier particles, or the above described techniques. Strategies for administering targeted therapeutic oligodeoxynucleotides are known to those of skill in the art (see e.g. Wickstrom, Trends Biotechnol 1992, 10: 281-287). In some cases, delivery can be performed by mere topical application. Further approaches are directed to intracellular expression of antisense RNA. In this strategy, cells are transformed *ex vivo* with a recombinant gene that directs the synthesis of an RNA that is complementary to a region of target nucleic acid. Therapeutical use of intracellularly expressed antisense RNA is procedurally similar to gene therapy. A recently developed method of regulating the intracellular expression of genes by the use of double-stranded RNA, known variously as RNA interference (RNAi), can be another effective approach for nucleic acid therapy (Hannon, Nature 2002, 418: 244-251).

Preferably, the method comprises grafting donor cells into the central nervous system, preferably the brain, of said subject, or donor cells preferably treated so as to minimize or reduce graft rejection, wherein said donor cells are genetically modified by insertion of at least one transgene encoding said agent or agents. Said transgene might be carried by a viral vector, in particular a retroviral vector. The transgene can be inserted into the donor cells by a nonviral physical transfection of DNA encoding a transgene, in particular by microinjection. Insertion of the transgene can also be performed by electroporation, chemically mediated transfection, in particular calcium phosphate transfection or liposomal mediated transfection (see Mc Celland and Pardee, Expression Genetics: Accelerated and High-Throughput Methods, Eaton Publishing, Natick, MA, 1999).

Preferably, said agent for treating and preventing AD, is a therapeutic protein which can be administered to said subject, preferably a human, by a process comprising introducing subject cells into said subject, said subject cells having been treated *in vitro* to insert a DNA segment encoding said therapeutic protein, said subject cells expressing *in vivo* in said subject a therapeutically effective amount of said therapeutic protein. Said DNA segment can be inserted into said cells *in vitro* by a viral vector, in particular a retroviral vector.

Methods of treatment, comprise the application of therapeutic cloning, transplantation, and stem cell therapy using embryonic stem cells or embryonic germ cells and neuronal adult stem cells, combined with any of the previously described cell- and gene therapeutic methods. Stem cells may be totipotent or pluripotent. They may also be organ-specific. Strategies for repairing diseased and/or damaged brain cells or tissue comprise (i) taking donor cells from an adult tissue. Nuclei of those cells are transplanted into unfertilized egg cells from which the genetic material has been removed. Embryonic stem cells are isolated from the blastocyst stage of the cells which underwent somatic cell nuclear transfer. Use of differentiation factors then leads to a directed development of the stem cells to specialized cell types, preferably neuronal cells (Lanza et al., Nature Medicine 1999, 9: 975-977), or (ii) purifying adult stem cells, isolated from the central nervous system, or from bone marrow (mesenchymal stem cells), for in vitro expansion and subsequent grafting and transplantation, or (iii) directly inducing endogenous neural stem cells to proliferate, migrate, and differentiate into functional neurons (Peterson DA, Curr. Opin. Pharmacol. 2002, 2: 34-42) Adult neural stem cells are of great potential for repairing damaged or diseased brain tissues, as the germinal centers of the adult brain are free of neuronal damage or dysfunction (Colman A, Drug Discovery World 2001, 7: 66-71).

Preferably, the subject for treatment or prevention, can be a human, or a non-human experimental animal, e.g. a mouse or a rat, a domestic animal, or a non-human primate. The experimental animal can be an animal model for a neurodegenerative disorder, e.g. a transgenic mouse and/or a knock-out mouse with an AD-type neuropathology.

In a further preferred embodiment, a method to investigate the effects of compounds and/or agents and/or modulators on a potassium channel formed by KCNN3 subunits or on a heteromeric potassium channel formed by KCNN3 and/or KCNN1 and/or KCNN2 and/or KCNN4 subunits, is provided. Thereby, the electrophysiological effect of compounds and/or agents on the potassium current mediated by KCNN3 subunits expressed alone or co-expressed with KCNN1 and/or KCNN2 and/or KCNN4 potassium channel subunits in appropriate cells, for example CHO-K1 cells, COS-7 cells or HEK293 cells, or in neuronal cell lines, is examined. To conduct said examination the cDNA coding for human gene product KCNN3 is cloned into an appropriate expression-vector. The cDNA coding for KCNN3 and/or KCNN1 and/or KCNN2 and/or KCNN4, is cloned into another appropriate expression-vector. Appropriate cell lines, as mentioned above, are transfected with said plasmids, preferably using a reagent like DMRIE-C (liposome formulation of the cationic lipid 1,2-dimyristyloxypropyl-3-dimethyl-hydroxy ethyl ammonium bromide-cholesterol). Patch-clamp experiments can be performed in the voltage-clamp mode (Hamill et al., Pflügers Arch. 1981, 391: 85-100), and whole-cell currents will be recorded, and the obtained signals will be amplified, digitized, stored and analyzed using an appropriate software, for example Pulse/Pulsefit (HEKA, Lambrecht, Germany). If current "run-down" or "run-up" (Varnum et al., Pro. Natl. Acad. Sci. USA 1993, 90: 11528-11532) remains to be too strong for compound and/or agent effect evaluation, investigations on the mediated currents of said potassium channels can be performed with the perforated patch-clamp method to prevent unspecific current amplitude changes (Dart et al., J. Physiol. 1995, 483: 29-39; Dinesh & Hablitz, Brain Res. 1990, 535: 318-322). An example of stimulation protocol for the investigation of the effects and reversibility of test compounds on KCNN3 alone or co-expressed with KCNN1 and/or KCNN2 and/or KCNN4, is given below. Cells will be clamped at a holding potential of e.g. -60 mV. The pulse cycling rate may be 15 sec. For the compound and/or agent testing, stably transfected cells can be hyperpolarized from a holding potential of e.g. -50 mV for e.g. 100 msec or 250 msec or 500 msec to e.g. -160 mV in -10 or -20 mV increments, followed by, for instance, a 1s depolarization to +90 mV. The current amplitude at the end of the test pulse to +90 mV will be used for the analysis. The method is also suitable to identify and test compounds and/or agents which are capable for opening, closing, activating, inactivating, or modifying the biophysical properties of KCNN3 alone or co-expressed with KCNN1 and/or KCNN2 and/or KCNN4. The cell lines can be used as well in high-throughput screening techniques (Netzer et al., Curr Opin Drug Discov Devel 2003, 4: 462-469). Modulators of potassium channels, in particular of small conductance calcium activated potassium channels, thus identified and tested, can potentially influence learning and memory functions and can be used for therapeutic approaches, for example for neurodegenerative diseases and Alzheimer's disease.

Further disclosed is an agent, a selective antagonist or agonist or a modulator of an activity, or a level, or both said activity and said level, and/or of expression of at least one substance which is selected from the group consisting of (i) the gene coding for KCNN3 protein, and/or (ii) a transcription product of the gene coding for KCNN3 protein, and/or (iii) a translation product of the gene coding for KCNN3 protein, and/or (iv) a fragment, or derivative, or variant of (i) to (iii), and said agent, selective antagonist or agonist, or said modulator has a potential activity in the treatment of neurodegenerative diseases, in particular AD.

Also disclosed is the use of an agent, an antibody, a selective antagonist or agonist, or a modulator of an activity, or a level, or both said activity and said level, and/or of expression of at least one substance which is selected from the group consisting of (i) the gene coding for KCNN3 protein, and/or (ii) a transcription product of the gene coding for KCNN3 protein, and/or (iii) a translation product of the gene coding for KCNN3 protein, and/or (iv) a fragment, or derivative, or variant of (i) to (iii) in the manufacture of a medicament for treating or preventing a neurodegenerative disease, in particular AD. Said antibody may be specifically immunoreactive with an immunogen which is a translation product of a gene coding for KCNN3 having in particular SEQ ID NO: 1 or a fragment, or a derivative, or variant thereof.

Additionally disclosed is a pharmaceutical composition comprising said agent, antibody, selective antagonist or agonist, or modulator and preferably a pharmaceutical carrier. Said carrier refers to a diluent, adjuvant, excipient, or vehicle with which the modulator is administered.

Further disclosed is a kit comprising one or more containers filled with a therapeutically or prophylactically effective amount of said pharmaceutical composition.

Further disclosed is a recombinant, genetically modified non-human animal comprising a non-native KCNN3 gene sequence coding for a KCNN3 protein having in particular SEQ ID NO: 1, or a fragment, or a derivative, or variant thereof under the control of a transcriptional element which is not the native KCNN3 gene transcriptional control element. The generation of said recombinant, non-human animal comprises (i) providing a gene targeting construct containing said gene sequence and a selectable marker sequence, and (ii) introducing said targeting construct into a stem cell of a non-human animal, and (iii) introducing said non-human animal stem cell into a non-human embryo, and (iv) transplanting said embryo into a pseudopregnant non-human animal, and (v) allowing said embryo to develop to term, and (vi) identifying a genetically altered non-human animal whose genome comprises a modification of said gene sequence in both alleles, and (vii) breeding the genetically altered non-human animal of step (vi) to obtain a genetically altered non-human animal whose genome comprises a modification of said gene sequence, wherein the expression of said gene, a mis-expression, under-expression, non-expression or over-expression, and wherein the disruption or alteration of said gene sequence results in said non-human animal exhibiting a predisposition to developing signs and symptoms of a neurodegenerative disease, in particular AD. Strategies and techniques for the generation and construction of such an animal are known to those of ordinary skill in the art (see e.g. Capecchi, Science 1989, 244: 1288-1292 and Hogan et al., Manipulating the Mouse Embryo: A Laboratory Manual, Cold Spring Harbor Laboratory Press, Cold Spring Harbor, New York, 1994 and Jackson and Abbott, Mouse Genetics and Transgenics: A Practical Approach*,*

Oxford University Press, Oxford, England, 1999).

It is preferred to make use of such a genetically modified, recombinant non-human animal as an animal model, as test animal or as a control animal for investigating neurodegenerative diseases, in particular Alzheimer's disease. Such an animal may be useful for screening, testing and validating compounds, agents and modulators in the development of diagnostics and therapeutics to treat neurodegenerative diseases, in particular Alzheimer's disease. The use of such a genetically modified animal in a screening method is disclosed in the instant invention.

Disclosed is the use of a cell, in which a gene sequence coding for a KCNN3 protein having in particular SEQ ID NO: 1, or a fragment, or derivative, or variant thereof is mis-expressed, under-expressed, non-expressed or over-expressed, or disrupted or in another way alterated for screening, testing and validating compounds, agents and modulators in the development of diagnostics and therapeutics to treat Alzheimer's disease. The use of such a cell in a screening method is disclosed in the instant invention.

In another aspect, the invention features a method of screening for identifying an agent, a modulator, a selective antagonist or agonist for use in the treatment of AD, which agents, modulators or selective antagonists or agonists have an ability to alter expression and/or level and/or activity of one or more substances selected from the group consisting of (i) the gene coding for KCNN3 protein having SEQ ID NO:1, SEQ ID NO:2, SEQ ID NO:3 or SEQ ID NO:4, and/or (ii) a transcription product of the gene coding for KCNN3 protein having SEQ ID NO:1, SEQ ID NO:2, SEQ ID NO:3 or SEQ ID NO:4, and/or (iii) a translation product of the gene coding for KCNN3 protein having SEQ ID NO:1, SEQ ID NO:2, SEQ ID NO:3 or SEQ ID NO:4 and/or (iv) a variant of (i) to (iii) comprising a sequence that has at least about 80% sequence identity with the amino acid sequences of said KCNN3 proteins. This screening method comprises (a) contacting a cell with a test compound, and (b) measuring the activity and/or the level, and/or the expression of one or more substances recited in (i) to (iv), and (c) measuring the activity and/or the level, and/or the expression of said substances in a control cell not contacted with said test compound, and (d) comparing the levels and/or activities and/or the expression of the substance in the cells of step (b) and (c), wherein an alteration in the activity and/or level and/or expression of said substances in the contacted cells indicates that the test compound is an agent, modulator, selective antagonist or agonist for use in the treatment of AD. Said cells may be cells as disclosed herein.

In one further aspect, the invention features a method of screening for identifying an agent, a modulator, a selective antagonist or agonist for use in the treatment of AD, which agents, modulators or selective antagonists or agonists have an ability to alter expression and/or level and/or activity of one or more substances selected from the group consisting of (i) the gene coding for KCNN3 protein having SEQ ID NO:1, SEQ ID NO:2, SEQ ID NO:3 or SEQ ID NO:4, and/or (ii) a transcription product of the gene coding for KCNN3 protein having SEQ ID NO:1, SEQ ID NO:2, SEQ ID NO:3 or SEQ ID NO:4, and/or (iii) a translation product of the gene coding for KCNN3 protein having SEQ ID NO:1, SEQ ID NO:2, SEQ ID NO:3 or SEQ ID NO:4 and/or (iv) a variant of (i) to (iii) comprising a sequence that has at least about 80% sequence identity with the amino acid sequences of said KCNN3 proteins, comprising (a) administering a test compound to a non-human test animal which is predisposed to developing or has already developed signs and symptoms of AD, said animal may be an animal model as disclosed in the instant invention, and (b) measuring the activity and/or level and/or expression of one or more substances recited in (i) to (iv), and (c) measuring the activity and/or level and/or expression of said substances in a non-human control animal which is predisposed to developing or has already developed said signs and symptoms of AD, and to which non-human animal no such test compound has been administered, and (d) comparing the activity and/or level and/or expression of the substances in the animals of step (b) and (c), wherein an alteration in the activity and/or level and/or expression of substances in the non-human test animal indicates that the test compound is an agent, modulator, selective antagonist or agonist for use in the treatment of AD.

Further disclosed herein is a method for producing a medicament comprising the steps of (i) identifying an agent, modulator, selective antagonists or agonists of neurodegenerative diseases by a method of the aforementioned screening assays and (ii) admixing said agent, modulator, selective antagonist or agonist with a pharmaceutical carrier. However, said agent, modulator, selective antagonist or agonist may also be identifiable by other types of screening methods and assays.

Disclosed is an assay for testing a compound or compounds, preferably for screening a plurality of compounds in high-throughput format, to determine the degree of inhibition of binding or the enhancement of binding between a ligand and a KCNN3 protein having in particular SEQ ID NO:1, or a fragment, or derivative, or variant thereof and/or to determine the degree of binding of said compounds to a KCNN3 protein having in particular SEQ ID NO:1, or a fragment, or derivative, or variant thereof. For determination of inhibition of binding between a ligand and KCNN3 protein, or a fragment, or derivative, or variant thereof, said screening assay comprises the steps of (i) adding a liquid suspension of said KCNN3 protein, or a fragment, or derivative, or variant thereof, to a plurality of containers, and (ii) adding a compound or a plurality of compounds to be screened for said inhibition to said plurality of containers, and (iii) adding a detectable, preferably a fluorescently labelled ligand to said containers, and (iv) incubating said KCNN3 protein, or said fragment, or derivative or variant thereof, and said compound or plurality of compounds, and said detectable, preferably fluorescently labelled ligand, and (v) measuring the amounts of preferably the fluorescence associated with said KCNN3 protein, or with said fragment, or derivative, or variant thereof, and (vi) determining the degree of inhibition by one or more of said compounds of binding of said ligand to said KCNN3 protein, or said fragment, or derivative, or variant thereof. It might be preferred to reconstitute said KCNN3 translation product, or fragment, or derivative, or variant thereof into artificial liposomes to generate the corresponding proteoliposomes to determine the inhibition of binding between a ligand and said KCNN3 translation product. Methods of reconstitution of KCNN3 translation products from detergent into liposomes have been detailed (Schwarz et al., Biochemistry 1999, 38: 9456-9464; Krivosheev and Usanov, Biochemistry-Moscow 1997, 62: 1064-1073). Instead of utilizing a fluorescently labelled ligand, it might in some aspects be preferred to use any other detectable label known to the person skilled in the art, e.g. radioactive labels, and detect it accordingly. Said method may be useful for the identification of novel compounds as well as for evaluating compounds which have been improved or otherwise optimized in their ability to inhibit the binding of a ligand to a gene product of the gene coding for KCNN3 protein, or a fragment, or derivative, or variant thereof. One example of a fluorescent binding assay, in this case based on the use of carrier particles, is disclosed and described in patent application WO00/52451. A further example is the competitive assay method as described in patent WO02/01226. Preferred signal detection methods for screening assays of the instant invention are described in the following patent applications: WO96/13744, WO98/16814, WO98/23942, WO99/17086, WO99/34195, WO00/66985, WO01/59436, WO01/59416.

Further disclosed herein is a method for producing a medicament comprising the steps of (i) identifying a compound as an inhibitor of binding between a ligand and a gene product of the gene coding for KCNN3 protein by the aforementioned inhibitory binding assay and (ii) admixing the compound with a pharmaceutical carrier. However, said compound may also be e tifia le by other types of screening assays.

An assay is disclosed for testing a compound or compounds, preferably for screening a plurality of compounds in high-throughput format to determine the degree of binding of said compounds to KCNN3 protein having in particular SEQ ID NO: 1, or to a fragment, or derivative, or variant thereof, said screening assay comprises (i) adding a liquid suspension of said KCNN3 protein, or a fragment, or derivative, or variant thereof, to a plurality of containers, and (ii) adding a detectable, preferably a fluorescently labelled compound or a plurality of detectable, preferably fluorescently labelled compounds to be screened for said binding to said plurality of containers, and (iii) incubating said KCNN3 protein, or said fragment, or derivative, or variant thereof, and said detectable, preferably fluorescently labelled compound or detectable, preferably fluorescently labelled compounds, and (iv) measuring the amounts of preferably the fluorescence associated with said KCNN3 protein, or with said fragment, or derivative, or variant thereof, and (v) determining the degree of binding by one or more of said compounds to said KCNN3 protein, or said fragment, or derivative, or variant thereof. In this type of assay it might be preferred to use a fluorescent label. However, any other type of detectable label might also be employed. Also in this type of assay it might be preferred to reconstitute a KCNN3 translation product or a fragment, or derivative, or variant thereof into artificial liposomes as described in the present invention. Said assay methods may be useful for the identification of novel compounds as well as for evaluating compounds which have been improved or otherwise optimized in their ability to bind to KCNN3 protein, or a fragment, or derivative, or variant thereof. Further disclosed is a method for producing a medicament comprising the steps of (i) identifying a compound as a binder to a gene product of the gene coding for KCNN3 protein by the aforementioned binding assays and (ii) admixing the compound with a pharmaceutical carrier. However, said compound may also be identifiable by other types of screening assays.

Further disclosed is the provision of a medicament obtainable by any of the methods according to the herein claimed screening assays and a medicament obtained by any of the methods according to the herein claimed screening assays.

Also disclosed are protein molecules and the use of said protein molecules having in particular SEQ ID NO: 1, said protein molecules being translation products of the gene coding for KCNN3, or fragments, or derivatives, or variants thereof, as diagnostic targets for detecting a neurodegenerative disease, in particular Alzheimer's disease.

The present invention further discloses protein molecules and the use of said protein molecules having in particular SEQ ID NO: 1, said protein molecules being translation products of the gene coding for KCNN3, or fragments, or derivatives, or variants thereof, as screening targets for agents, modulators, selective antagonists, agonists, reagents or compounds preventing, or treating, or ameliorating a neurodegenerative disease, in particular Alzheimer's disease.

The present invention features the use of antibodies which are specifically immunoreactive with an immunogen, wherein said immunogen is a translation product of the gene KCNN3 coding for KCNN3 protein having SEQ ID NO:1, SEQ ID NO:2, SEQ ID NO:3 or SEQ ID NO:4, or variants thereof comprising a sequence that has at least about 80% sequence identity with the amino acid sequences of said KCNN3 proteins for detecting the pathological state of a cell in a brain tissue sample obtained from a subject, comprising immunocytochemical staining of said cell with said antibody, wherein an altered degree of staining, or an altered staining pattern in: said cell compared to a cell representing a known health status indicates a pathological state of said cell which is Alzheimer's disease. The immunogen may comprise immunogenic or antigenic epitopes or portions of a translation product of said gene, wherein said immunogenic or antigenic portion of a translation product is a polypeptide, and wherein said polypeptide elicits an antibody response in an animal, and wherein said polypeptide is immunospecifically bound by said antibody. Methods for generating antibodies are well known in the art (see Harlow et al., Antibodies, A Laboratory Manual, Cold Spring Harbor Laboratory Press, Cold Spring Harbor, New York, 1988). The term "antibody", as employed in the present invention, encompasses all forms of antibodies known in the art, such as polyclonal, monoclonal, chimeric, recombinatorial, anti-idiotypic, humanized, or single chain antibodies, as well as fragments thereof (see Dubel and Breitling, Recombinant Antibodies, Wiley-Liss, New York, NY, 1999). Antibodies of the present invention are useful, for instance, in a variety of diagnostic and therapeutic methods, based on state-in-the-art techniques (see Harlow and Lane, Using Antibodies: A Laboratory Manual, Cold Spring Harbor Laboratory Press, Cold Spring Harbor, New York, 1999 and Edwards R., Immunodiagnostics: A Practical Approach, Oxford University Press, Oxford, England, 1999) such as enzyme-immuno assays (e.g. enzyme-linked immunosorbent assay, ELISA), radioimmuno assays, chemoluminescence-immuno assays, Western-blot, immunoprecipitation and antibody microarrays. These methods involve the detection of translation products of the KCNN3 gene, or fragments, or derivatives, or variants thereof. Immunocytochemical staining of a cell can be carried out by a number of different experimental methods well known in the art. It might be preferred, however, to apply an automated method for the detection of antibody binding, wherein the determination of the degree of staining of a cell, or the determination of the cellular or subcellular staining pattern of a cell, or the topological distribution of an antigen on the cell surface or among organelles and other subcellular structures within the cell, are carried out according to the method described in US6150173.

Other features and advantages of the invention will be apparent from the following description of figures and examples which are illustrative only and not intended to limit the remainder of the disclosure in any way.

FIGURES:

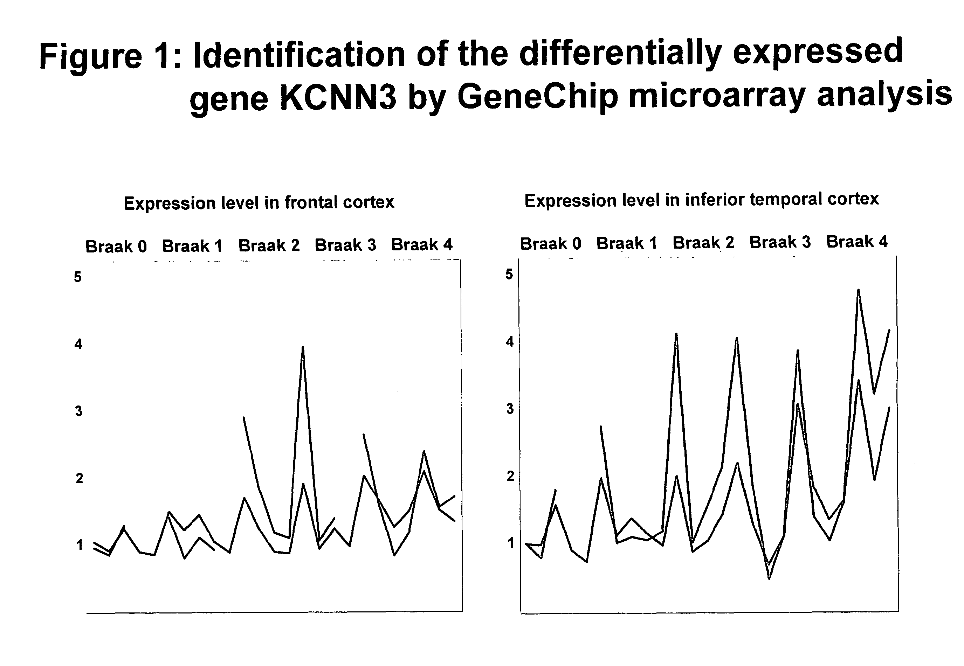

Figure 1 shows the identification of differences in the levels of KCNN3 gene derived mRNA in human brain tissue samples from individuals corresponding to different Braak stages as measured and compared by GeneChip analyses.

It indicates that the levels of the respective mRNA species correlate quantitatively with AD progression and thus are indicative for AD as measured by the neuropathological staging of brain tissue samples according to Braak and Braak (Braak staging). cRNA probes of frontal cortex as well as of inferior temporal cortex each of 5 different donors with Braak stage 0 (C011, C012, C026, C027, and C032), 7 different donors with Braak stage 1 (C014, C028, C029, C030, C036, C038, and C039), 5 different donors with Braak stage 2 (C008, C031, C033, C034, and DE03), 4 different donors with Braak stage 3 (C025, DE07, DE11, and C057), and 4 different donors with Braak stage 4 (P012, P046, P047, and P068) have been applied to an analysis of an Affymetrix Human Genome U133 Plus 2.0 Array respectively. Obvious differences reflecting an up-regulation of the KCNN3 gene progressively with Braak stages predominantly in inferior temporal tissue are shown.

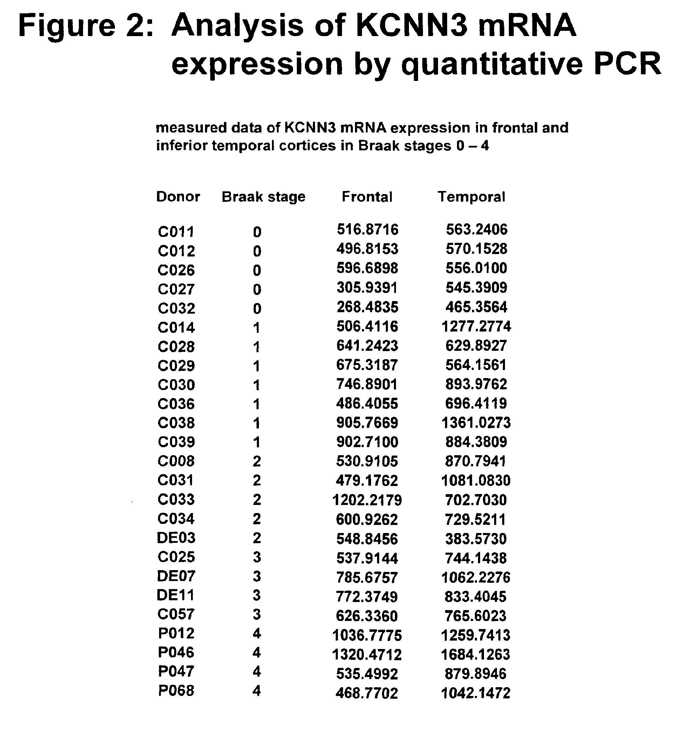

Figure 2 lists the data for the verification of differences in the levels of KCNN3 gene derived mRNA in human brain tissue samples from individuals corresponding to different Braak stages indicative for AD as measured by quantitative RT-PCR analysis. Quantitative RT-PCR using the Roche Lightcycler rapid thermal cycling technique was performed applying cDNA of the frontal cortex (Frontal) and the inferior temporal cortex (Temporal) of the same donors as used for GeneChip analysis. The data were normalized to values of cyclophilin B, a standard gene that showed no significant differences in its gene expression levels. The comparison between samples of the lowest Braak stage 0 with samples representing high Braak stage 4 clearly demonstrates a substantial difference in gene expression level of KCNN3.

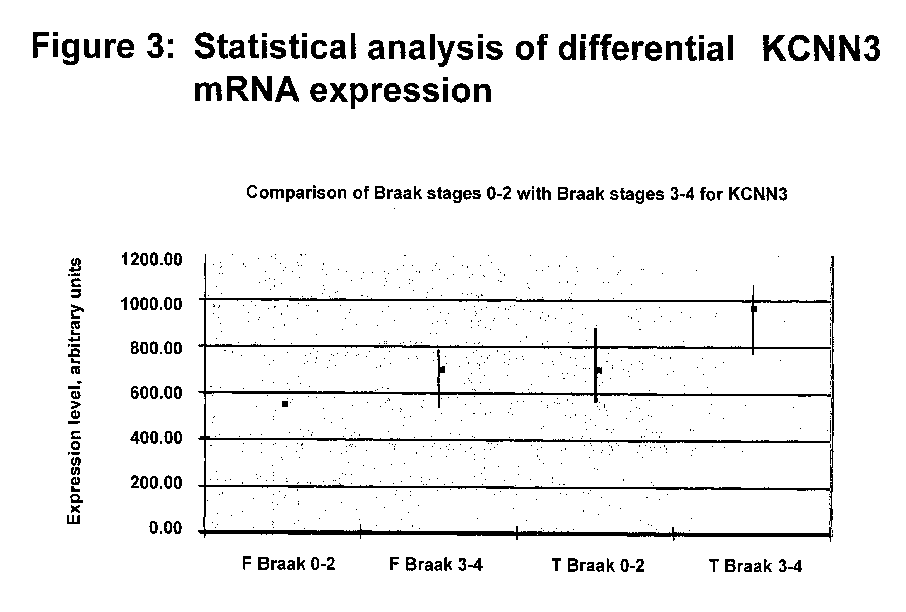

Figure 3 shows the analysis of absolute levels of KCNN3 gene derived mRNA in human brain tissue samples from individuals corresponding to different Braak stages indicative for AD as measured by quantitative RT-PCR and using statistical method of the median at 98%-confidence level (Sachs L (1988) Statistische Methoden: Planung und Auswertung. Heidelberg New York, p. 60). The data were calculated by defining control groups including subjects with Braak stages 0 to 2, which are compared with the data calculated for the defined groups with advanced AD pathology including Braak stages 3 to 4. An obvious difference reflecting an up-regulation is shown in frontal as well as in inferior temporal cortices corroborating results from the GeneChip analysis. Most prominent difference is obvious comparing inferior temporal cortex (T) of Braak stage 0-2 with Braak stage 3-4. Said difference reflects an up-regulation of KCNN3 in the temporal cortex and in the frontal cortex of individuals with advanced AD pathology relative to the inferior temporal cortex and the frontal cortex of control persons, and an up-regulation of KCNN3 in the inferior temporal cortex of individuals with advanced AD pathology compared to their frontal cortices.

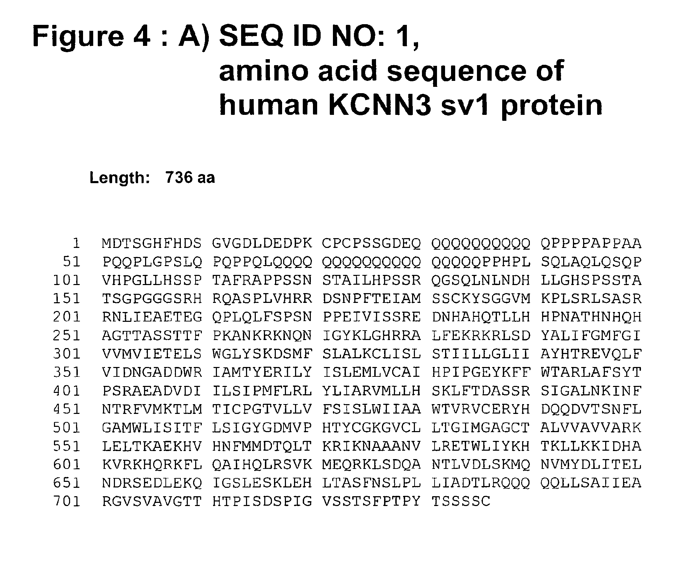

Figure 4A discloses SEQ ID NO: 1, the amino acid sequence of the human KCNN3 protein (splice variant 1, sv1) (UniProt primary accession number Q9UG16). This KCNN3 protein comprises 736 amino acids.

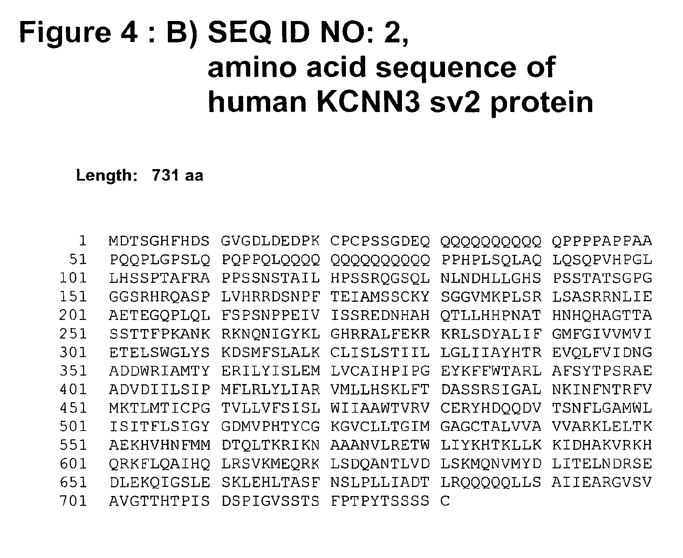

Figure 4B discloses SEQ ID NO: 2, the amino acid sequence of the human KCNN3 protein (splice variant 2, sv2) (UniProt primary accession number Q5VT74). This KCNN3 protein comprises 731 amino acids.

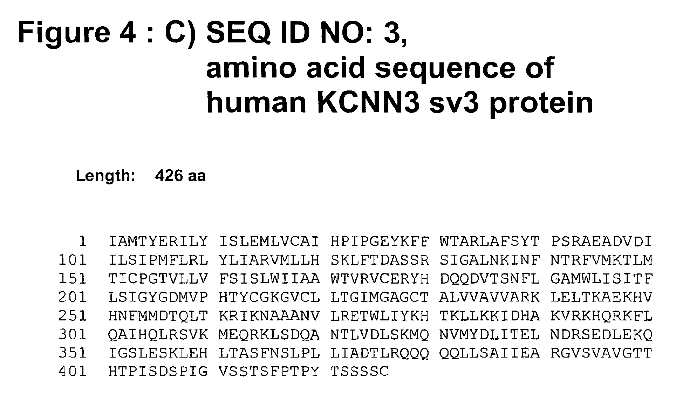

Figure 4C discloses SEQ ID NO: 3, the amino acid sequence of the human KCNN3 protein (splice variant 3, sv3) (UniProt primary accession number Q8WXG7). This KCNN3 protein comprises 426 amino acids.

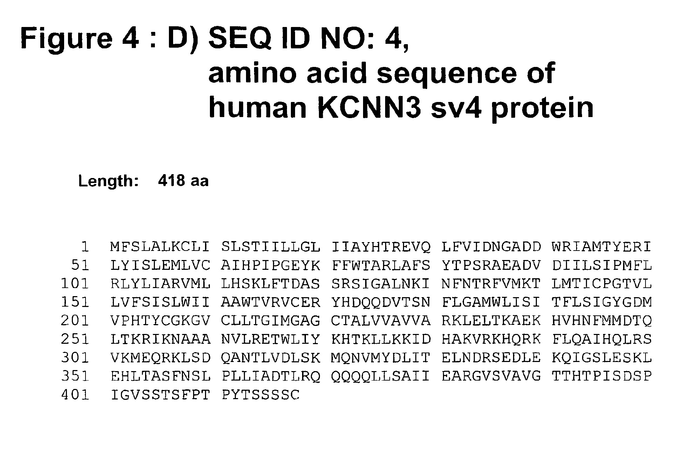

Figure 4D discloses SEQ ID NO: 4, the amino acid sequence of the human KCNN3 protein (splice variant 4, sv4) (UniProt primary accession number Q86VF9). This KCNN3 protein comprises 418 amino acids.

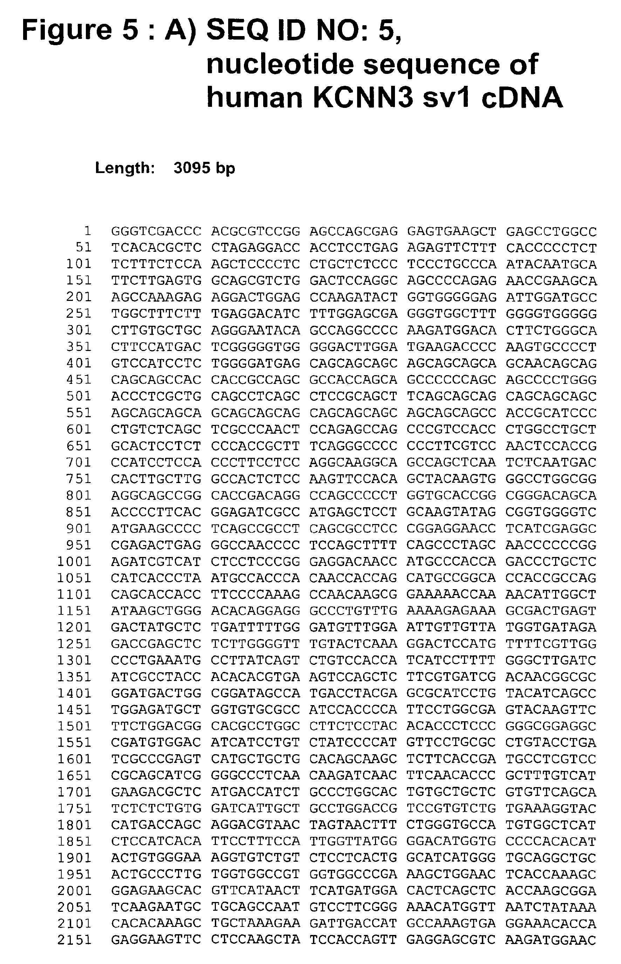

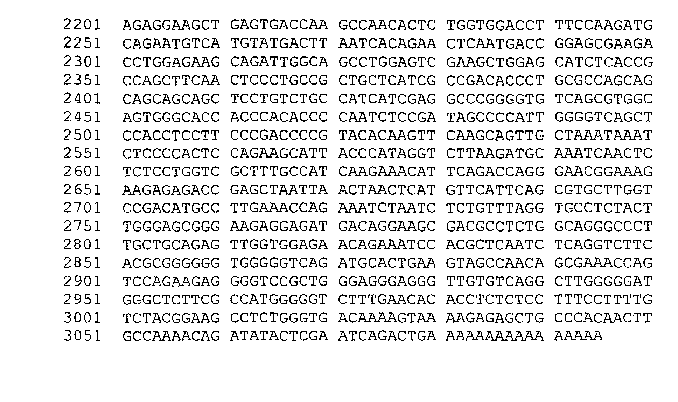

Figure 5A shows SEQ ID NO: 5, the nucleotide sequence of the human KCNN3 cDNA (splice variant 1, sv1) (Genbank accession number AJ251016) encoding the KCNN3 sv1 protein, comprising 3095 nucleotides.

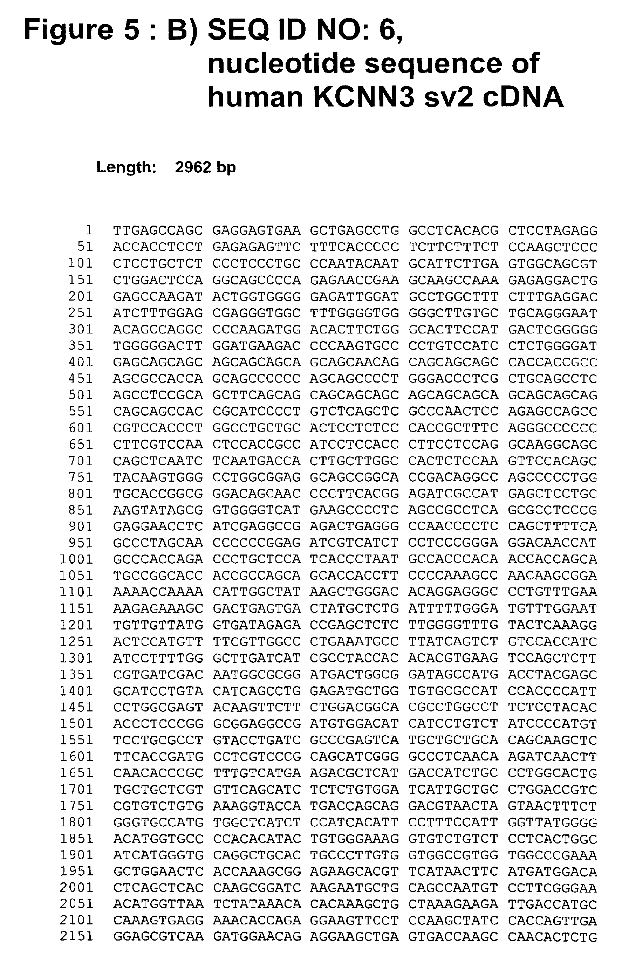

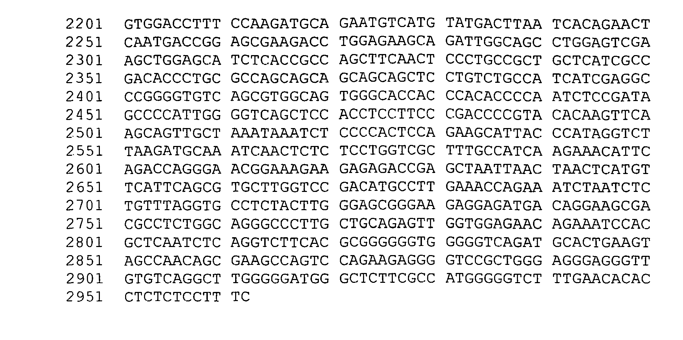

Figure 5B shows SEQ ID NO: 6, the nucleotide sequence of the human KCNN3 cDNA (splice variant 2, sv2) (Ensembl transcript ID number ENST00000368469) encoding the KCNN3 sv2 protein, comprising 2962 nucleotides.

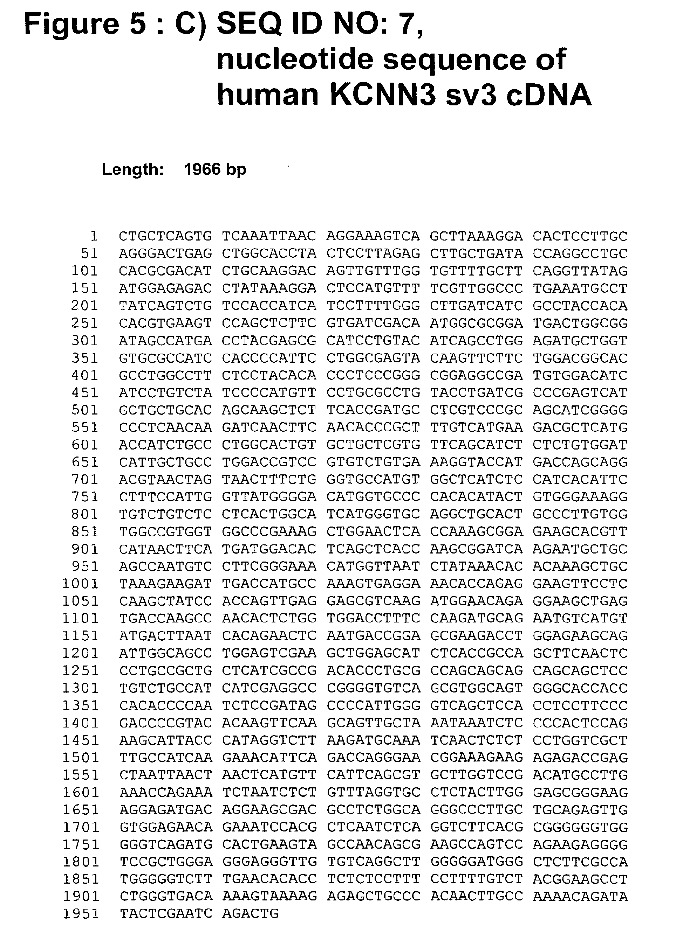

Figure 5C shows SEQ ID NO: 7, the nucleotide sequence of the human KCNN3 cDNA (splice variant 3, sv3) (Ensembl transcript ID number ENST00000361147) encoding the KCNN3 sv3 protein, comprising 1966 nucleotides.

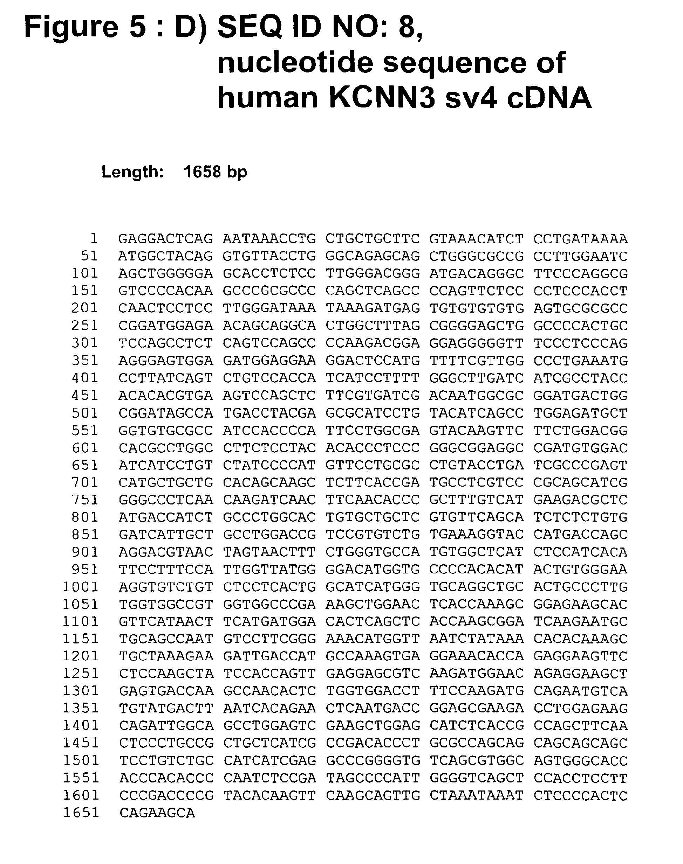

Figure 5D shows SEQ ID NO: 8, the nucleotide sequence of the human KCNN3 cDNA (splice variant 4, sv4) (Ensembl transcript ID number ENST00000358505) encoding the KCNN3 sv4 protein, comprising 1658 nucleotides.

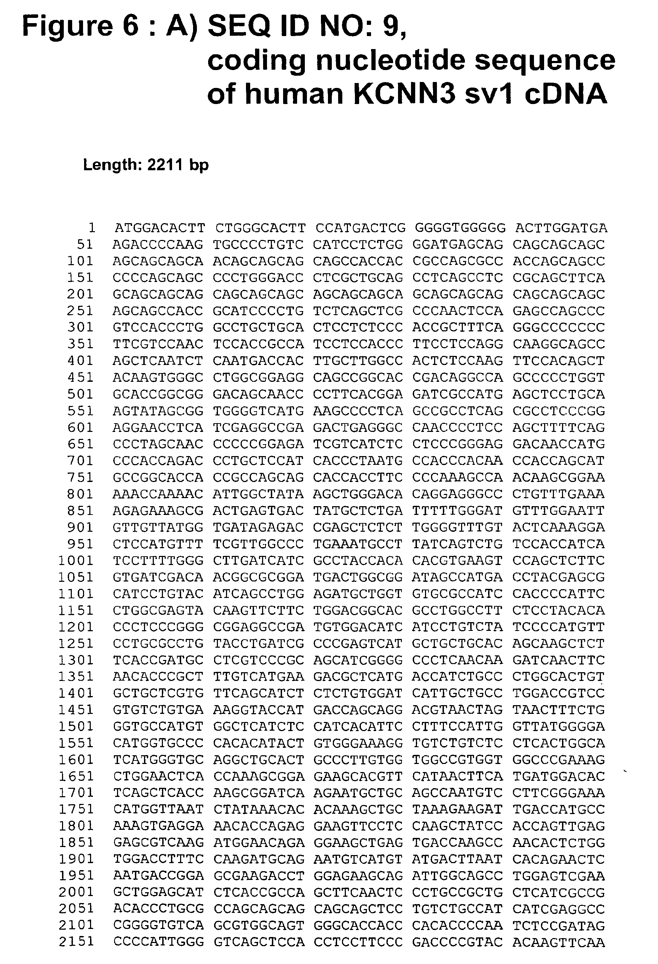

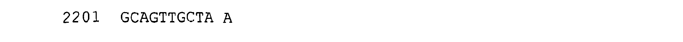

Figure 6A depicts SEQ ID NO: 9, the coding sequence (cds) of the human KCNN3 sv1, comprising 2211 nucleotides, harbouring nucleotides 334 to 2544 of SEQ ID NO. 5.

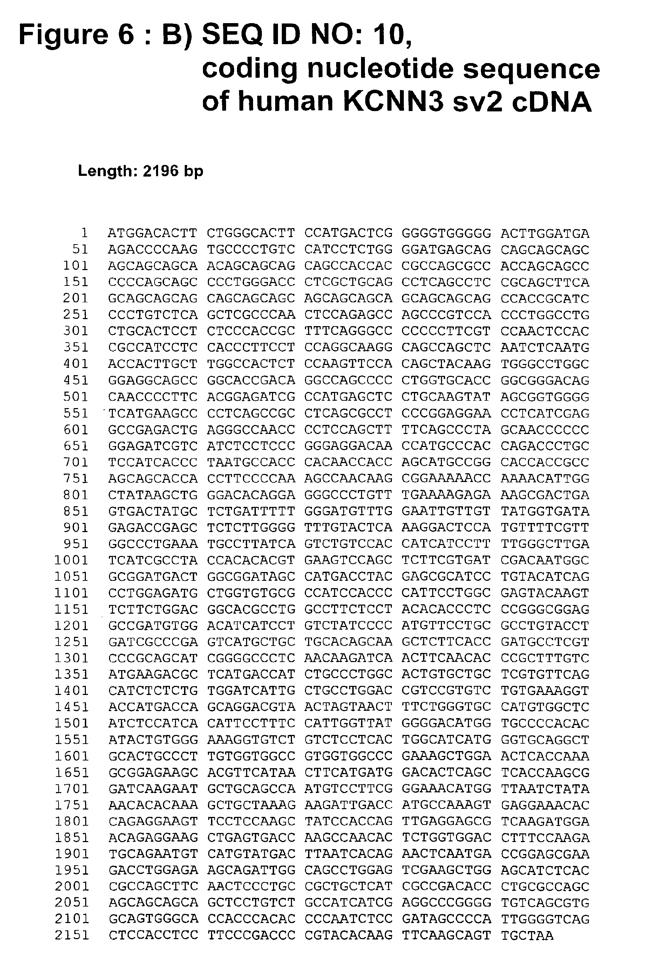

Figure 6B depicts SEQ ID NO: 10, the coding sequence (cds) of the human KCNN3 sv2, comprising 2196 nucleotides, harbouring nucleotides 317 to 2512 of SEQ ID NO. 6.

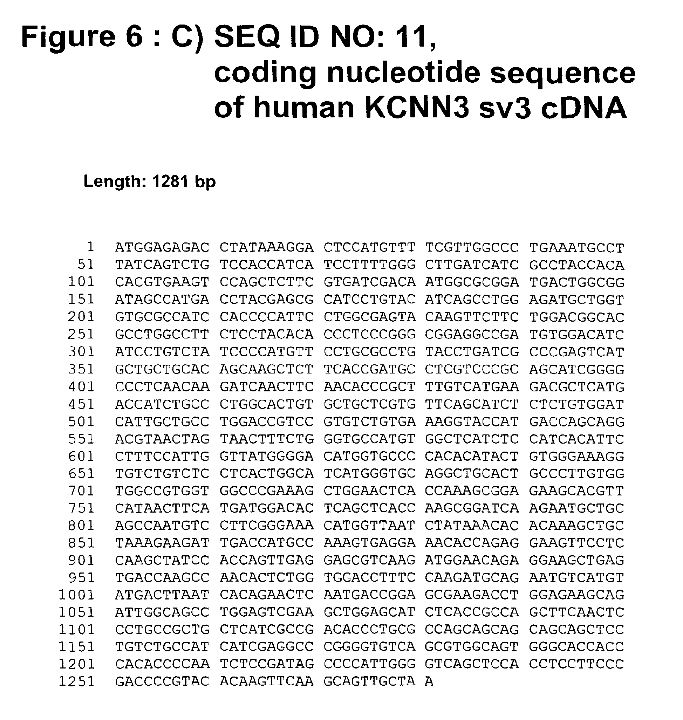

Figure 6C depicts SEQ ID NO: 11, the coding sequence (cds) of the human KCNN3 sv3, comprising 1281 nucleotides, harbouring nucleotides 151 to 1431 of SEQ ID NO. 7.

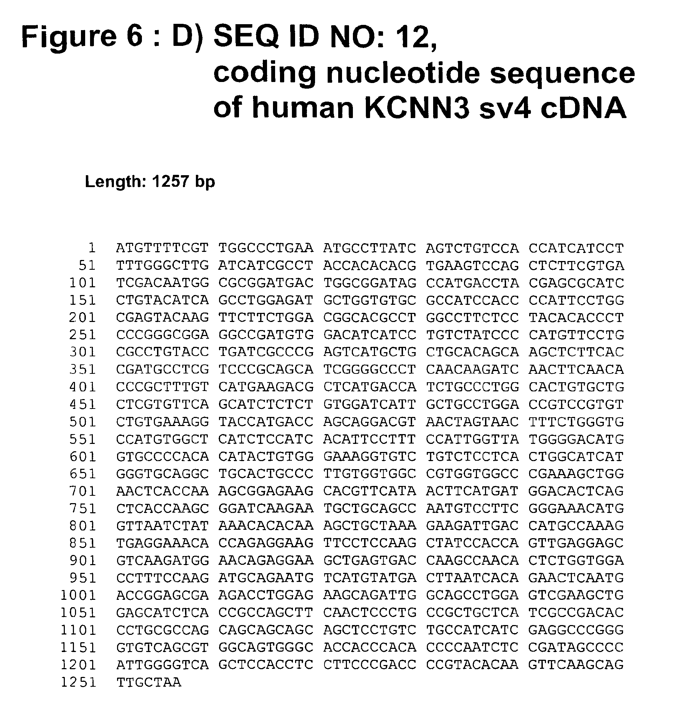

Figure 6D depicts SEQ ID NO: 12, the coding sequence (cds) of the human KCNN3 sv4, comprising 1257 nucleotides, harbouring nucleotides 378 to 1634 of SEQ ID NO. 8.

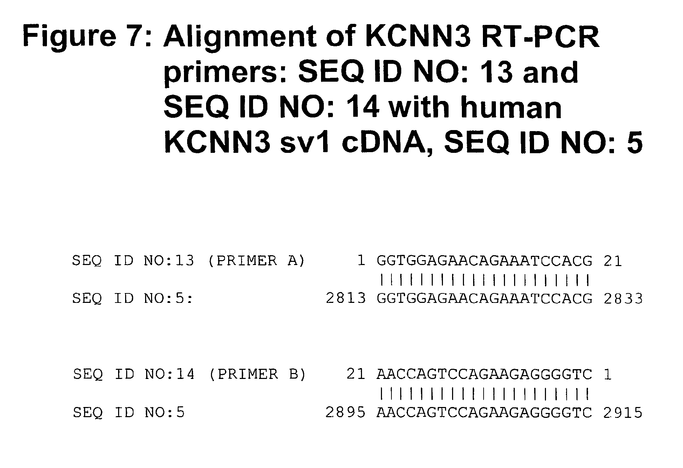

Figure 7 depicts the sequence alignment of the primers used for KCNN3 transcription level profiling (primer A, SEQ ID NO: 13 and primer B, SEQ ID NO: 14) by quantitative RT-PCR with the corresponding clippings of SEQ ID NO: 5, KCNN3 cDNA.

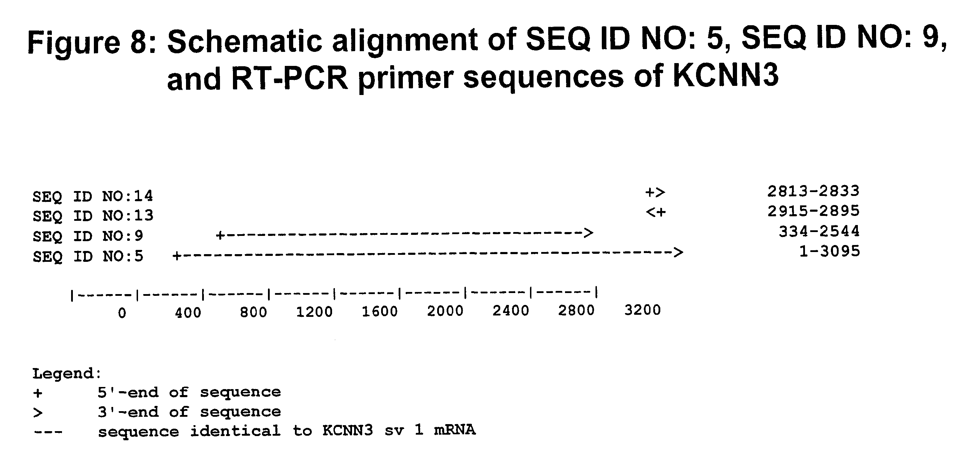

Figure 8 schematically charts the alignment of the KCNN3 cDNA sequence SEQ ID NO: 5, the coding sequence SEQ ID NO: 9 and both primer sequences used for KCNN3 transcription level profiling (primer A, SEQ ID NO: 13, primer B, SEQ ID NO: 14). Sequence positions are indicated on the right side.

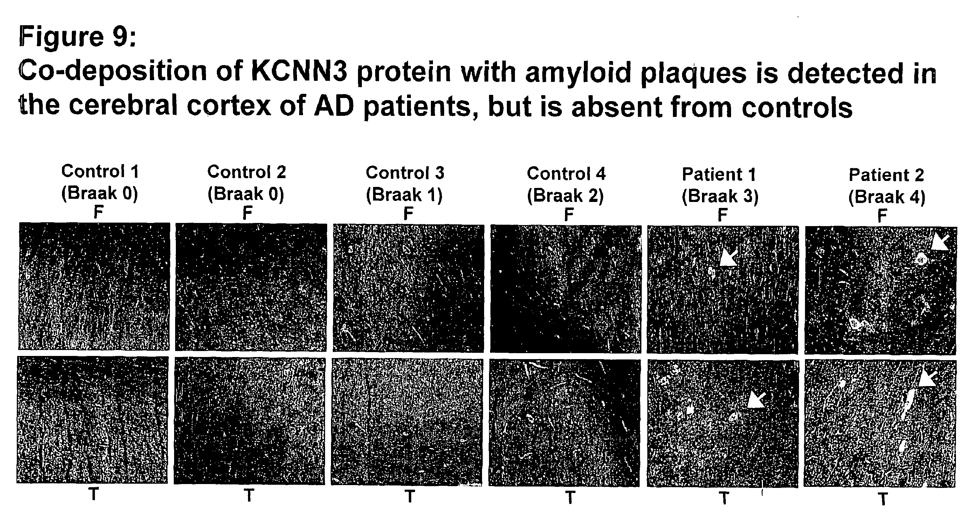

Figure 9 exemplifies the co-deposition of KCNN3 protein with cortical beta-amyloid plaques observed in human brain specimens from AD patients (starting from Braak stage 3). In contrast no such deposition of KCNN3 protein is observed in brain specimens from age-matched controls which have not been diagnosed to suffer from AD signs and symptoms and have been neuropathologically staged into Braak stages 0 to 2. The typical example demonstrates the general finding that KCNN3 protein is co-deposited with amyloid plaques (e.g. arrow) in AD patients, which is not observed in controls. Depicted are double-immunofluorescence micrographs (original magnification x10) of acetone-fixed cryostat sections of fresh-frozen post-mortem human brain frontal (F, upper row) and inferior temporal (T, lower row) cortex specimens from AD patients and age-matched controls, at Braak stages 0 to 4. Green signals represent KCNN3 specific immunoreactivity revealed by the affinity-purified polyclonal rabbit anti-KCNN3 antiserum (Alomone Labs) detected by AlexaFluor-488 conjugated goat anti-rabbit IgG secondary antiserum (Molecular Probes/ Invitrogen). Red signals reveal the neuron-specific somatic marker protein NeuN as detected by the mouse monoclonal anti-NeuN antibody (Chemicon) followed by Cy3-conjugated goat anti-mouse IgG secondary antiserum (Jackson/Dianova). Nuclei are stained blue by DAPI (Sigma). The areas showing diffuse green background staining of the neuropil represent cortical gray matter, whereas the white matter is not labeled and therefore appears dark.

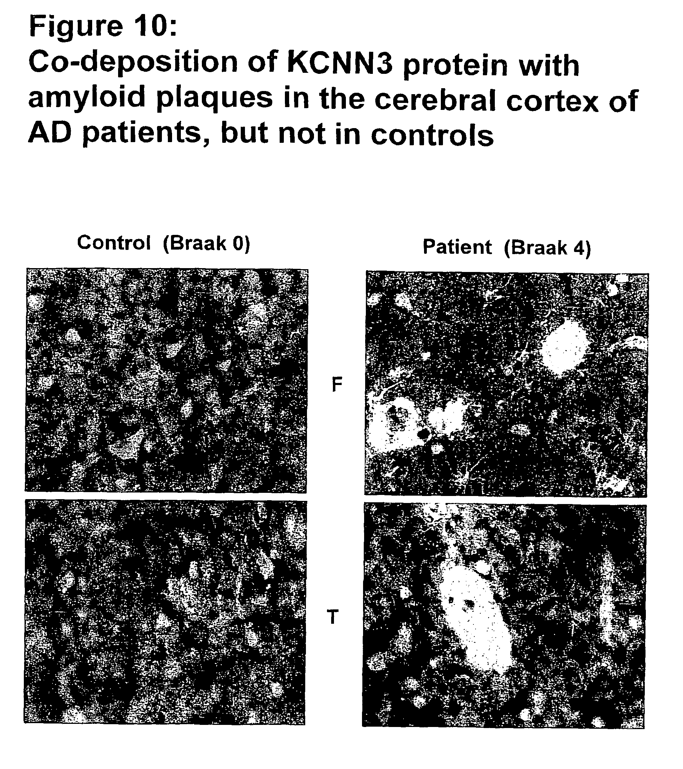

Figure 10 exemplifies in a magnificated picture the co-deposition of KCNN3 protein with cortical beta-amyloid plaques in human brain specimen from an AD patients (Braak stage 4). In contrast no such deposition of KCNN3 protein is observed in brain specimen from age-matched controls which have not been diagnosed to suffer from AD signs and symptoms and have been neuropathologically staged into Braak stage 0. These characteristic images demonstrate that KCNN3 protein is co-deposited with amyloid plaques in patients but not in controls. Depicted is a high power view (original magnification x40) of double-immunofluorescence micrographs of acetone-fixed cryostat sections of fresh-frozen post-mortem human brain frontal (F) and inferior temporal (T) cortex specimens from an AD patient and an age-matched control, at Braak stages 4 and 0, respectively. Green signals represent specific KCNN3 immunoreactivity revealed by the affinity-purified polyclonal rabbit anti-KCNN3 antiserum (Alomone Labs) detected by AlexaFluor-488 conjugated goat anti-rabbit IgG secondary antiserum (Molecular Probes/invitrogen). Red signals reveal the neuron-specific somatic marker protein NeuN as detected by the mouse monoclonal anti-NeuN antibody (Chemicon) followed by Cy3-conjugated goat anti-mouse IgG secondary antiserum (Jackson/Dianova). Nuclei are stained blue by DAPI (Sigma).

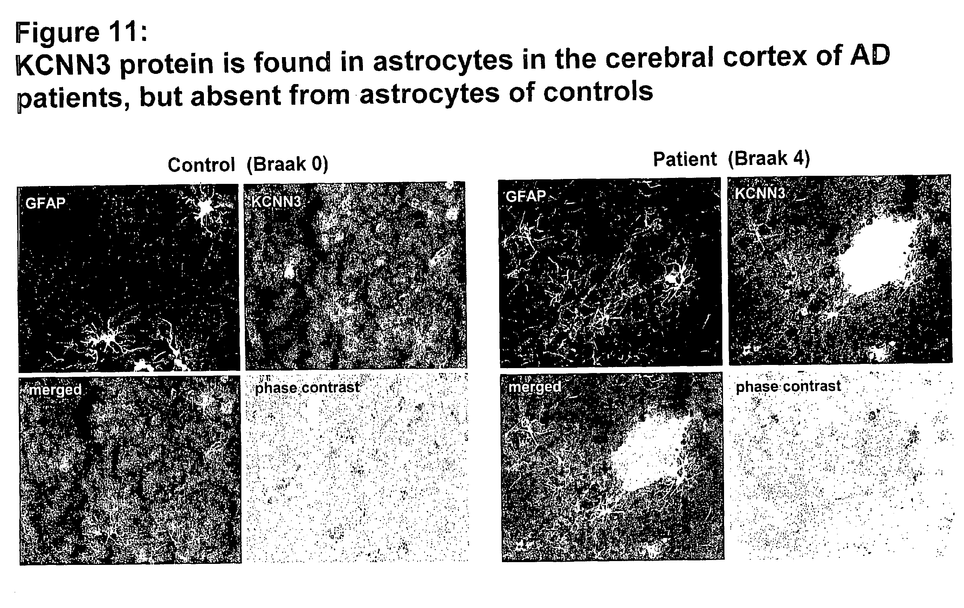

Figure 11 exemplifies that reactive astrocytes in the cortex of AD patients (Braak stage 4) contain the KCNN3 protein at high levels. In contrast no PRKX protein can be found in astrocytes in the cortex of age-matched controls which have not been diagnosed to suffer from AD signs and symptoms and have been neuropathologically staged into Braak stage 0. In addition, the figure again demonstrates a KCNN3 protein-containing plaque deposit which is present only in the AD patient sample but absent from the control. Depicted is a high power view (original magnification x40) of double-immunofluorescence micrographs of acetone-fixed cryostat sections of fresh-frozen post-mortem human brain frontal cortex specimens from an AD patient and an age-matched control, at Braak stages 4 and 0, respectively. Specific KCNN3 immunoreactivity is revealed by the affinity-purified polyclonal rabbit anti-KCNN3 antiserum (Alomone Labs) followed by AlexaFluor-488 conjugated goat anti-rabbit IgG secondary antiserum (Molecular Probes/invitrogen), visualized as either grayscale images (right upper quadrant of each panel) or green signals in the merged image (left lower quadrant of each panel). The astrocyte-specific marker protein GFAP is detected by the mouse monoclonal anti-GFAP antibody (Abcam) followed by Cy3-conjugated goat anti-mouse IgG secondary antiserum (Jackson/Dianova), visualized as either grayscale images (left upper quadrant of each panel) or red signals in the merged image (left lower quadrant of each panel). Nuclei are stained blue by DAPI (Sigma). The right lower quadrants show the corresponding phase contrast images.

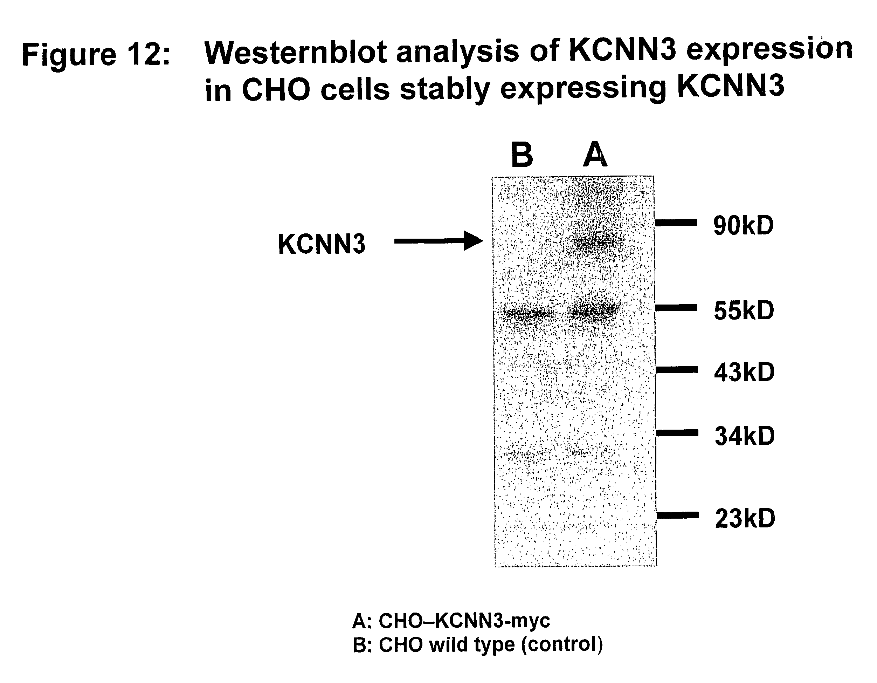

Figure 12 shows Western blot analysis of KCNN3 protein production in CHO cells. KCNN3 was myc-tagged at the C-terminus and introduced into tissue culture cells. Expression of KCNN3 is driven by the CMV-promoter. Cells were harvested, lysed and subjected to Western Blot analysis using an antibody directed against the myc-epitope at a 1:3000 dilution. In lane A a strong band running at approx. 80 kDa becomes visible. In the control CHO wild type cell line no corresponding band running at the same molecular weight is visible (lane B).

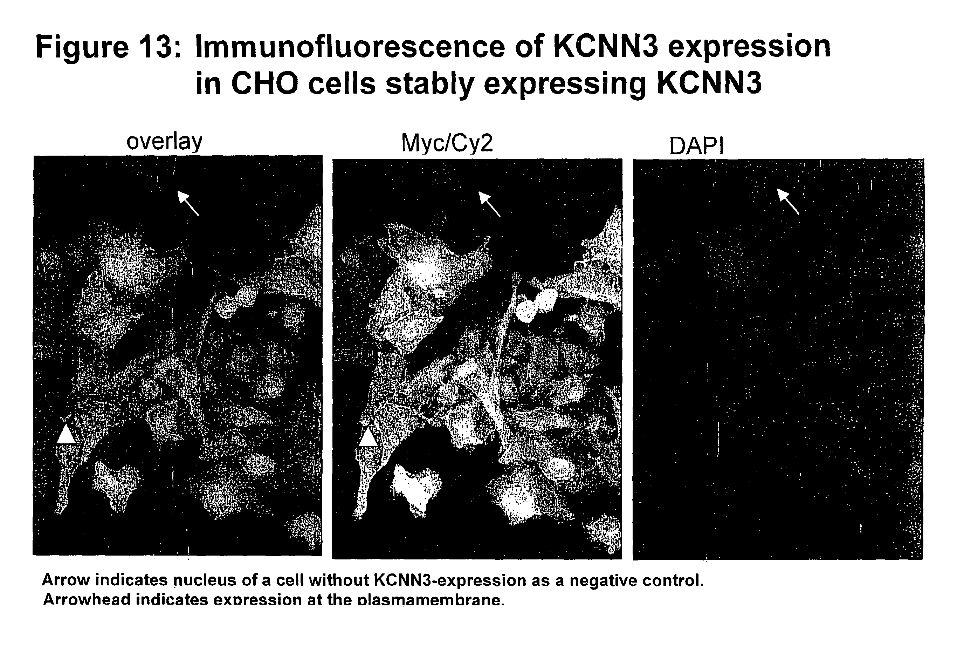

Figure 13 shows immunofluorescence analysis of KCNN3 expression in CHO cells. KCNN3 was myc-tagged at the C-terminus and introduced into tissue culture cells. Expression of KCNN3 is under the control of the CMV-promoter. KCNN3 -expressing cells were seeded onto a glass cover slip and after 24 hours of incubation cells where fixed with methanol for immunofluorescence analysis. Expression of KCNN3 was detected using an antibody directed against the myc-epitope at a 1:3000 dilution followed by incubation with a fluorescently labelled antibody directed against the anti-myc antibody (1:1000). Cells were then mounted onto a microscope slide and analysed under a fluorescence microscope. Expression of KCNN3 is visible in the cytoplasm and at the plasma-membrane of the cells in the left and middle pictures (see arrowhead pointing to the expression at the border of the cell indicating the localization at the membrane). For comparison arrow points to the nucleus of a cell where no or significantly lower fluorescence can be detected indicating no or a very low expression of KCNN3 (left and right panel). The blue colour in the left and right pictures is indicative of the nucleus of the cells that has been stained by means of DAPI (1:1000).

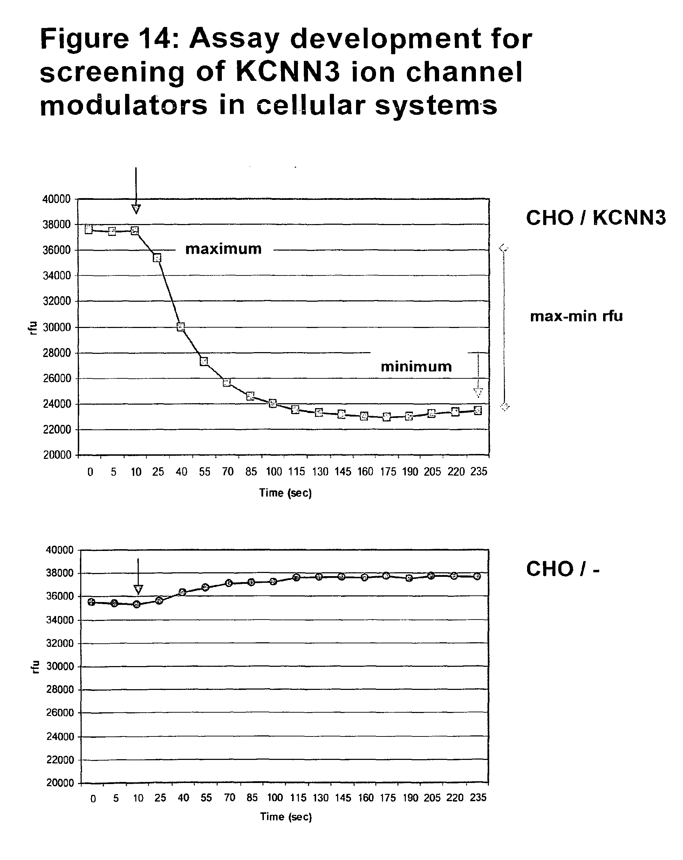

Figure 14 summarizes the assay development for screening of KCNN3 ion channel modulating compounds in cellular systems. CHO cells stably expressing KCNN3 (CHO/KCNN3) under the CMV promoter were incubated with apamin and trifluorperatine 30 minutes before the addition of ionomycin which activates the ion channel (1 $\mu$M final concentration). Upon entry of calcium into the cells the potassium channel is activated and thus impacts on the resting membrane potential of the cells which is mirrored by the fluorescent dye. The minimum fluorescence values measured during the following 5 minutes period were subtracted from the maximum signal recorded at the beginning of the experiment and plotted against the concentration of the substances incubated with the cells. The values obtained in the absence and at the lowest concentrations are indicative of a fully active potassium channel which leads to a hyperpolarization of the membrane potential. The figure also shows that after addition of ionomycin (red arrow) the fluorescence decreases indicative of an opening of KCNN3.

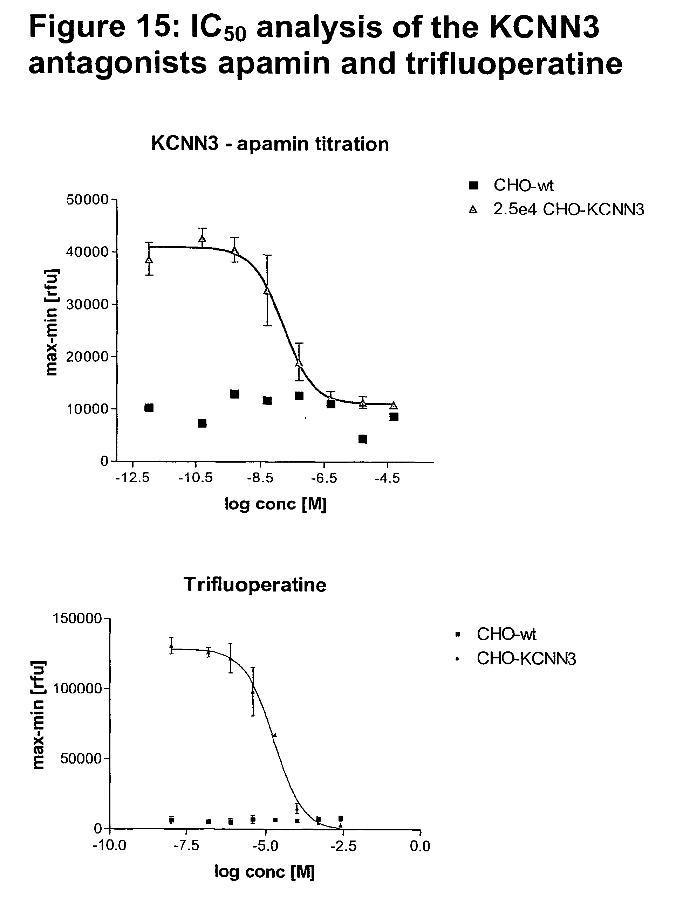

Figure 15 shows the IC50 determination of the KCNN3 antagonists apamin and trifluorperatine. Apamin and trifluorperatine were incubated with the cells 30 minutes before the addition of ionomycin (1$\mu$M final concentration). The minimum fluorescence values during the following 5 minutes measurement period were subtracted from the maximum signal recorded at the beginning of the experiment and plotted against the concentration of the substances. The calculated IC50 was 16 nM for apamin and 18 $\mu$M for trifluorperatine which fits to the $IC_{50}$-value reported in Terstappen et al. (Neuropharmacology 40, 2001:772-783). In case of CHO wild type cells (CHO/-) no effect could be seen by the addition of ionomycin and apamin, respectively.

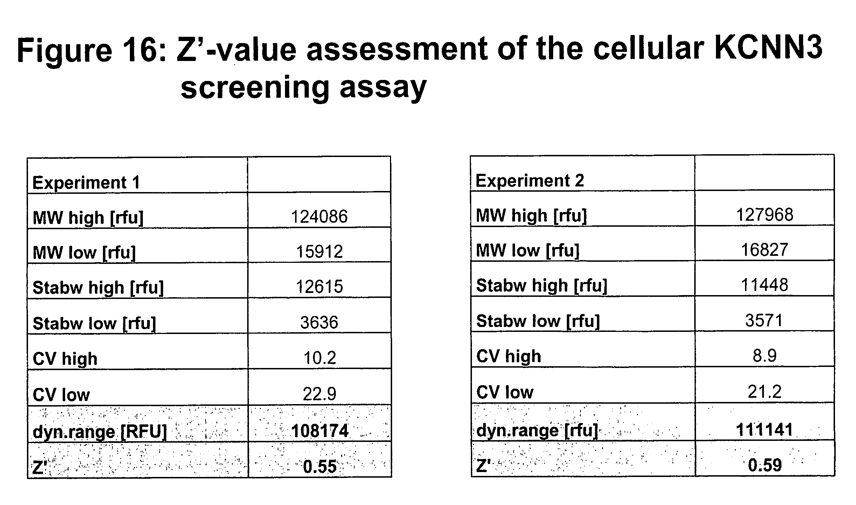

Figure 16 shows the Z'-value assessment of the cellular KCNN3 screening assay. The difference of the fluorescence intensity before addition of ionomycin and after the 120 seconds incubation period was determined where the cells were incubated with fluoxetine. The influx of calcium mediated by ionomycin leads to an opening of the channels and a subsequent hyperpolarization of the resting membrane potential of the cells. In the presence of fluoxetine on the other hand the ion channel is blocked and the influx of calcium into the cells leads to a comparably small change of the fluorescence intensity only and, hence, the resting membrane potential. This indicates that the ion channel is blocked. The mean value of the difference of the fluorescence in presence of ionomycin and fluoxetine in the above experiment is 15912 rfu (standard deviation 3636 rfu) and the mean value of the fluorescence of cells in presence of ionomycin only is 124086 rfu (standard deviation 12615 rfu). The Z'-value is calculated to be 0.55. The Z' in a second series of experiments was determined to be 0.59.

EXAMPLE 1: Identification and verification of Alzheimer's disease related differentially expressed genes in human brain tissue samples.

In order to identify specific differences in the expression of genes that are associated with AD, GeneChip microarray (Affymetrix) analyses were performed with a diversity of cRNA probes derived from human brain tissue specimens from clinically and neuropathologically well characterized individuals. This technique is widely used to generate expression profiles of multiple genes and to compare populations of mRNA present in different tissue samples. In the present invention, mRNA populations present in selected post-mortem brain tissue specimens (frontal and inferior temporal cortex) were analyzed. Tissue samples were derived from individuals that could be grouped into different Braak stages reflecting the full range between healthy control individuals (Braak 0) and individuals that suffered from AD signs and symptoms (Braak 4). Verification of the differential expression of individual genes was performed applying real-time quantitative PCR using gene-specific oligonucleotides. Furthermore specific differences between healthy and disease stages were analysed at the protein level using gene product specific antibodies for immunohistochemical analyses. The methods were designed to specifically detect differences of expression levels at early Braak stages, which is indicative for pathological events occurring early in the course of the disease. Thus, said genes identified to be differential are effectively implicated in the pathogenesis of AD.

(i) Brain tissue dissection from patients with AD:

Brain tissue samples from AD patients and age-matched control subjects were collected. Within 6 hours post-mortem time the samples were immediately frozen on dry ice. Sample sections from each tissue were fixed in paraformaldehyde and neuropathologically staged at various stages of neurofibrillary pathology according to Braak and Braak into Braak stages (0-4); Brain areas for differential expression analysis were identified and stored at-80°C until RNA extractions were performed.

(ii) Isolation of total mRNA:

Total RNA was extracted from frozen post-mortem brain tissue by using the RNeasy kit (Qiagen) according to the manufacturer's protocol. The accurate RNA concentration and the RNA quality were determined applying the *Eukaryote total RNA Nano* LabChip system by using the *2100 Bioanalyzer* (Agilent Technologies). For additional quality testing of the prepared RNA, i.e. exclusion of partial degradation and testing for DNA contamination, specifically designed intronic GAPDH oligonucleotides and genomic DNA as reference control were utilised to generate a melting curve with the LightCycler technology (Roche) as described in the supplied protocol by the manufacturer.

(iii) Probe synthesis:

Here, total RNA was used as starting material, which had been extracted as described above (ii). For production of cDNAs, the *cDNA Synthesis System* was performed according to the manufacturer's protocol (Roche). cDNA samples were transcribed to cRNA and labeled with biotin applying the *in vitro*-transcription *T7-Megascript-Kit* (Ambion) according to the manufacturer's protocol. The cRNA quality was determined applying the *mRNA Smear Nano* LabChip system using the *2100 Bioanalyzer* (Agilent Technologies). The accurate cRNA concentration was determined by photometric analysis ($OD_{260/280\ nm}$).

(iv) GeneChip hybridization:

The purified and fragmented biotin labeled cRNA probes together with commercial spike controls (Affymetrix) *bioB (1.5 pM), bioC* (5 *pM), bioD (25 pM), and cre (100 pM)* were resuspended each at a concentration of 60 ng/µl in hybridization buffer (0.1 mg/ml Herring Sperm DNA, 0.5 mg/ml Acetylated BSA, 1 x MES) and subsequently denaturated for 5 min at 99°C. Subsequently, probes were applied each onto one prehybridized (1 x MES) *Human Genome U133 Plus 2.0 Array* (Affymetrix). Array hybridization was performed over night at 45°C and 60 rpm. Washing and staining of the microarrays followed according to the instruction *EukGe_WS2v4* (Affymetrix) controlled by *GeneChip Operating System (GCOS)* 1.2 (Affymetrix).

(v) GeneChip data analysis:

Fluorescence raw data were taken using the *GeneScanner 3000* (Affymetrix) controlled by *GCOS 1.2 software* (Affymetrix). Data analysis was performed using *DecisionSite 8.0 for Functional Genomics* (Spotfire): raw data were delimitated to those that were flagged as "present" by the *GCOS 1.2 software* (Affymetrix); normalization of raw data was performed by percentile value; detection of differential mRNA expression profiles was performed using profile search tool of the Spotfire software. The result of such GeneChip data analysis for the gene coding for KCNN3 protein is shown in Figure 1.

(vi) Quantitative RT-PCR:

Positive corroboration of differential KCNN3 gene expression was performed using the LightCycler technology (Roche). This technique features rapid thermal cycling for the polymerase chain reaction as well as real-time measurement of fluorescent signals during amplification and therefore allows for highly accurate quantification of RT-PCR products by using a kinetic, rather than endpoint readout. The relative quantity of KCNN3 cDNAs from the frontal and inferior temporal cortices of AD patients and age-matched control individuals respectively, were determined in a number of four up to nine tissues per Braak stage.

First, a standard curve was generated to determine the efficiency of the PCR with specific primers for the gene coding for KCNN3:

Primer A, SEQ ID NO: 13, 5'-GGTGGAGAACAGAAATCCACG-3' (nucleotides 2813-2833 of SEQ ID NO: 2) and Primer B, SEQ ID NO: 14, 3'-AACCAGTCCAGAAGAGGGGTC-5' (nucleotides 2895-2915 of SEQ ID NO: 5).

PCR amplification (95°C and 1 sec, 56°C and 5 sec, and 72°C and 5 sec) was performed in a volume of 20 μl containing LightCycler-FastStart DNA Master SYBR Green I mix (contains FastStart Taq DNA polymerase, reaction buffer, dNTP mix with dUTP instead of dTTP, SYBR Green I dye, and 1 mM $MgCl_2$; Roche), 0.5 μM primers, 2 μl of a cDNA dilution series (final concentration of 40, 20, 10, 5, 1 and 0.5 ng human total brain cDNA; Clontech) and additional 3 mM $MgCl_2$. Melting curve analysis revealed a single peak at approximately 86°C with no visible primer dimers. Quality and size of the qPCR product were determined applying the *DNA 500* LabChip system using the *2100 Bioanalyzer* (Agilent Technologies). A single peak at the expected size of 103 bp for the gene coding for KCNN3 protein was observed in the electropherogram of the sample.

In an analogous manner, the qPCR protocol was applied to determine the PCR efficiency of cyclophilin B, using the specific primers SEQ ID NO:15, 5'-ACTGAAGCACTACGGGCCTG-3' and SEQ ID NO:16, 5'-AGCCGTTGGTGTCTTTGCC-3' except for $MgCl_2$ (an additional 1 mM was added instead of 3 mM). Melting curve analysis revealed a single peak at approximately 87°C with no visible primer dimers. Bioanalyzer analysis of the PCR product showed one single peak of the expected size (62 bp).

For calculation of the standard values, first the logarithm of the used cDNA concentration was plotted against the threshold cycle value $C_t$ for KCNN3 and Cyclophilin B respectively. The slopes and the intercepts of the standard curves (i.e. linear regressions) were calculated. In a second step, mRNA expression from frontal and inferior temporal cortices of controls and AD patients were analyzed in parallel. The $C_t$ values were measured and converted to ng total brain cDNA using the corresponding standard curves:

$$10 \wedge (C_t \text{ value - intercept}) / \text{slope} \qquad [\text{ng total brain cDNA}]$$

Calculated cDNA concentration values were normalized to Cyclophilin B that was analyzed in parallel for each tested tissue probe, thus resulting values are defined as arbitrary relative expression levels. The results of such quantitative RT-PCR analysis for the gene coding for KCNN3 protein are shown in Figure 2. (vii) Statistical analysis of the mRNA expression comparing donor groups with different Braak stages.

For this analysis it was proven that absolute values of real-time quantitative PCR (Lightcycler method) between different experiments at different time points are consistent enough to be used for quantitative comparisons without usage of calibrators. Cyclophilin was used as a standard for normalization in any of the qPCR experiments for more than 100 tissues. Between others it was found to be the most consistently expressed housekeeping gene in the normalization experiments. Therefore a proof of concept was done by using values that were generated for cyclophilin.

First analysis used cyclophilin values from qPCR experiments of frontal cortex and inferior temporal cortex tissues from three different donors. From each tissue the same cDNA preparation was used in all analyzed experiments. Within this analysis no normal distribution of values was achieved due to small number of data. Therefore the method of median and its 98 %-confidence level was applied. This analysis revealed a middle deviation of 8.7 % from the median for comparison of absolute values and a middle deviation of 6.6 % from the median for relative comparison.

Second analysis used cyclophilin values from qPCR experiments of frontal cortex and inferior temporal cortex tissues from two different donors each, but different cDNA preparations from different time points were used. This analysis revealed a middle deviation of 29.2 % from the median for comparison of absolute values and a middle deviation of 17.6 % from the median for relative comparison. From this analysis it was concluded, that absolute values from qPCR experiments can be used, but the middle deviation from median should be taken into further considerations.

A detailed analysis of absolute values for KCNN3 was performed. Therefore, absolute levels of KCNN3 were used after relative normalization with cyclophilin. The median as well as the 98 %-confidence level was calculated for a group consisting of low level Braak stages (Braak 0 - Braak 2) and another group consisting of high level Braak stages (Braak 3 - Braak 4). The analysis was aimed to identify early onset of mRNA expression differences within the course of AD pathology. Said analysis described above is shown in Figure 3.

(viii) Verification of differential expression of the KCNN3 gene and association with AD at the protein level applying immunohistochemical analyses:

For immunofluorescence staining of KCNN3 in human brain, and for the comparison of AD-affected tissues with control tissues, post-mortem fresh-frozen frontal and inferior temporal forebrain specimens from donors comprising patients with clinically diagnosed and neuropathologically confirmed AD at various stages of neurofibrillary pathology according to Braak and Braak (herein before and after briefly called "Braak stages") as well as age-matched controls with neither clinical nor neuropathological signs of AD were cut at 14 μm thickness using a cryostat (Leica CM3050S). The tissue sections were air-dried at room temperature and fixed in acetone for 10 min, and air-dried again. After washing in PBS, the sections were pre-incubated with blocking buffer (10% normal horse serum, 0.2% Triton X-100 in PBS) for 30 min and then incubated with affinity-purified anti-KCNN3 rabbit polyclonal antibodies (APC-025, Alomone Labs, Jerusalem, Israel) in a 1:20 dilution in blocking buffer, overnight at 4°C. After rinsing three times in 0.1% Triton X-100/PBS, the sections were incubated with AlexaFluor-488-conjugated goat anti-rabbit IgG antiserum (Jackson/Dianova, Hamburg, Germany), in a 1:1500 dilution in 1% BSA/PBS for 2 hours at room temperature and then again washed with PBS. Simultaneous staining of either (i) neuronal somata or (ii) astrocytes was performed as described above using additional mouse monoclonal antibodies against either (i) the neuron-specific somatic marker protein NeuN (Chemicon, Hampshire, UK; dilution 1:350) or (ii) the astrocyte-specific marker protein glial acidic fibrillary protein (GFAP, Abcam, Cambridge, UK; dilution 1:250), respectively, in either case followed by a secondary Cy3-conjugated goat anti-mouse antibody (Jackson/Dianova; dilution 1:800). Staining of the nuclei was performed by incubation of the sections with 5 μM DAPI in PBS for 3 min. In order to block lipofuscin autofluoresence, the sections were treated with the lipophilic black dye Sudan Black B (1% w/v) in 70% ethanol for 5 min at room temperature and then sequentially dipped in 70% ethanol, destilled water and PBS. The sections were coverslipped with ProLong-Gold antifade mounting medium (invitrogen/Molecular Probes, Karlsruhe, Germany). Microscopic images were obtained using epifluorescence or phase contrast illumination conditions using an upright microscope with a mercury-arc lamp (BX51, Olympus, Hamburg, Germany). The appropriate dichromic filter and mirror combinations (hereinafter called "channels") were used for the specific excitation of either fluorochrome (AlexaFluor-488, Cy3, DAPI) and for reading out the emitted fluorescence light resulting from the specific labeling by said antibodies or the nuclear DAPI stain. Microscopic images were digitally captured with a charge-coupled display camera and the appropriate image acquisiton and processing software (ColorView-ll and AnalySIS, Soft Imaging System, Olympus, Germany). Fluorescence micrographs obtained from the different channels were overlaid in order to generate simultaneous views of the above specified immunolabelings and nuclei (DAPI) in the RGB mode, e.g. for analyzing co-localization of signals from up to three different channels.

EXAMPLE 2: Analyses of KCNN3 functions in AD using cell culture systems.

(i) Generation of cell lines stably producing KCNN3:

The KCNN3 gene under the control of the CMV promoter was cloned using a standard expression plasmid, transduced into CHO cells and clonal cell lines were isolated after the addition of the antibiotic G418 essentially following the manufacturer's protocol (Stratagene, Cat. No. 217561). The production of KCNN3 protein and its localization in different cell lines was analysed using indirect immunofluorescence microscopy (Figure 13) and a cell line showing stable production was selected. This CHO cell line was used subsequently to establish an assay for investigating the activity of the ion channel in the natural cellular environment.

(ii) Development of a cellular assay for the identification of KCNN3 modulators:

Basically, the assay protocol has been developed as outlined in the publication by Terstappen et al. (Neuropharmacology 40:772-783, 2001). The assay makes use of membrane potential sensitive, fluorescent dyes and the activation of the calcium-sensitive KCNN3-potassium channel by the addition of ionomycin. Upon entry of calcium into the cells KCNN3 is activated and thus impacts on the resting membrane potential of the cells which is mirrored by the fluorescent dye. In more detail: CHO cells expressing KCNN3 are cultured on black 384-well plates with clear bottom at a density of 1.5·104 for 24 hours in a humidified incubator at 37°C. The cell culture medium is replaced with assay buffer (1 mM KCl, 2.3 mM CaCl2, 5 mM NaHCO3, 1 mM MgCl2, 154 mM NaCl, 5.5 mM d(+)-glucose, 5 mM HEPES, pH 7.4) containing 5μM DiBAC4(3). After 30 min of cell loading with the dye at 37°C fluorescence (excitation 488 nm, emission 538 nm) is read by using a fluorescence plate reader (Flex-station, Molecular Devices). Activation of KCNN3 is achieved by the addition of ionomycin at a 1μM final concentration (Figure 14 and 15). As standard blockers the bee-venom apamin as well as trifluorperatine have been added to the cells 30 minutes prior to the addition of ionomycin. Fluorescence is monitored for a period of 5 min.

(iii) Optimization of a cellular KCNN3 screening assay:

In order to optimize the KCNN3 assay for screening purposes the Z'-value was assessed (Figure 16). After 24 hours of incubation the medium was removed from a 384-well plate where CHO-KCNN3 cells were grown. 20 μl dye solution in assay buffer was pipetted onto the cells and afterwards another 20 μl assay buffer only or 20 μl assay buffer plus 100 μM fluoxetine was added. After 30 minutes of incubation at 37°C the plate was transferred to the automated pipetting device/fluorescence reader (FlexStation, Molecular Devices) and 10 μl ionomycin was added at a final concentration of 500 nM. Fluorescence was then measured for 120 sec every 10 sec. Excitation wavelength was 485 nm and emission wavelength was 538 nm (cut-off filter 530 nm).

## Claims

**1.** A method of diagnosing Alzheimer's disease in a subject, or of monitoring the effect of a treatment administered to a subject having said disease, which method comprises the steps of:

(a) determining a level and/or an activity of (i) a transcription product of the gene coding for KCNN3 proteins having SEQ ID NO: 1, SEQ ID NO: 2, SEQ ID NO: 3 or SEQ ID NO: 4, and/or (ii) a translation product of the gene coding for KCNN3 proteins having SEQ ID NO: 1, SEQ ID NO: 2, SEQ ID NO: 3 or SEQ ID NO: 4, and/or (iii) a variant of said transcription or translation product comprising a sequence that has at least about 80% sequence identity with the amino acid sequences of said KCNN3 proteins in a brain tissue sample obtained from said subject,
(b) comparing said level and/or said activity of said transcription product and/or said translation product to a reference value representing a known disease status and/or representing a known health status and/or representing a known Braak stage
(c) analysing whether said level and/or said activity is altered compared to a reference value representing a known health status, and/or is similar or equal to a reference value representing a known disease status or representing a known Braak stage which is an indication that said subject has Alzheimer's disease, or that said subject is at increased risk of developing said disease.

**2.** The method according to claim 1 wherein said gene coding for KCNN3 proteins is the gene coding for KCNN3 having SEQ ID NO: 1 and wherein said translation product of a gene coding for KCNN3 proteins is the KCNN3 protein having SEQ ID NO: 1.

**3.** Use of a kit for diagnosing Alzheimer's disease in a subject, or of monitoring the effect of a treatment administered to a subject having Alzheimer's disease according to the method of claim 1, wherein the kit comprises at least one reagent which is selected from the group consisting of (i) a transcription product of the gene coding for KCNN3 proteins having SEQ ID NO: 1, SEQ ID NO: 2, SEQ ID NO: 3 or SEQ ID NO: 4, and/or (ii) a translation product of the gene coding for KCNN3 proteins having SEQ ID NO: 1, SEQ ID NO: 2, SEQ ID NO: 3 or SEQ ID NO: 4, and/or (iii) a variant of said transcription or translation product comprising a sequence that has at least about 80% sequence identity with the amino acid sequences of said KCNN3 proteins.

**4.** Use of claim 3 wherein the KCNN3 protein has the SEQ ID NO: 1.

**5.** A method of screening for identifying agents, modulators or selective antagonists or agonists for use in the treatment of Alzheimer's disease, which agents, modulators or selective antagonists or agonists have an ability to alter expression or level or activity of one or more substances selected from the group consisting of

(i) a gene coding for KCNN3 proteins having SEQ ID NO: 1, SEQ ID NO: 2, SEQ ID NO: 3 or SEQ ID NO: 4, and/or
(ii) a transcription product of the gene coding for KCNN3 proteins having SEQ ID NO: 1, SEQ ID NO: 2, SEQ ID NO: 3 or SEQ ID NO: 4, and/or
(iii) a translation product of the gene coding for KCNN3 proteins having SEQ ID NO: 1, SEQ ID NO: 2, SEQ ID NO: 3 or SEQ ID NO: 4, and/or
(iv) a variant of (i) to (iii) comprising a sequence that has at least about 80% sequence identity with the amino acid sequences of said KCNN3 proteins, said method comprises:

(a) contacting a cell with a test compound;
(b) measuring the activity and/or level and/or expression of one or more substances recited in (i) to (iv);
(c) measuring the activity and/or level and/or expression of one or more substances recited in (i) to (iv) in a control cell not contacted with said test compound; and

comparing the levels and/or activities and/or expression of the substances in the cells of step (b) and (c), wherein an alteration in the activity and/or level and/or expression of the substances in the contacted cells indicates that the test compound is an agent, modulator or selective antagonist or agonist for use in the treatment of Alzheimer's disease.

**6.** The method according to claim 5 wherein the KCNN3 protein has an amino acid sequence of SEQ ID NO:1.

**7.** A method of screening for identifying agents, modulators or selective antagonists or agonists for use in the treatment of Alzheimer's disease, which agents, modulators or selective antagonists or agonists have an ability to alter expression or level or activity of one or more substances selected from the group consisting of

(i) the gene coding for KCNN3 proteins having SEQ ID NO: 1, SEQ ID NO: 2, SEQ ID NO: 3 or SEQ ID NO: 4, and/or
(ii) a transcription product of the gene coding for KCNN3 proteins having SEQ ID NO: 1, SEQ ID NO: 2, SEQ ID NO: 3 or SEQ ID NO: 4, and/or
(iii) a translation product of the gene coding for KCNN3 proteins having SEQ ID NO: 1, SEQ ID NO: 2, SEQ ID NO: 3 or SEQ ID NO: 4, and/or
(iv) a variant of (i) to (iii) comprising a sequence that has at least about 80% sequence identity with the amino acid sequences of said KCNN3 proteins,

said method comprises:

(a) administering a test compound to a non-human test animal which is predisposed to developing or has already developed signs and symptoms of Alzheimer's disease;
(b) measuring the activity and/or level and/or expression of one or more substances recited in (i) to (iv);
(c) measuring the activity and/or level and/or expression of one or more substances recited in (i) or (iv) in a non-human control animal which is predisposed to developing or has already developed signs and symptoms of Alzheimer's disease and to which non-human animal no such test compound has been administered;
(d) comparing the activity and/or level and/or expression of the substances in the animals of step (b) and (c), wherein an alteration in the activity and/or level and/or expression of substances in the non-human test animal indicates that the test compound is an agent, modulator or selective antagonist or agonist for use in the treatment of Alzheimer's disease.

**8.** The method of claim 7 wherein the KCNN3 protein has an amino acid sequence of SEQ ID NO: 1.

**9.** The use of an antibody specifically immunoreactive with an immunogen, wherein said immunogen are translation products of a gene coding for KCNN3 having SEQ ID NO: 1, SEQ ID NO: 2, SEQ ID NO: 3 or SEQ ID NO: 4, or a variant thereof comprising a sequence that has at least about 80% sequence identity with the amino acid sequences of said KCNN3 proteins, for detecting the pathological state of a cell in a brain tissue sample obtained from a subject, comprising immunocytochemical staining of said cell with said antibody, wherein an altered degree of staining, or an altered staining pattern in said cell compared to a cell representing a known health status indicates a pathological state of said cell which is Alzheimer's disease.

**10.** The use of claim 9 wherein the KCNN3 has an amino acid sequence of SEQ ID NO: 1.

**Patentansprüche**

**1.** Verfahren zum Diagnostizieren der Alzheimer-Krankheit bei einem Patienten oder zum Überwachen der Wirkung einer Behandlung, die einem Patienten mit dieser Krankheit zuteil wird, wobei das Verfahren die folgenden Schritte umfasst:

(a) Bestimmen eines Pegels und/oder einer Aktivität von (i) einem Transcriptionsprodukt des Gens, das für KCNN3-Proteine codiert, die SEQ ID Nr. 1, SEQ ID Nr. 2, SEQ ID Nr. 3 oder SEQ ID Nr. 4 entsprechen, und/oder (ii) einem Translationsprodukt des Gens, das für KCNN3-Proteine codiert, die SEQ ID Nr. 1, SEQ ID Nr. 2, SEQ ID Nr. 3 oder SEQ ID Nr. 4 entsprechen, und/oder einer Variante des Transcriptions- oder Translationsprodukts, die eine Sequenz umfasst, welche wenigstens etwa 80% Sequenzidentität mit den Aminosäuresequenzen der KCNN3-Proteine aufweist, in einer von dem Patienten erhaltenen Gehirngewebeprobe;
(b) Vergleichen des Pegels und/oder der Aktivität des Transcriptionsprodukts und/oder des Translationsprodukts mit einem Referenzwert, der einen bekannten Krankheitszustand darstellt und/oder einen bekannten Gesundheitszustand darstellt und/oder ein bekanntes Braak-Stadium darstellt;
(c) Analysieren, ob der Pegel und/oder die Aktivität im Vergleich zu einem Referenzwert, der einen bekannten Gesundheitszustand darstellt, verändert ist und/oder ähnlich oder gleich ist wie ein Referenzwert, der einen bekannten Krankheitszustand der Alzheimer-Krankheit darstellt oder ein bekanntes Braak-Stadium darstellt, was darauf hinweist, dass der Patient an Alzheimer-Krankheit leidet oder dass der Patient ein erhöhtes Risiko hat, diese Krankheit zu entwickeln.

**2.** Verfahren gemäß Anspruch 1, wobei das Gen, das für KCNN3-Proteine codiert, das Gen ist, das für KCNN3 mit SEQ ID Nr. 1 codiert, und wobei das Translationsprodukt eines Gens, das für KCNN3-Proteine codiert, das KCNN3-Protein mit SEQ ID Nr. 1 ist.

**3.** Verwendung eines Kits zum Diagnostizieren der Alzheimer-Krankheit bei einem Patienten oder zum Überwachen der Wirkung einer Behandlung, die einem Patienten mit Alzheimer-Krankheit zuteil wird, gemäß dem Verfahren von Anspruch 1, wobei der Kit wenigstens ein Reagens umfasst, das aus der Gruppe ausgewählt ist, die aus (i) einem Transcriptionsprodukt des Gens, das für KCNN3-Proteine codiert, die SEQ ID Nr. 1, SEQ ID Nr. 2, SEQ ID Nr. 3 oder SEQ ID Nr. 4 entsprechen, und/oder (ii) einem Translationsprodukt des Gens, das für KCNN3-Proteine codiert, die SEQ ID Nr. 1, SEQ ID Nr. 2, SEQ ID Nr. 3 oder SEQ ID Nr. 4 entsprechen, und/oder (iii) einer Variante des Transcriptions- oder Translationsprodukts, die eine Sequenz umfasst, welche wenigstens etwa 80% Sequenzidentität mit den Aminosäuresequenzen der KCNN3-Proteine aufweist.

**4.** Verwendung gemäß Anspruch 3, wobei das KCNN3-Protein die SEQ ID Nr. 1 aufweist.

**5.** Verfahren zum Screening nach Mitteln, Modulatoren oder selektiven Antagonisten oder Agonisten zur Verwendung bei der Behandlung von Alzheimer-Krankheit, wobei die Mittel, Modulatoren oder selektiven Antagonisten oder Agonisten die Fähigkeit haben, die Expression oder den Pegel oder die Aktivität von einer oder mehreren Substanzen zu verändern, die aus der Gruppe ausgewählt sind, die aus Folgenden besteht:

(i) einem Gen, das für KCNN3-Proteine codiert, die SEQ ID Nr. 1, SEQ ID Nr. 2, SEQ ID Nr. 3 oder SEQ ID Nr. 4 entsprechen; und/oder
(ii) einem Transcriptionsprodukt des Gens, das für KCNN3-Proteine codiert, die SEQ ID Nr. 1, SEQ ID Nr. 2, SEQ ID Nr. 3 oder SEQ ID Nr. 4 entsprechen; und/oder
(iii) einem Translationsprodukt des Gens, das für KCNN3-Proteine codiert, die SEQ ID Nr. 1, SEQ ID Nr. 2, SEQ ID Nr. 3 oder SEQ ID Nr. 4 entsprechen; und/oder
(iv) einer Variante von (i) bis (iii), die eine Sequenz umfasst, welche wenigstens etwa 80% Sequenzidentität mit den Aminosäuresequenzen der KCNN3-Proteine aufweist;

wobei das Verfahren Folgendes umfasst:

(a) In-Kontakt-Bringen einer Zelle mit einer Testverbindung;
(b) Messen der Aktivität und/oder des Pegels und/oder der Expression von einer oder mehreren Substanzen, die in (i) bis (iv) genannt sind;
c) Messen der Aktivität und/oder des Pegels und/oder der Expression von einer oder mehreren Substanzen, die in (i) bis (iv) genannt sind, in einer Kontrollzelle, die nicht mit der Testverbindung in Kontakt gebracht wurde; und

Vergleichen der Pegel und/oder Aktivitäten und/oder der Expression der Substanzen in den Zellen der Schritte (b) und (c), wobei eine Änderung der Aktivität und/oder des Pegels und/oder der Expression der Substanzen in den kontaktierten Zellen anzeigt, dass die Testverbindung ein Mittel, Modulator oder ein selektiver Antagonist oder Agonist zur Verwendung bei der Behandlung der Alzheimer-Krankheit ist.

6. Verfahren gemäß Anspruch 5, wobei das KCNN3-Proteine eine Aminosäuresequenz von SEQ ID Nr. 1 aufweist.

7. Verfahren zum Screening nach Mitteln, Modulatoren oder selektiven Antagonisten oder Agonisten zur Verwendung bei der Behandlung von Alzheimer-Krankheit, wobei die Mittel, Modulatoren oder selektiven Antagonisten oder Agonisten die Fähigkeit haben, die Expression oder den Pegel oder die Aktivität von einer oder mehreren Substanzen zu verändern, die aus der Gruppe ausgewählt sind, die aus Folgenden besteht:

(i) dem Gen, das für KCNN3-Proteine codiert, die SEQ ID Nr. 1, SEQ ID Nr. 2, SEQ ID Nr. 3 oder SEQ ID Nr. 4 entsprechen; und/oder
(ii) einem Transcriptionsprodukt des Gens, das für KCNN3-Proteine codiert, die SEQ ID Nr. 1, SEQ ID Nr. 2, SEQ ID Nr. 3 oder SEQ ID Nr. 4 entsprechen; und/oder
(iii) einem Translationsprodukt des Gens, das für KCNN3-Proteine codiert, die SEQ ID Nr. 1, SEQ ID Nr. 2, SEQ ID Nr. 3 oder SEQ ID Nr. 4 entsprechen; und/oder
(iv) einer Variante von (i) bis (iii), die eine Sequenz umfasst, welche wenigstens etwa 80% Sequenzidentität mit den Aminosäuresequenzen der KCNN3-Proteine aufweist;

wobei das Verfahren Folgendes umfasst:

(a) Verabreichen einer Testverbindung an ein nichthumanes Versuchstier, das prädisponiert ist, Alzheimer-Krankheit zu entwickeln, oder das bereits Anzeichen und Symptome der Alzheimer-Krankheit entwickelt hat;
(b) Messen der Aktivität und/oder des Pegels und/oder der Expression von einer oder mehreren Substanzen, die in (i) bis (iv) genannt sind;
(c) Messen der Aktivität und/oder des Pegels und/oder der Expression von einer oder mehreren Substanzen, die in (i) bis (iv) genannt sind, bei einem nichthumanen Versuchstier, das prädisponiert ist, Alzheimer-Krankheit zu entwickeln, oder das bereits Anzeichen und Symptome der Alzheimer-Krankheit entwickelt hat, wobei diesem nichthumanen Tier keine solche Testverbindung verabreicht wurde;
(d) Vergleichen der Aktivität und/oder des Pegels und/oder der Expression der Substanzen in den Tieren der Schritte (b) und (c), wobei eine Änderung der Aktivität und/oder des Pegels und/oder der Expression der Substanzen in dem nichthumanen Versuchstier anzeigt, dass die Testverbindung ein Mittel, Modulator oder ein selektiver Antagonist oder Agonist zur Verwendung bei der Behandlung der Alzheimer-Krankheit ist.

8. Verfahren gemäß Anspruch 7, wobei das KCNN3-Proteine eine Aminosäuresequenz von SEQ ID Nr. 1 aufweist.

9. Verwendung eines Antikörpers, der spezifisch immunreaktiv gegenüber einem Immunogen ist, wobei es sich bei dem Immunogen um Translationsprodukte eines Gens, das für KCNN3-Proteine codiert, die SEQ ID Nr. 1, SEQ ID Nr. 2, SEQ ID Nr. 3 oder SEQ ID Nr. 4 entsprechen, oder eine Variante davon, die eine Sequenz umfasst, welche wenigstens etwa 80% Sequenzidentität mit den Aminosäuresequenzen der KCNN3-Proteine aufweist, handelt, zum Nachweisen des pathologischen Zustands einer Zelle in einer Gehirngewebeprobe, die von einem Patienten erhalten wurde, umfassend das immuncytochemische Anfärben der Zelle mit dem Antikörper, wobei ein veränderter Grad der Anfärbung oder ein verändertes Anfärbungsmuster in der Zelle im Vergleich zu einer Zelle, die einen bekannten Gesundheitszustand darstellt, einen pathologischen Zustand der Zelle anzeigt, bei dem es sich um die Alzheimer-Krankheit handelt.

10. Verwendung gemäß Anspruch 9, wobei das KCNN3-Proteine eine Aminosäuresequenz von SEQ ID Nr. 1 aufweist.

**Revendications**

1. Procédé de diagnostic de la maladie d'Alzheimer chez un sujet, ou de contrôle de l'effet d'un traitement administré à un sujet atteint de ladite maladie, lequel procédé comprend les étapes consistant à :

(a) déterminer un taux et/ou une activité de (i) un produit de transcription du gène codant pour des protéines KCNN3 ayant SEQ ID NO : 1, SEQ ID NO : 2, SEQ ID NO : 3 ou SEQ ID NO : 4, et/ou (ii) un produit de traduction du gène codant pour des protéines KCNN3 ayant SEQ ID NO : 1, SEQ ID NO : 2, SEQ ID NO : 3 ou SEQ ID NO : 4, et/ou (iii) un variant dudit produit de transcription ou de traduction comprenant une séquence qui a au moins environ 80 % d'identité de séquence avec les séquences d'acides aminés desdites protéines KCNN3 dans un échantillon de tissu de cerveau obtenu auprès dudit sujet,
(b) comparer ledit niveau et/ou ladite activité dudit produit de transcription et/ou dudit produit de traduction à une valeur de référence représentant un état de maladie connu et/ou représentant un état de santé connu et/ou représentant un stade de Braak connu
(c) analyser si ledit taux et/ou ladite activité sont altérés par rapport à une valeur de référence représentant un état de santé connu, et/ou est similaire ou égal à une valeur de référence représentant un état de maladie connu ou représentant un stade de Braak connu qui est une indication selon laquelle ledit sujet a la maladie d'Alzheimer, ou selon laquelle ledit sujet présente un risque accru de développer ladite maladie.

**2.** Procédé selon la revendication 1, dans lequel ledit gène codant pour des protéines KCNN3 est le gène codant pour KCNN3 ayant SEQ ID NO : 1 et dans lequel ledit produit de traduction d'un gène codant pour des protéines KCNN3 est la protéine KCNN3 ayant SEQ ID NO : 1.

**3.** Utilisation d'un nécessaire pour diagnostiquer la maladie d'Alzheimer chez un sujet, ou contrôler l'effet d'un traitement administré à un sujet atteint de la maladie d'Alzheimer selon le procédé de la revendication 1, dans laquelle le nécessaire comprend au moins un réactif qui est choisi dans le groupe constitué par (i) un produit de transcription du gène codant pour des protéines KCNN3 ayant SEQ ID NO : 1, SEQ ID NO : 2, SEQ ID NO : 3 ou SEQ ID NO : 4, et/ou (ii) un produit de traduction du gène codant pour des protéines KCNN3 ayant SEQ ID NO : 1, SEQ ID NO : 2, SEQ ID NO : 3 ou SEQ ID NO : 4, et/ou (iii) un variant dudit produit de transcription ou de traduction comprenant une séquence qui a au moins environ 80 % d'identité de séquence avec les séquences d'acides aminés desdites protéines KCNN3.

**4.** Utilisation selon la revendication 3, dans laquelle la protéine KCNN3 a la SEQ ID NO : 1.

**5.** Procédé de criblage pour identifier des agents, des modulateurs ou des antagonistes ou agonistes sélectifs à utiliser dans le traitement de la maladie d'Alzheimer, lesquels agents, modulateurs ou antagonistes ou agonistes sélectifs ont une capacité à altérer l'expression ou le taux ou l'activité d'une ou plusieurs substances choisies dans le groupe constitué par

(i) un gène codant pour des protéines KCNN3 ayant SEQ ID NO : 1, SEQ ID NO : 2, SEQ ID NO : 3 ou SEQ ID NO : 4, et/ou
(ii) un produit de transcription du gène codant pour des protéines KCNN3 ayant SEQ ID NO : 1, SEQ ID NO : 2, SEQ ID NO : 3 ou SEQ ID NO : 4, et/ou
(iii) un produit de traduction du gène codant pour des protéines KCNN3 ayant SEQ ID NO : 1, SEQ ID NO : 2, SEQ ID NO : 3 ou SEQ ID NO : 4, et/ou
(iv) un variant de (i) à (iii) comprenant une séquence qui a au moins environ 80 % d'identité de séquence avec les séquences d'acides aminés desdites protéines KCNN3, ledit procédé comprenant les étapes consistant à :

(a) mettre en contact une cellule avec un composé d'essai ;
(b) mesurer l'activité et/ou le taux et/ou l'expression d'une ou plusieurs substances précisées dans (i) à (iv) ;
(c) mesurer l'activité et/ou le taux et/ou l'expression d'une ou plusieurs substances précisées dans (i) à (iv) dans une cellule témoin non mise en contact avec ledit composé d'essai ; et

comparer les taux et/ou activité et/ou expression des substances dans les cellules des étapes (b) et (c), où une altération de l'activité et/ou du taux et/ou de l'expression des substances dans les cellules mises en contact indique que le composé d'essai est un agent, un modulateur, un antagoniste ou agoniste sélectif à utiliser dans le traitement de la maladie d'Alzheimer.

**6.** Procédé selon la revendication 5, dans lequel la protéine KCNN3 a une séquence d'acides aminés de SEQ ID NO : 1.

**7.** Procédé de criblage pour identifier des agents, des modulateurs ou des antagonistes ou agonistes sélectifs à utiliser dans le traitement de la maladie d'Alzheimer, lesquels agents, modulateurs ou antagonistes ou agonistes sélectifs ont une capacité à altérer l'expression ou le taux ou l'activité d'une ou plusieurs substances choisies dans le groupe constitué par

(i) le gène codant pour des protéines KCNN3 ayant SEQ ID NO : 1, SEQ ID NO : 2, SEQ ID NO : 3 ou SEQ ID NO : 4, et/ou
(ii) un produit de transcription du gène codant pour des protéines KCNN3 ayant SEQ ID NO : 1, SEQ ID NO : 2, SEQ ID NO : 3 ou SEQ ID NO : 4, et/ou
(iii) un produit de traduction du gène codant pour des protéines KCNN3 ayant SEQ ID NO : 1, SEQ ID NO : 2, SEQ ID NO : 3 ou SEQ ID NO : 4, et/ou
(iv) un variant de (i) à (iii) comprenant une séquence qui a au moins environ 80 % d'identité de séquence avec les séquences d'acides aminés desdites protéines KCNN3, ledit procédé comprenant les étapes consistant à :

(a) administrer un composé d'essai à un animal d'essai non humain qui est prédisposé à développer ou a déjà développé des signes et symptômes de la maladie d'Alzheimer ;
(b) mesurer l'activité et/ou le taux et/ou l'expression d'une ou plusieurs substances précisées dans (i) à (iv) ;
(c) mesurer l'activité et/ou le taux et/ou l'expression d'une ou plusieurs substances précisées dans (i) ou (iv) chez un animal témoin non humain qui est prédisposé à développer ou a déjà développé des signes et symptômes de la maladie d'Alzheimer et auquel animal non humain aucun composé d'essai de ce genre n'a été administré ;
(d) comparer l'activité et/ou le taux et/ou l'expression des substances chez les animaux de l'étape (b) et (c), où une altération de l'activité et/ou du taux et/ou de l'expression de substances dans l'animal d'essai non humain indique que le composé d'essai est un agent, un modulateur ou un antagoniste ou agoniste sélectif à utiliser dans le traitement de la maladie d'Alzheimer.

**8.** Procédé selon la revendication 7, dans lequel la protéine KCNN3 a une séquence d'acides aminés de SEQ ID NO : 1.

**9.** Utilisation d'un anticorps spécifiquement immunoréactif avec un immunogène, ledit immunogène étant composé de produits de traduction d'un gène codant pour KCNN3 ayant SEQ ID NO : 1, SEQ ID NO : 2, SEQ ID NO : 3 ou SEQ ID NO : 4, ou un variant de celui-ci comprenant une séquence qui a au moins environ 80 % d'identité de séquence avec les séquences d'acides aminés desdites protéines KCNN3, pour détecter l'état pathologique d'une cellule dans un échantillon de tissu de cerveau obtenu auprès d'un sujet, comprenant la coloration immunocytochimique de ladite cellule avec ledit anticorps, utilisation dans laquelle un degré de coloration altéré, ou un motif de coloration altéré dans ladite cellule par rapport à une cellule représentant un état de santé connu indique un état pathologique de ladite cellule qui est la maladie d'Alzheimer.

**10.** Utilisation selon la revendication 9, dans laquelle la KCNN3 a une séquence d'acides aminés de SEQ ID NO : 1.

# Figure 1: Identification of the differentially expressed gene KCNN3 by GeneChip microarray analysis

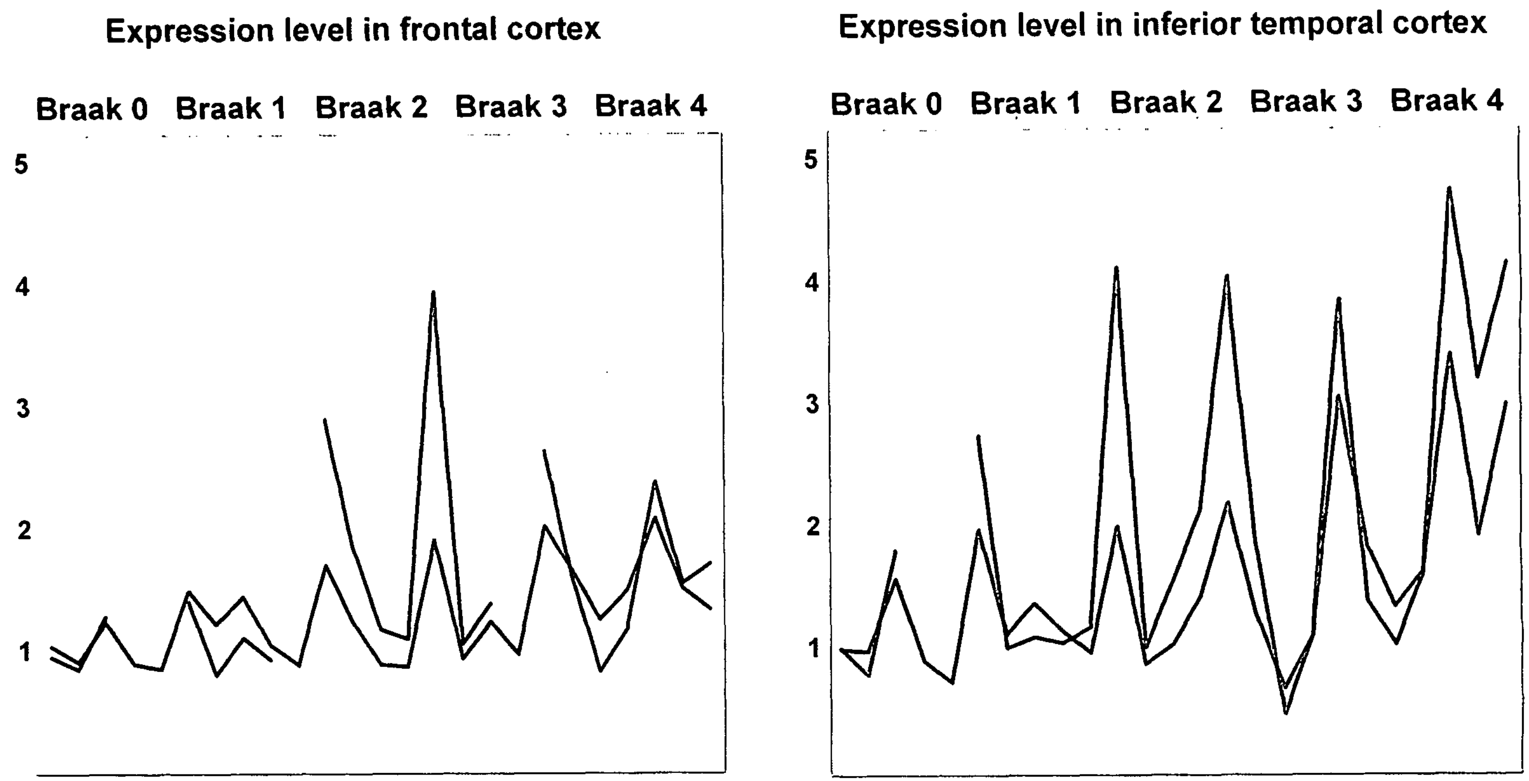

# Figure 2: Analysis of KCNN3 mRNA expression by quantitative PCR

measured data of KCNN3 mRNA expression in frontal and inferior temporal cortices in Braak stages 0 – 4

| Donor | Braak stage | Frontal | Temporal |
|---|---|---|---|
| C011 | 0 | 516.8716 | 563.2406 |
| C012 | 0 | 496.8153 | 570.1528 |
| C026 | 0 | 596.6898 | 556.0100 |
| C027 | 0 | 305.9391 | 545.3909 |
| C032 | 0 | 268.4835 | 465.3564 |
| C014 | 1 | 506.4116 | 1277.2774 |
| C028 | 1 | 641.2423 | 629.8927 |
| C029 | 1 | 675.3187 | 564.1561 |
| C030 | 1 | 746.8901 | 893.9762 |
| C036 | 1 | 486.4055 | 696.4119 |
| C038 | 1 | 905.7669 | 1361.0273 |
| C039 | 1 | 902.7100 | 884.3809 |
| C008 | 2 | 530.9105 | 870.7941 |
| C031 | 2 | 479.1762 | 1081.0830 |
| C033 | 2 | 1202.2179 | 702.7030 |
| C034 | 2 | 600.9262 | 729.5211 |
| DE03 | 2 | 548.8456 | 383.5730 |
| C025 | 3 | 537.9144 | 744.1438 |
| DE07 | 3 | 785.6757 | 1062.2276 |
| DE11 | 3 | 772.3749 | 833.4045 |
| C057 | 3 | 626.3360 | 765.6023 |
| P012 | 4 | 1036.7775 | 1259.7413 |
| P046 | 4 | 1320.4712 | 1684.1263 |
| P047 | 4 | 535.4992 | 879.8946 |
| P068 | 4 | 468.7702 | 1042.1472 |

# Figure 3: Statistical analysis of differential KCNN3 mRNA expression

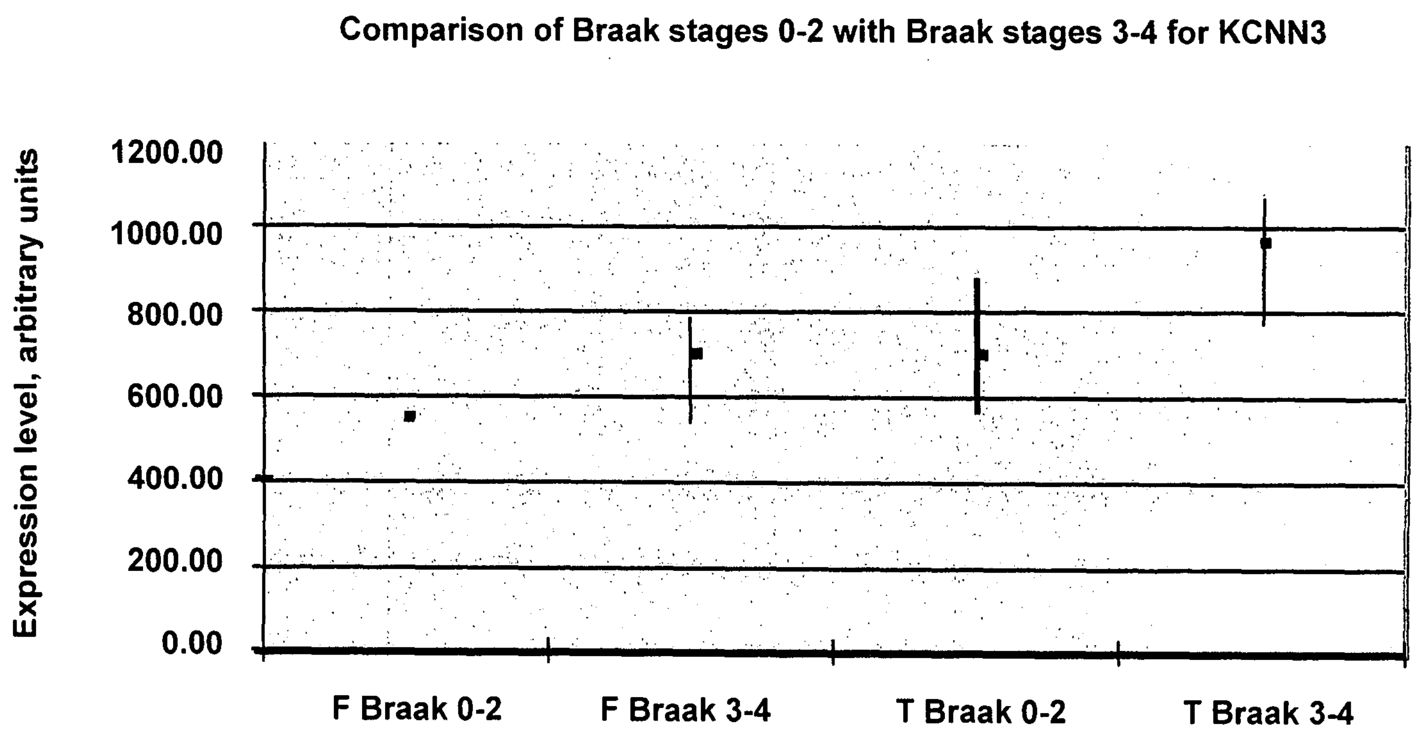

# Figure 4 : A) SEQ ID NO: 1, amino acid sequence of human KCNN3 sv1 protein

**Length: 736 aa**

```
  1 MDTSGHFHDS GVGDLDEDPK CPCPSSGDEQ QQQQQQQQQQ QPPPPAPPAA
 51 PQQPLGPSLQ PQPPQLQQQQ QQQQQQQQQQ QQQQQPPHPL SQLAQLQSQP
101 VHPGLLHSSP TAFRAPPSSN STAILHPSSR QGSQLNLNDH LLGHSPSSTA
151 TSGPGGGSRH RQASPLVHRR DSNPFTEIAM SSCKYSGGVM KPLSRLSASR
201 RNLIEAETEG QPLQLFSPSN PPEIVISSRE DNHAHQTLLH HPNATHNHQH
251 AGTTASSTTF PKANKRKNQN IGYKLGHRRA LFEKRKRLSD YALIFGMFGI
301 VVMVIETELS WGLYSKDSMF SLALKCLISL STIILLGLII AYHTREVQLF
351 VIDNGADDWR IAMTYERILY ISLEMLVCAI HPIPGEYKFF WTARLAFSYT
401 PSRAEADVDI ILSIPMFLRL YLIARVMLLH SKLFTDASSR SIGALNKINF
451 NTRFVMKTLM TICPGTVLLV FSISLWIIAA WTVRVCERYH DQQDVTSNFL
501 GAMWLISITF LSIGYGDMVP HTYCGKGVCL LTGIMGAGCT ALVVAVVARK
551 LELTKAEKHV HNFMMDTQLT KRIKNAAANV LRETWLIYKH TKLLKKIDHA
601 KVRKHQRKFL QAIHQLRSVK MEQRKLSDQA NTLVDLSKMQ NVMYDLITEL
651 NDRSEDLEKQ IGSLESKLEH LTASFNSLPL LIADTLRQQQ QQLLSAIIEA
701 RGVSVAVGTT HTPISDSPIG VSSTSFPTPY TSSSSC
```

# Figure 4 : B) SEQ ID NO: 2, amino acid sequence of human KCNN3 sv2 protein

**Length: 731 aa**

```
  1  MDTSGHFHDS GVGDLDEDPK CPCPSSGDEQ QQQQQQQQQQ QPPPPAPPAA
 51  PQQPLGPSLQ PQPPQLQQQQ QQQQQQQQQQ PPHPLSQLAQ LQSQPVHPGL
101  LHSSPTAFRA PPSSNSTAIL HPSSRQGSQL NLNDHLLGHS PSSTATSGPG
151  GGSRHRQASP LVHRRDSNPF TEIAMSSCKY SGGVMKPLSR LSASRRNLIE
201  AETEGQPLQL FSPSNPPEIV ISSREDNHAH QTLLHHPNAT HNHQHAGTTA
251  SSTTFPKANK RKNQNIGYKL GHRRALFEKR KRLSDYALIF GMFGIVVMVI
301  ETELSWGLYS KDSMFSLALK CLISLSTIIL LGLIIAYHTR EVQLFVIDNG
351  ADDWRIAMTY ERILYISLEM LVCAIHPIPG EYKFFWTARL AFSYTPSRAE
401  ADVDIILSIP MFLRLYLIAR VMLLHSKLFT DASSRSIGAL NKINFNTRFV
451  MKTLMTICPG TVLLVFSISL WIIAAWTVRV CERYHDQQDV TSNFLGAMWL
501  ISITFLSIGY GDMVPHTYCG KGVCLLTGIM GAGCTALVVA VVARKLELTK
551  AEKHVHNFMM DTQLTKRIKN AAANVLRETW LIYKHTKLLK KIDHAKVRKH
601  QRKFLQAIHQ LRSVKMEQRK LSDQANTLVD LSKMQNVMYD LITELNDRSE
651  DLEKQIGSLE SKLEHLTASF NSLPLLIADT LRQQQQLLS AIIEARGVSV
701  AVGTTHTPIS DSPIGVSSTS FPTPYTSSSS C
```

# Figure 4 : C) SEQ ID NO: 3, amino acid sequence of human KCNN3 sv3 protein

**Length: 426 aa**

```
  1  IAMTYERILY ISLEMLVCAI HPIPGEYKFF WTARLAFSYT PSRAEADVDI
101  ILSIPMFLRL YLIARVMLLH SKLFTDASSR SIGALNKINF NTRFVMKTLM
151  TICPGTVLLV FSISLWIIAA WTVRVCERYH DQQDVTSNFL GAMWLISITF
201  LSIGYGDMVP HTYCGKGVCL LTGIMGAGCT ALVVAVVARK LELTKAEKHV
251  HNFMMDTQLT KRIKNAAANV LRETWLIYKH TKLLKKIDHA KVRKHQRKFL
301  QAIHQLRSVK MEQRKLSDQA NTLVDLSKMQ NVMYDLITEL NDRSEDLEKQ
351  IGSLESKLEH LTASFNSLPL LIADTLRQQQ QQLLSAIIEA RGVSVAVGTT
401  HTPISDSPIG VSSTSFPTPY TSSSSC
```

# Figure 4 : D) SEQ ID NO: 4, amino acid sequence of human KCNN3 sv4 protein

**Length: 418 aa**

```
  1  MFSLALKCLI SLSTIILLGL IIAYHTREVQ LFVIDNGADD WRIAMTYERI
 51  LYISLEMLVC AIHPIPGEYK FFWTARLAFS YTPSRAEADV DIILSIPMFL
101  RLYLIARVML LHSKLFTDAS SRSIGALNKI NFNTRFVMKT LMTICPGTVL
151  LVFSISLWII AAWTVRVCER YHDQQDVTSN FLGAMWLISI TFLSIGYGDM
201  VPHTYCGKGV CLLTGIMGAG CTALVVAVVA RKLELTKAEK HVHNFMMDTQ
251  LTKRIKNAAA NVLRETWLIY KHTKLLKKID HAKVRKHQRK FLQAIHQLRS
301  VKMEQRKLSD QANTLVDLSK MQNVMYDLIT ELNDRSEDLE KQIGSLESKL
351  EHLTASFNSL PLLIADTLRQ QQQLLSAII EARGVSVAVG TTHTPISDSP
401  IGVSSTSFPT PYTSSSSC
```

# Figure 5 : A) SEQ ID NO: 5, nucleotide sequence of human KCNN3 sv1 cDNA

**Length: 3095 bp**

```
   1  GGGTCGACCC ACGCGTCCGG AGCCAGCGAG GAGTGAAGCT GAGCCTGGCC
  51  TCACACGCTC CTAGAGGACC ACCTCCTGAG AGAGTTCTTT CACCCCCTCT
 101  TCTTTCTCCA AGCTCCCCTC CTGCTCTCCC TCCCTGCCCA ATACAATGCA
 151  TTCTTGAGTG GCAGCGTCTG GACTCCAGGC AGCCCCAGAG AACCGAAGCA
 201  AGCCAAAGAG AGGACTGGAG CCAAGATACT GGTGGGGGAG ATTGGATGCC
 251  TGGCTTTCTT TGAGGACATC TTTGGAGCGA GGGTGGCTTT GGGGTGGGGG
 301  CTTGTGCTGC AGGGAATACA GCCAGGCCCC AAGATGGACA CTTCTGGGCA
 351  CTTCCATGAC TCGGGGGTGG GGGACTTGGA TGAAGACCCC AAGTGCCCCT
 401  GTCCATCCTC TGGGGATGAG CAGCAGCAGC AGCAGCAGCA GCAACAGCAG
 451  CAGCAGCCAC CACCGCCAGC GCCACCAGCA GCCCCCCAGC AGCCCCTGGG
 501  ACCCTCGCTG CAGCCTCAGC CTCCGCAGCT TCAGCAGCAG CAGCAGCAGC
 551  AGCAGCAGCA GCAGCAGCAG CAGCAGCAGC AGCAGCAGCC ACCGCATCCC
 601  CTGTCTCAGC TCGCCCAACT CCAGAGCCAG CCCGTCCACC CTGGCCTGCT
 651  GCACTCCTCT CCCACCGCTT TCAGGGCCCC CCCTTCGTCC AACTCCACCG
 701  CCATCCTCCA CCCTTCCTCC AGGCAAGGCA GCCAGCTCAA TCTCAATGAC
 751  CACTTGCTTG GCCACTCTCC AAGTTCCACA GCTACAAGTG GGCCTGGCGG
 801  AGGCAGCCGG CACCGACAGG CCAGCCCCCT GGTGCACCGG CGGGACAGCA
 851  ACCCCTTCAC GGAGATCGCC ATGAGCTCCT GCAAGTATAG CGGTGGGGTC
 901  ATGAAGCCCC TCAGCCGCCT CAGCGCCTCC CGGAGGAACC TCATCGAGGC
 951  CGAGACTGAG GGCCAACCCC TCCAGCTTTT CAGCCCTAGC AACCCCCCGG
1001  AGATCGTCAT CTCCTCCCGG GAGGACAACC ATGCCCACCA GACCCTGCTC
1051  CATCACCCTA ATGCCACCCA CAACCACCAG CATGCCGGCA CCACCGCCAG
1101  CAGCACCACC TTCCCCAAAG CCAACAAGCG GAAAAACCAA AACATTGGCT
1151  ATAAGCTGGG ACACAGGAGG GCCCTGTTTG AAAAGAGAAA GCGACTGAGT
1201  GACTATGCTC TGATTTTTGG GATGTTTGGA ATTGTTGTTA TGGTGATAGA
1251  GACCGAGCTC TCTTGGGGTT TGTACTCAAA GGACTCCATG TTTTCGTTGG
1301  CCCTGAAATG CCTTATCAGT CTGTCCACCA TCATCCTTTT GGGCTTGATC
1351  ATCGCCTACC ACACACGTGA AGTCCAGCTC TTCGTGATCG ACAACGGCGC
1401  GGATGACTGG CGGATAGCCA TGACCTACGA GCGCATCCTG TACATCAGCC
1451  TGGAGATGCT GGTGTGCGCC ATCCACCCCA TTCCTGGCGA GTACAAGTTC
1501  TTCTGGACGG CACGCCTGGC CTTCTCCTAC ACACCCTCCC GGGCGGAGGC
1551  CGATGTGGAC ATCATCCTGT CTATCCCCAT GTTCCTGCGC CTGTACCTGA
1601  TCGCCCGAGT CATGCTGCTG CACAGCAAGC TCTTCACCGA TGCCTCGTCC
1651  CGCAGCATCG GGGCCCTCAA CAAGATCAAC TTCAACACCC GCTTTGTCAT
1701  GAAGACGCTC ATGACCATCT GCCCTGGCAC TGTGCTGCTC GTGTTCAGCA
1751  TCTCTCTGTG GATCATTGCT GCCTGGACCG TCCGTGTCTG TGAAAGGTAC
1801  CATGACCAGC AGGACGTAAC TAGTAACTTT CTGGGTGCCA TGTGGCTCAT
1851  CTCCATCACA TTCCTTTCCA TTGGTTATGG GGACATGGTG CCCCACACAT
1901  ACTGTGGGAA AGGTGTCTGT CTCCTCACTG GCATCATGGG TGCAGGCTGC
1951  ACTGCCCTTG TGGTGGCCGT GGTGGCCCGA AAGCTGGAAC TCACCAAAGC
2001  GGAGAAGCAC GTTCATAACT TCATGATGGA CACTCAGCTC ACCAAGCGGA
2051  TCAAGAATGC TGCAGCCAAT GTCCTTCGGG AAACATGGTT AATCTATAAA
2101  CACACAAAGC TGCTAAAGAA GATTGACCAT GCCAAAGTGA GGAAACACCA
2151  GAGGAAGTTC CTCCAAGCTA TCCACCAGTT GAGGAGCGTC AAGATGGAAC
```

```
2201  AGAGGAAGCT GAGTGACCAA GCCAACACTC TGGTGGACCT TTCCAAGATG
2251  CAGAATGTCA TGTATGACTT AATCACAGAA CTCAATGACC GGAGCGAAGA
2301  CCTGGAGAAG CAGATTGGCA GCCTGGAGTC GAAGCTGGAG CATCTCACCG
2351  CCAGCTTCAA CTCCCTGCCG CTGCTCATCG CCGACACCCT GCGCCAGCAG
2401  CAGCAGCAGC TCCTGTCTGC CATCATCGAG GCCCGGGGTG TCAGCGTGGC
2451  AGTGGGCACC ACCCACACCC CAATCTCCGA TAGCCCCATT GGGGTCAGCT
2501  CCACCTCCTT CCCGACCCCG TACACAAGTT CAAGCAGTTG CTAAATAAAT
2551  CTCCCCACTC CAGAAGCATT ACCCATAGGT CTTAAGATGC AAATCAACTC
2601  TCTCCTGGTC GCTTTGCCAT CAAGAAACAT TCAGACCAGG GAACGGAAAG
2651  AAGAGAGACC GAGCTAATTA ACTAACTCAT GTTCATTCAG CGTGCTTGGT
2701  CCGACATGCC TTGAAACCAG AAATCTAATC TCTGTTTAGG TGCCTCTACT
2751  TGGGAGCGGG AAGAGGAGAT GACAGGAAGC GACGCCTCTG GCAGGGCCCT
2801  TGCTGCAGAG TTGGTGGAGA ACAGAAATCC ACGCTCAATC TCAGGTCTTC
2851  ACGCGGGGGG TGGGGGTCAG ATGCACTGAA GTAGCCAACA GCGAAACCAG
2901  TCCAGAAGAG GGGTCCGCTG GGAGGGAGGG TTGTGTCAGG CTTGGGGGAT
2951  GGGCTCTTCG CCATGGGGGT CTTTGAACAC ACCTCTCTCC TTTCCTTTTG
3001  TCTACGGAAG CCTCTGGGTG ACAAAAGTAA AAGAGAGCTG CCCACAACTT
3051  GCCAAAACAG ATATACTCGA ATCAGACTGA AAAAAAAAAA AAAAA
```

# Figure 5 : B) SEQ ID NO: 6, nucleotide sequence of human KCNN3 sv2 cDNA

**Length: 2962 bp**

```
   1  TTGAGCCAGC GAGGAGTGAA GCTGAGCCTG GCCTCACACG CTCCTAGAGG
  51  ACCACCTCCT GAGAGAGTTC TTTCACCCCC TCTTCTTTCT CCAAGCTCCC
 101  CTCCTGCTCT CCCTCCCTGC CCAATACAAT GCATTCTTGA GTGGCAGCGT
 151  CTGGACTCCA GGCAGCCCCA GAGAACCGAA GCAAGCCAAA GAGAGGACTG
 201  GAGCCAAGAT ACTGGTGGGG GAGATTGGAT GCCTGGCTTT CTTTGAGGAC
 251  ATCTTTGGAG CGAGGGTGGC TTTGGGGTGG GGGCTTGTGC TGCAGGGAAT
 301  ACAGCCAGGC CCCAAGATGG ACACTTCTGG GCACTTCCAT GACTCGGGGG
 351  TGGGGGACTT GGATGAAGAC CCCAAGTGCC CCTGTCCATC CTCTGGGGAT
 401  GAGCAGCAGC AGCAGCAGCA GCAGCAACAG CAGCAGCAGC CACCACCGCC
 451  AGCGCCACCA GCAGCCCCCC AGCAGCCCCT GGGACCCTCG CTGCAGCCTC
 501  AGCCTCCGCA GCTTCAGCAG CAGCAGCAGC AGCAGCAGCA GCAGCAGCAG
 551  CAGCAGCCAC CGCATCCCCT GTCTCAGCTC GCCCAACTCC AGAGCCAGCC
 601  CGTCCACCCT GGCCTGCTGC ACTCCTCTCC CACCGCTTTC AGGGCCCCCC
 651  CTTCGTCCAA CTCCACCGCC ATCCTCCACC CTTCCTCCAG GCAAGGCAGC
 701  CAGCTCAATC TCAATGACCA CTTGCTTGGC CACTCTCCAA GTTCCACAGC
 751  TACAAGTGGG CCTGGCGGAG GCAGCCGGCA CCGACAGGCC AGCCCCCTGG
 801  TGCACCGGCG GGACAGCAAC CCCTTCACGG AGATCGCCAT GAGCTCCTGC
 851  AAGTATAGCG GTGGGGTCAT GAAGCCCCTC AGCCGCCTCA GCGCCTCCCG
 901  GAGGAACCTC ATCGAGGCCG AGACTGAGGG CCAACCCCTC CAGCTTTTCA
 951  GCCCTAGCAA CCCCCCGGAG ATCGTCATCT CCTCCCGGGA GGACAACCAT
1001  GCCCACCAGA CCCTGCTCCA TCACCCTAAT GCCACCCACA ACCACCAGCA
1051  TGCCGGCACC ACCGCCAGCA GCACCACCTT CCCCAAAGCC AACAAGCGGA
1101  AAAACCAAAA CATTGGCTAT AAGCTGGGAC ACAGGAGGGC CCTGTTTGAA
1151  AAGAGAAAGC GACTGAGTGA CTATGCTCTG ATTTTTGGGA TGTTTGGAAT
1201  TGTTGTTATG GTGATAGAGA CCGAGCTCTC TTGGGGTTTG TACTCAAAGG
1251  ACTCCATGTT TTCGTTGGCC CTGAAATGCC TTATCAGTCT GTCCACCATC
1301  ATCCTTTTGG GCTTGATCAT CGCCTACCAC ACACGTGAAG TCCAGCTCTT
1351  CGTGATCGAC AATGGCGCGG ATGACTGGCG GATAGCCATG ACCTACGAGC
1401  GCATCCTGTA CATCAGCCTG GAGATGCTGG TGTGCGCCAT CCACCCCATT
1451  CCTGGCGAGT ACAAGTTCTT CTGGACGGCA CGCCTGGCCT TCTCCTACAC
1501  ACCCTCCCGG GCGGAGGCCG ATGTGGACAT CATCCTGTCT ATCCCCATGT
1551  TCCTGCGCCT GTACCTGATC GCCCGAGTCA TGCTGCTGCA CAGCAAGCTC
1601  TTCACCGATG CCTCGTCCCG CAGCATCGGG GCCCTCAACA AGATCAACTT
1651  CAACACCCGC TTTGTCATGA AGACGCTCAT GACCATCTGC CCTGGCACTG
1701  TGCTGCTCGT GTTCAGCATC TCTCTGTGGA TCATTGCTGC CTGGACCGTC
1751  CGTGTCTGTG AAAGGTACCA TGACCAGCAG GACGTAACTA GTAACTTTCT
1801  GGGTGCCATG TGGCTCATCT CCATCACATT CCTTTCCATT GGTTATGGGG
1851  ACATGGTGCC CCACACATAC TGTGGGAAAG GTGTCTGTCT CCTCACTGGC
1901  ATCATGGGTG CAGGCTGCAC TGCCCTTGTG GTGGCCGTGG TGGCCCGAAA
1951  GCTGGAACTC ACCAAAGCGG AGAAGCACGT TCATAACTTC ATGATGGACA
2001  CTCAGCTCAC CAAGCGGATC AAGAATGCTG CAGCCAATGT CCTTCGGGAA
2051  ACATGGTTAA TCTATAAACA CACAAAGCTG CTAAAGAAGA TTGACCATGC
2101  CAAAGTGAGG AAACACCAGA GGAAGTTCCT CCAAGCTATC CACCAGTTGA
2151  GGAGCGTCAA GATGGAACAG AGGAAGCTGA GTGACCAAGC CAACACTCTG
```

```
2201  GTGGACCTTT CCAAGATGCA GAATGTCATG TATGACTTAA TCACAGAACT
2251  CAATGACCGG AGCGAAGACC TGGAGAAGCA GATTGGCAGC CTGGAGTCGA
2301  AGCTGGAGCA TCTCACCGCC AGCTTCAACT CCCTGCCGCT GCTCATCGCC
2351  GACACCCTGC GCCAGCAGCA GCAGCAGCTC CTGTCTGCCA TCATCGAGGC
2401  CCGGGGTGTC AGCGTGGCAG TGGGCACCAC CCACACCCCA ATCTCCGATA
2451  GCCCCATTGG GGTCAGCTCC ACCTCCTTCC CGACCCCGTA CACAAGTTCA
2501  AGCAGTTGCT AAATAAATCT CCCCACTCCA GAAGCATTAC CCATAGGTCT
2551  TAAGATGCAA ATCAACTCTC TCCTGGTCGC TTTGCCATCA AGAAACATTC
2601  AGACCAGGGA ACGGAAAGAA GAGAGACCGA GCTAATTAAC TAACTCATGT
2651  TCATTCAGCG TGCTTGGTCC GACATGCCTT GAAACCAGAA ATCTAATCTC
2701  TGTTTAGGTG CCTCTACTTG GGAGCGGGAA GAGGAGATGA CAGGAAGCGA
2751  CGCCTCTGGC AGGGCCCTTG CTGCAGAGTT GGTGGAGAAC AGAAATCCAC
2801  GCTCAATCTC AGGTCTTCAC GCGGGGGGTG GGGGTCAGAT GCACTGAAGT
2851  AGCCAACAGC GAAGCCAGTC CAGAAGAGGG GTCCGCTGGG AGGGAGGGTT
2901  GTGTCAGGCT TGGGGGATGG GCTCTTCGCC ATGGGGGTCT TTGAACACAC
2951  CTCTCTCCTT TC
```

# Figure 5 : C) SEQ ID NO: 7, nucleotide sequence of human KCNN3 sv3 cDNA

**Length: 1966 bp**

```
   1 CTGCTCAGTG TCAAATTAAC AGGAAAGTCA GCTTAAAGGA CACTCCTTGC
  51 AGGGACTGAG CTGGCACCTA CTCCTTAGAG CTTGCTGATA CCAGGCCTGC
 101 CACGCGACAT CTGCAAGGAC AGTTGTTTGG TGTTTTGCTT CAGGTTATAG
 151 ATGGAGAGAC CTATAAAGGA CTCCATGTTT TCGTTGGCCC TGAAATGCCT
 201 TATCAGTCTG TCCACCATCA TCCTTTTGGG CTTGATCATC GCCTACCACA
 251 CACGTGAAGT CCAGCTCTTC GTGATCGACA ATGGCGCGGA TGACTGGCGG
 301 ATAGCCATGA CCTACGAGCG CATCCTGTAC ATCAGCCTGG AGATGCTGGT
 351 GTGCGCCATC CACCCCATTC CTGGCGAGTA CAAGTTCTTC TGGACGGCAC
 401 GCCTGGCCTT CTCCTACACA CCCTCCCGGG CGGAGGCCGA TGTGGACATC
 451 ATCCTGTCTA TCCCCATGTT CCTGCGCCTG TACCTGATCG CCCGAGTCAT
 501 GCTGCTGCAC AGCAAGCTCT TCACCGATGC CTCGTCCCGC AGCATCGGGG
 551 CCCTCAACAA GATCAACTTC AACACCCGCT TTGTCATGAA GACGCTCATG
 601 ACCATCTGCC CTGGCACTGT GCTGCTCGTG TTCAGCATCT CTCTGTGGAT
 651 CATTGCTGCC TGGACCGTCC GTGTCTGTGA AAGGTACCAT GACCAGCAGG
 701 ACGTAACTAG TAACTTTCTG GGTGCCATGT GGCTCATCTC CATCACATTC
 751 CTTTCCATTG GTTATGGGGA CATGGTGCCC CACACATACT GTGGGAAAGG
 801 TGTCTGTCTC CTCACTGGCA TCATGGGTGC AGGCTGCACT GCCCTTGTGG
 851 TGGCCGTGGT GGCCCGAAAG CTGGAACTCA CCAAAGCGGA GAAGCACGTT
 901 CATAACTTCA TGATGGACAC TCAGCTCACC AAGCGGATCA AGAATGCTGC
 951 AGCCAATGTC CTTCGGGAAA CATGGTTAAT CTATAAACAC ACAAAGCTGC
1001 TAAAGAAGAT TGACCATGCC AAAGTGAGGA AACACCAGAG GAAGTTCCTC
1051 CAAGCTATCC ACCAGTTGAG GAGCGTCAAG ATGGAACAGA GGAAGCTGAG
1101 TGACCAAGCC AACACTCTGG TGGACCTTTC CAAGATGCAG AATGTCATGT
1151 ATGACTTAAT CACAGAACTC AATGACCGGA GCGAAGACCT GGAGAAGCAG
1201 ATTGGCAGCC TGGAGTCGAA GCTGGAGCAT CTCACCGCCA GCTTCAACTC
1251 CCTGCCGCTG CTCATCGCCG ACACCCTGCG CCAGCAGCAG CAGCAGCTCC
1301 TGTCTGCCAT CATCGAGGCC CGGGGTGTCA GCGTGGCAGT GGGCACCACC
1351 CACACCCCAA TCTCCGATAG CCCCATTGGG GTCAGCTCCA CCTCCTTCCC
1401 GACCCCGTAC ACAAGTTCAA GCAGTTGCTA AATAAATCTC CCCACTCCAG
1451 AAGCATTACC CATAGGTCTT AAGATGCAAA TCAACTCTCT CCTGGTCGCT
1501 TTGCCATCAA GAAACATTCA GACCAGGGAA CGGAAAGAAG AGAGACCGAG
1551 CTAATTAACT AACTCATGTT CATTCAGCGT GCTTGGTCCG ACATGCCTTG
1601 AAACCAGAAA TCTAATCTCT GTTTAGGTGC CTCTACTTGG GAGCGGGAAG
1651 AGGAGATGAC AGGAAGCGAC GCCTCTGGCA GGGCCCTTGC TGCAGAGTTG
1701 GTGGAGAACA GAAATCCACG CTCAATCTCA GGTCTTCACG CGGGGGGTGG
1751 GGGTCAGATG CACTGAAGTA GCCAACAGCG AAGCCAGTCC AGAAGAGGGG
1801 TCCGCTGGGA GGGAGGGTTG TGTCAGGCTT GGGGGATGGG CTCTTCGCCA
1851 TGGGGGTCTT TGAACACACC TCTCTCCTTT CCTTTTGTCT ACGGAAGCCT
1901 CTGGGTGACA AAAGTAAAAG AGAGCTGCCC ACAACTTGCC AAAACAGATA
1951 TACTCGAATC AGACTG
```

# Figure 5 : D) SEQ ID NO: 8, nucleotide sequence of human KCNN3 sv4 cDNA

**Length: 1658 bp**

```
   1 GAGGACTCAG AATAAACCTG CTGCTGCTTC GTAAACATCT CCTGATAAAA
  51 ATGGCTACAG GTGTTACCTG GGCAGAGCAG CTGGGCGCCG CCTTGGAATC
 101 AGCTGGGGGA GCACCTCTCC TTGGGACGGG ATGACAGGGC TTCCCAGGCG
 151 GTCCCCACAA GCCCGCGCCC CAGCTCAGCC CCAGTTCTCC CCTCCCACCT
 201 CAACTCCTCC TTGGGATAAA TAAAGATGAG TGTGTGTGTG AGTGCGCGCC
 251 CGGATGGAGA ACAGCAGGCA CTGGCTTTAG CGGGGAGCTG GCCCCACTGC
 301 TCCAGCCTCT CAGTCCAGCC CCAAGACGGA GGAGGGGGTT TCCCTCCCAG
 351 AGGGAGTGGA GATGGAGGAA GGACTCCATG TTTTCGTTGG CCCTGAAATG
 401 CCTTATCAGT CTGTCCACCA TCATCCTTTT GGGCTTGATC ATCGCCTACC
 451 ACACACGTGA AGTCCAGCTC TTCGTGATCG ACAATGGCGC GGATGACTGG
 501 CGGATAGCCA TGACCTACGA GCGCATCCTG TACATCAGCC TGGAGATGCT
 551 GGTGTGCGCC ATCCACCCCA TTCCTGGCGA GTACAAGTTC TTCTGGACGG
 601 CACGCCTGGC CTTCTCCTAC ACACCCTCCC GGGCGGAGGC CGATGTGGAC
 651 ATCATCCTGT CTATCCCCAT GTTCCTGCGC CTGTACCTGA TCGCCCGAGT
 701 CATGCTGCTG CACAGCAAGC TCTTCACCGA TGCCTCGTCC CGCAGCATCG
 751 GGGCCCTCAA CAAGATCAAC TTCAACACCC GCTTTGTCAT GAAGACGCTC
 801 ATGACCATCT GCCCTGGCAC TGTGCTGCTC GTGTTCAGCA TCTCTCTGTG
 851 GATCATTGCT GCCTGGACCG TCCGTGTCTG TGAAAGGTAC CATGACCAGC
 901 AGGACGTAAC TAGTAACTTT CTGGGTGCCA TGTGGCTCAT CTCCATCACA
 951 TTCCTTTCCA TTGGTTATGG GGACATGGTG CCCCACACAT ACTGTGGGAA
1001 AGGTGTCTGT CTCCTCACTG GCATCATGGG TGCAGGCTGC ACTGCCCTTG
1051 TGGTGGCCGT GGTGGCCCGA AAGCTGGAAC TCACCAAAGC GGAGAAGCAC
1101 GTTCATAACT TCATGATGGA CACTCAGCTC ACCAAGCGGA TCAAGAATGC
1151 TGCAGCCAAT GTCCTTCGGG AAACATGGTT AATCTATAAA CACACAAAGC
1201 TGCTAAAGAA GATTGACCAT GCCAAAGTGA GGAAACACCA GAGGAAGTTC
1251 CTCCAAGCTA TCCACCAGTT GAGGAGCGTC AAGATGGAAC AGAGGAAGCT
1301 GAGTGACCAA GCCAACACTC TGGTGGACCT TTCCAAGATG CAGAATGTCA
1351 TGTATGACTT AATCACAGAA CTCAATGACC GGAGCGAAGA CCTGGAGAAG
1401 CAGATTGGCA GCCTGGAGTC GAAGCTGGAG CATCTCACCG CCAGCTTCAA
1451 CTCCCTGCCG CTGCTCATCG CCGACACCCT GCGCCAGCAG CAGCAGCAGC
1501 TCCTGTCTGC CATCATCGAG GCCCGGGGTG TCAGCGTGGC AGTGGGCACC
1551 ACCCACACCC CAATCTCCGA TAGCCCCATT GGGGTCAGCT CCACCTCCTT
1601 CCCGACCCCG TACACAAGTT CAAGCAGTTG CTAAATAAAT CTCCCCACTC
1651 CAGAAGCA
```

# Figure 6 : A) SEQ ID NO: 9, coding nucleotide sequence of human KCNN3 sv1 cDNA

**Length: 2211 bp**

```
   1  ATGGACACTT CTGGGCACTT CCATGACTCG GGGGTGGGGG ACTTGGATGA
  51  AGACCCCAAG TGCCCCTGTC CATCCTCTGG GGATGAGCAG CAGCAGCAGC
 101  AGCAGCAGCA ACAGCAGCAG CAGCCACCAC CGCCAGCGCC ACCAGCAGCC
 151  CCCCAGCAGC CCCTGGGACC CTCGCTGCAG CCTCAGCCTC CGCAGCTTCA
 201  GCAGCAGCAG CAGCAGCAGC AGCAGCAGCA GCAGCAGCAG CAGCAGCAGC
 251  AGCAGCCACC GCATCCCCTG TCTCAGCTCG CCCAACTCCA GAGCCAGCCC
 301  GTCCACCCTG GCCTGCTGCA CTCCTCTCCC ACCGCTTTCA GGGCCCCCCC
 351  TTCGTCCAAC TCCACCGCCA TCCTCCACCC TTCCTCCAGG CAAGGCAGCC
 401  AGCTCAATCT CAATGACCAC TTGCTTGGCC ACTCTCCAAG TTCCACAGCT
 451  ACAAGTGGGC CTGGCGGAGG CAGCCGGCAC CGACAGGCCA GCCCCCTGGT
 501  GCACCGGCGG GACAGCAACC CCTTCACGGA GATCGCCATG AGCTCCTGCA
 551  AGTATAGCGG TGGGGTCATG AAGCCCCTCA GCCGCCTCAG CGCCTCCCGG
 601  AGGAACCTCA TCGAGGCCGA GACTGAGGGC CAACCCCTCC AGCTTTTCAG
 651  CCCTAGCAAC CCCCCGGAGA TCGTCATCTC CTCCCGGGAG GACAACCATG
 701  CCCACCAGAC CCTGCTCCAT CACCCTAATG CCACCCACAA CCACCAGCAT
 751  GCCGGCACCA CCGCCAGCAG CACCACCTTC CCCAAAGCCA ACAAGCGGAA
 801  AAACCAAAAC ATTGGCTATA AGCTGGGACA CAGGAGGGCC CTGTTTGAAA
 851  AGAGAAAGCG ACTGAGTGAC TATGCTCTGA TTTTTGGGAT GTTTGGAATT
 901  GTTGTTATGG TGATAGAGAC CGAGCTCTCT TGGGGTTTGT ACTCAAAGGA
 951  CTCCATGTTT TCGTTGGCCC TGAAATGCCT TATCAGTCTG TCCACCATCA
1001  TCCTTTTGGG CTTGATCATC GCCTACCACA CACGTGAAGT CCAGCTCTTC
1051  GTGATCGACA ACGGCGCGGA TGACTGGCGG ATAGCCATGA CCTACGAGCG
1101  CATCCTGTAC ATCAGCCTGG AGATGCTGGT GTGCGCCATC CACCCCATTC
1151  CTGGCGAGTA CAAGTTCTTC TGGACGGCAC GCCTGGCCTT CTCCTACACA
1201  CCCTCCCGGG CGGAGGCCGA TGTGGACATC ATCCTGTCTA TCCCCATGTT
1251  CCTGCGCCTG TACCTGATCG CCCGAGTCAT GCTGCTGCAC AGCAAGCTCT
1301  TCACCGATGC CTCGTCCCGC AGCATCGGGG CCCTCAACAA GATCAACTTC
1351  AACACCCGCT TTGTCATGAA GACGCTCATG ACCATCTGCC CTGGCACTGT
1401  GCTGCTCGTG TTCAGCATCT CTCTGTGGAT CATTGCTGCC TGGACCGTCC
1451  GTGTCTGTGA AAGGTACCAT GACCAGCAGG ACGTAACTAG TAACTTTCTG
1501  GGTGCCATGT GGCTCATCTC CATCACATTC CTTTCCATTG GTTATGGGGA
1551  CATGGTGCCC CACACATACT GTGGGAAAGG TGTCTGTCTC CTCACTGGCA
1601  TCATGGGTGC AGGCTGCACT GCCCTTGTGG TGGCCGTGGT GGCCCGAAAG
1651  CTGGAACTCA CCAAAGCGGA GAAGCACGTT CATAACTTCA TGATGGACAC
1701  TCAGCTCACC AAGCGGATCA AGAATGCTGC AGCCAATGTC CTTCGGGAAA
1751  CATGGTTAAT CTATAAACAC ACAAAGCTGC TAAAGAAGAT TGACCATGCC
1801  AAAGTGAGGA AACACCAGAG GAAGTTCCTC CAAGCTATCC ACCAGTTGAG
1851  GAGCGTCAAG ATGGAACAGA GGAAGCTGAG TGACCAAGCC AACACTCTGG
1901  TGGACCTTTC CAAGATGCAG AATGTCATGT ATGACTTAAT CACAGAACTC
1951  AATGACCGGA GCGAAGACCT GGAGAAGCAG ATTGGCAGCC TGGAGTCGAA
2001  GCTGGAGCAT CTCACCGCCA GCTTCAACTC CCTGCCGCTG CTCATCGCCG
2051  ACACCCTGCG CCAGCAGCAG CAGCAGCTCC TGTCTGCCAT CATCGAGGCC
2101  CGGGGTGTCA GCGTGGCAGT GGGCACCACC CACACCCCAA TCTCCGATAG
2151  CCCCATTGGG GTCAGCTCCA CCTCCTTCCC GACCCCGTAC ACAAGTTCAA
```

2201 GCAGTTGCTA A

# Figure 6 : B) SEQ ID NO: 10, coding nucleotide sequence of human KCNN3 sv2 cDNA

**Length: 2196 bp**

```
   1 ATGGACACTT CTGGGCACTT CCATGACTCG GGGGTGGGGG ACTTGGATGA
  51 AGACCCCAAG TGCCCCTGTC CATCCTCTGG GGATGAGCAG CAGCAGCAGC
 101 AGCAGCAGCA ACAGCAGCAG CAGCCACCAC CGCCAGCGCC ACCAGCAGCC
 151 CCCCAGCAGC CCCTGGGACC CTCGCTGCAG CCTCAGCCTC CGCAGCTTCA
 201 GCAGCAGCAG CAGCAGCAGC AGCAGCAGCA GCAGCAGCAG CCACCGCATC
 251 CCCTGTCTCA GCTCGCCCAA CTCCAGAGCC AGCCCGTCCA CCCTGGCCTG
 301 CTGCACTCCT CTCCCACCGC TTTCAGGGCC CCCCCTTCGT CCAACTCCAC
 351 CGCCATCCTC CACCCTTCCT CCAGGCAAGG CAGCCAGCTC AATCTCAATG
 401 ACCACTTGCT TGGCCACTCT CCAAGTTCCA CAGCTACAAG TGGGCCTGGC
 451 GGAGGCAGCC GGCACCGACA GGCCAGCCCC CTGGTGCACC GGCGGGACAG
 501 CAACCCCTTC ACGGAGATCG CCATGAGCTC CTGCAAGTAT AGCGGTGGGG
 551 TCATGAAGCC CCTCAGCCGC CTCAGCGCCT CCCGGAGGAA CCTCATCGAG
 601 GCCGAGACTG AGGGCCAACC CCTCCAGCTT TTCAGCCCTA GCAACCCCCC
 651 GGAGATCGTC ATCTCCTCCC GGGAGGACAA CCATGCCCAC CAGACCCTGC
 701 TCCATCACCC TAATGCCACC CACAACCACC AGCATGCCGG CACCACCGCC
 751 AGCAGCACCA CCTTCCCCAA AGCCAACAAG CGGAAAAACC AAAACATTGG
 801 CTATAAGCTG GGACACAGGA GGGCCCTGTT TGAAAAGAGA AAGCGACTGA
 851 GTGACTATGC TCTGATTTTT GGGATGTTTG GAATTGTTGT TATGGTGATA
 901 GAGACCGAGC TCTCTTGGGG TTTGTACTCA AAGGACTCCA TGTTTTCGTT
 951 GGCCCTGAAA TGCCTTATCA GTCTGTCCAC CATCATCCTT TTGGGCTTGA
1001 TCATCGCCTA CCACACACGT GAAGTCCAGC TCTTCGTGAT CGACAATGGC
1051 GCGGATGACT GGCGGATAGC CATGACCTAC GAGCGCATCC TGTACATCAG
1101 CCTGGAGATG CTGGTGTGCG CCATCCACCC CATTCCTGGC GAGTACAAGT
1151 TCTTCTGGAC GGCACGCCTG GCCTTCTCCT ACACACCCTC CCGGGCGGAG
1201 GCCGATGTGG ACATCATCCT GTCTATCCCC ATGTTCCTGC GCCTGTACCT
1251 GATCGCCCGA GTCATGCTGC TGCACAGCAA GCTCTTCACC GATGCCTCGT
1301 CCCGCAGCAT CGGGGCCCTC AACAAGATCA ACTTCAACAC CCGCTTTGTC
1351 ATGAAGACGC TCATGACCAT CTGCCCTGGC ACTGTGCTGC TCGTGTTCAG
1401 CATCTCTCTG TGGATCATTG CTGCCTGGAC CGTCCGTGTC TGTGAAAGGT
1451 ACCATGACCA GCAGGACGTA ACTAGTAACT TTCTGGGTGC CATGTGGCTC
1501 ATCTCCATCA CATTCCTTTC CATTGGTTAT GGGGACATGG TGCCCCACAC
1551 ATACTGTGGG AAAGGTGTCT GTCTCCTCAC TGGCATCATG GGTGCAGGCT
1601 GCACTGCCCT TGTGGTGGCC GTGGTGGCCC GAAAGCTGGA ACTCACCAAA
1651 GCGGAGAAGC ACGTTCATAA CTTCATGATG GACACTCAGC TCACCAAGCG
1701 GATCAAGAAT GCTGCAGCCA ATGTCCTTCG GGAAACATGG TTAATCTATA
1751 AACACACAAA GCTGCTAAAG AAGATTGACC ATGCCAAAGT GAGGAAACAC
1801 CAGAGGAAGT TCCTCCAAGC TATCCACCAG TTGAGGAGCG TCAAGATGGA
1851 ACAGAGGAAG CTGAGTGACC AAGCCAACAC TCTGGTGGAC CTTTCCAAGA
1901 TGCAGAATGT CATGTATGAC TTAATCACAG AACTCAATGA CCGGAGCGAA
1951 GACCTGGAGA AGCAGATTGG CAGCCTGGAG TCGAAGCTGG AGCATCTCAC
2001 CGCCAGCTTC AACTCCCTGC CGCTGCTCAT CGCCGACACC CTGCGCCAGC
2051 AGCAGCAGCA GCTCCTGTCT GCCATCATCG AGGCCCGGGG TGTCAGCGTG
2101 GCAGTGGGCA CCACCCACAC CCCAATCTCC GATAGCCCCA TTGGGGTCAG
2151 CTCCACCTCC TTCCCGACCC CGTACACAAG TTCAAGCAGT TGCTAA
```

# Figure 6 : C) SEQ ID NO: 11, coding nucleotide sequence of human KCNN3 sv3 cDNA

**Length: 1281 bp**

```
   1  ATGGAGAGAC CTATAAAGGA CTCCATGTTT TCGTTGGCCC TGAAATGCCT
  51  TATCAGTCTG TCCACCATCA TCCTTTTGGG CTTGATCATC GCCTACCACA
 101  CACGTGAAGT CCAGCTCTTC GTGATCGACA ATGGCGCGGA TGACTGGCGG
 151  ATAGCCATGA CCTACGAGCG CATCCTGTAC ATCAGCCTGG AGATGCTGGT
 201  GTGCGCCATC CACCCCATTC CTGGCGAGTA CAAGTTCTTC TGGACGGCAC
 251  GCCTGGCCTT CTCCTACACA CCCTCCCGGG CGGAGGCCGA TGTGGACATC
 301  ATCCTGTCTA TCCCCATGTT CCTGCGCCTG TACCTGATCG CCCGAGTCAT
 351  GCTGCTGCAC AGCAAGCTCT TCACCGATGC CTCGTCCCGC AGCATCGGGG
 401  CCCTCAACAA GATCAACTTC AACACCCGCT TTGTCATGAA GACGCTCATG
 451  ACCATCTGCC CTGGCACTGT GCTGCTCGTG TTCAGCATCT CTCTGTGGAT
 501  CATTGCTGCC TGGACCGTCC GTGTCTGTGA AAGGTACCAT GACCAGCAGG
 551  ACGTAACTAG TAACTTTCTG GGTGCCATGT GGCTCATCTC CATCACATTC
 601  CTTTCCATTG GTTATGGGGA CATGGTGCCC CACACATACT GTGGGAAAGG
 651  TGTCTGTCTC CTCACTGGCA TCATGGGTGC AGGCTGCACT GCCCTTGTGG
 701  TGGCCGTGGT GGCCCGAAAG CTGGAACTCA CCAAAGCGGA GAAGCACGTT
 751  CATAACTTCA TGATGGACAC TCAGCTCACC AAGCGGATCA AGAATGCTGC
 801  AGCCAATGTC CTTCGGGAAA CATGGTTAAT CTATAAACAC ACAAAGCTGC
 851  TAAAGAAGAT TGACCATGCC AAAGTGAGGA AACACCAGAG GAAGTTCCTC
 901  CAAGCTATCC ACCAGTTGAG GAGCGTCAAG ATGGAACAGA GGAAGCTGAG
 951  TGACCAAGCC AACACTCTGG TGGACCTTTC CAAGATGCAG AATGTCATGT
1001  ATGACTTAAT CACAGAACTC AATGACCGGA GCGAAGACCT GGAGAAGCAG
1051  ATTGGCAGCC TGGAGTCGAA GCTGGAGCAT CTCACCGCCA GCTTCAACTC
1101  CCTGCCGCTG CTCATCGCCG ACACCCTGCG CCAGCAGCAG CAGCAGCTCC
1151  TGTCTGCCAT CATCGAGGCC CGGGGTGTCA GCGTGGCAGT GGGCACCACC
1201  CACACCCCAA TCTCCGATAG CCCCATTGGG GTCAGCTCCA CCTCCTTCCC
1251  GACCCCGTAC ACAAGTTCAA GCAGTTGCTA A
```

# Figure 6 : D) SEQ ID NO: 12, coding nucleotide sequence of human KCNN3 sv4 cDNA

**Length: 1257 bp**

```
   1  ATGTTTTCGT TGGCCCTGAA ATGCCTTATC AGTCTGTCCA CCATCATCCT
  51  TTTGGGCTTG ATCATCGCCT ACCACACACG TGAAGTCCAG CTCTTCGTGA
 101  TCGACAATGG CGCGGATGAC TGGCGGATAG CCATGACCTA CGAGCGCATC
 151  CTGTACATCA GCCTGGAGAT GCTGGTGTGC GCCATCCACC CCATTCCTGG
 201  CGAGTACAAG TTCTTCTGGA CGGCACGCCT GGCCTTCTCC TACACACCCT
 251  CCCGGGCGGA GGCCGATGTG GACATCATCC TGTCTATCCC CATGTTCCTG
 301  CGCCTGTACC TGATCGCCCG AGTCATGCTG CTGCACAGCA AGCTCTTCAC
 351  CGATGCCTCG TCCCGCAGCA TCGGGGCCCT CAACAAGATC AACTTCAACA
 401  CCCGCTTTGT CATGAAGACG CTCATGACCA TCTGCCCTGG CACTGTGCTG
 451  CTCGTGTTCA GCATCTCTCT GTGGATCATT GCTGCCTGGA CCGTCCGTGT
 501  CTGTGAAAGG TACCATGACC AGCAGGACGT AACTAGTAAC TTTCTGGGTG
 551  CCATGTGGCT CATCTCCATC ACATTCCTTT CCATTGGTTA TGGGGACATG
 601  GTGCCCCACA CATACTGTGG GAAAGGTGTC TGTCTCCTCA CTGGCATCAT
 651  GGGTGCAGGC TGCACTGCCC TTGTGGTGGC CGTGGTGGCC CGAAAGCTGG
 701  AACTCACCAA AGCGGAGAAG CACGTTCATA ACTTCATGAT GGACACTCAG
 751  CTCACCAAGC GGATCAAGAA TGCTGCAGCC AATGTCCTTC GGGAAACATG
 801  GTTAATCTAT AAACACACAA AGCTGCTAAA GAAGATTGAC CATGCCAAAG
 851  TGAGGAAACA CCAGAGGAAG TTCCTCCAAG CTATCCACCA GTTGAGGAGC
 901  GTCAAGATGG AACAGAGGAA GCTGAGTGAC CAAGCCAACA CTCTGGTGGA
 951  CCTTTCCAAG ATGCAGAATG TCATGTATGA CTTAATCACA GAACTCAATG
1001  ACCGGAGCGA AGACCTGGAG AAGCAGATTG GCAGCCTGGA GTCGAAGCTG
1051  GAGCATCTCA CCGCCAGCTT CAACTCCCTG CCGCTGCTCA TCGCCGACAC
1101  CCTGCGCCAG CAGCAGCAGC AGCTCCTGTC TGCCATCATC GAGGCCCGGG
1151  GTGTCAGCGT GGCAGTGGGC ACCACCCACA CCCCAATCTC CGATAGCCCC
1201  ATTGGGGTCA GCTCCACCTC CTTCCCGACC CCGTACACAA GTTCAAGCAG
1251  TTGCTAA
```

# Figure 7: Alignment of KCNN3 RT-PCR primers: SEQ ID NO: 13 and SEQ ID NO: 14 with human KCNN3 sv1 cDNA, SEQ ID NO: 5

```
SEQ ID NO:13 (PRIMER A)      1 GGTGGAGAACAGAAATCCACG 21
                                 |||||||||||||||||||||
SEQ ID NO:5:              2813 GGTGGAGAACAGAAATCCACG 2833


SEQ ID NO:14 (PRIMER B)     21 AACCAGTCCAGAAGAGGGGTC 1
                                 |||||||||||||||||||||
SEQ ID NO:5               2895 AACCAGTCCAGAAGAGGGGTC 2915
```

# Figure 8: Schematic alignment of SEQ ID NO: 5, SEQ ID NO: 9, and RT-PCR primer sequences of KCNN3

```
SEQ ID NO:14                                                 +>          2813-2833
SEQ ID NO:13                                                 <+          2915-2895
SEQ ID NO:9        +------------------------------------->                334-2544
SEQ ID NO:5      +----------------------------------------------->          1-3095

   |------|------|------|------|------|------|------|------|
       0     400    800   1200   1600   2000   2400   2800   3200

Legend:
+      5'-end of sequence
>      3'-end of sequence
---    sequence identical to KCNN3 sv 1 mRNA
```

Figure 9:
Co-deposition of KCNN3 protein with amyloid plaques is detected in the cerebral cortex of AD patients, but is absent from controls

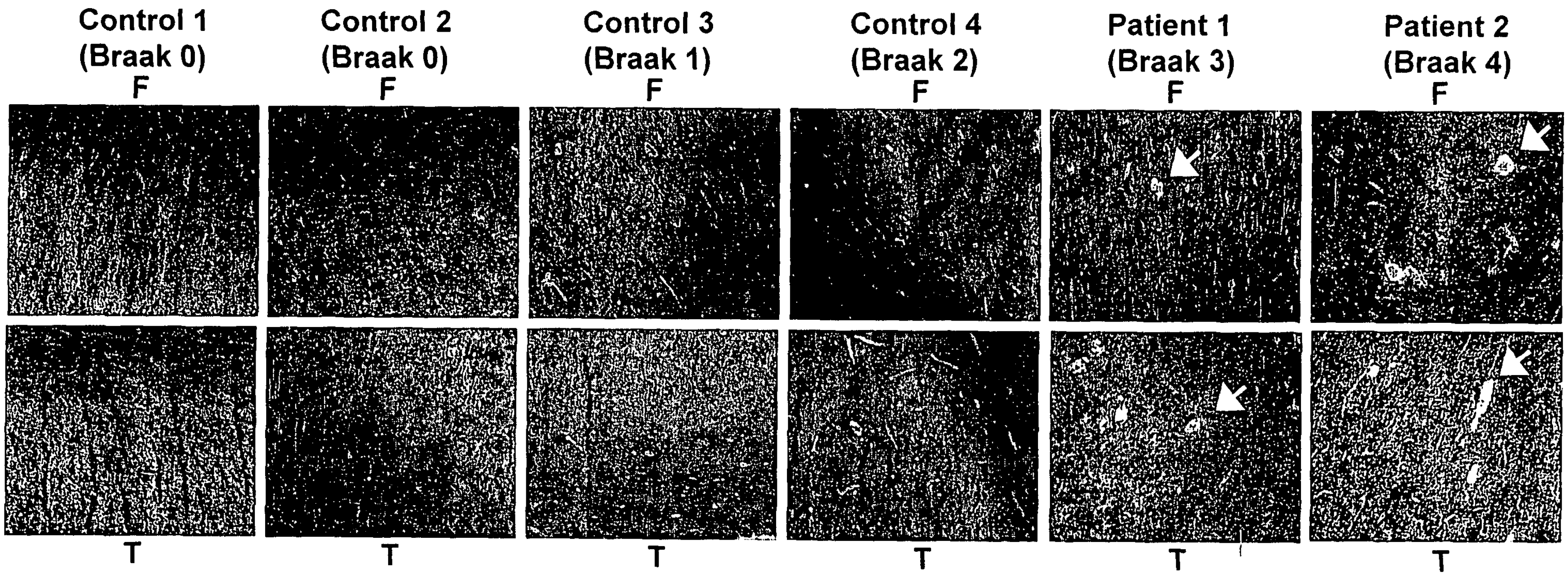

# Figure 10:
# Co-deposition of KCNN3 protein with amyloid plaques in the cerebral cortex of AD patients, but not in controls

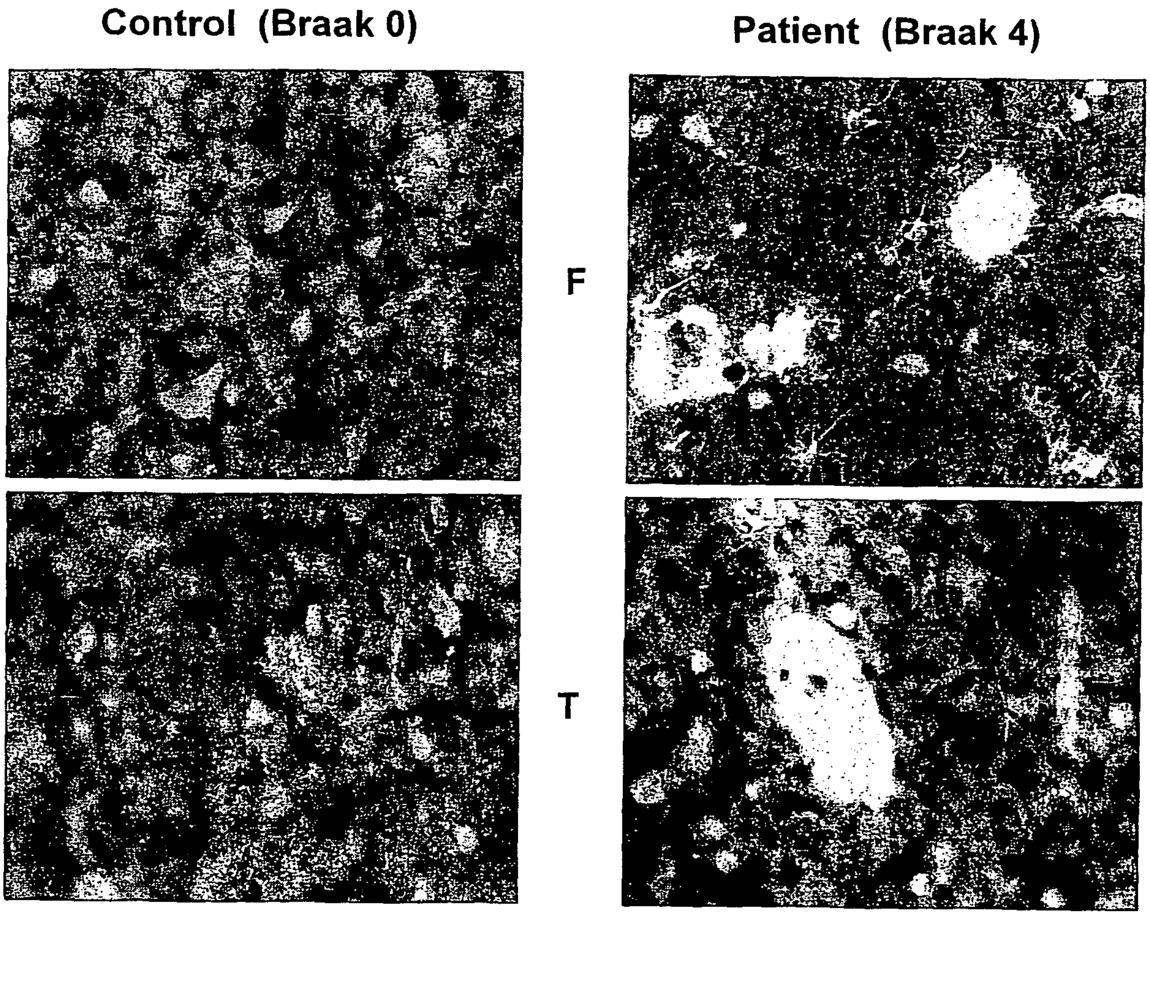

Figure 11:
KCNN3 protein is found in astrocytes in the cerebral cortex of AD patients, but absent from astrocytes of controls

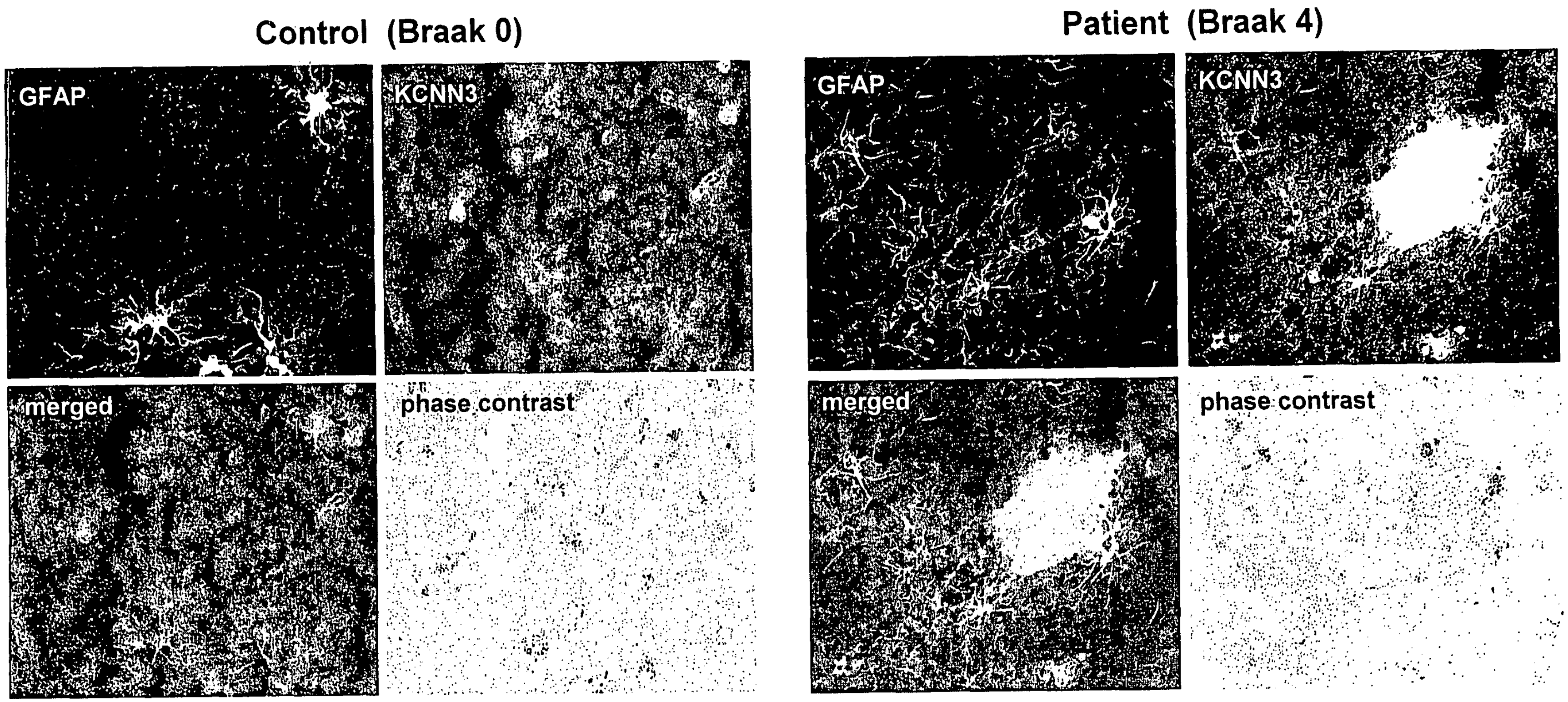

Figure 12: Westernblot analysis of KCNN3 expression in CHO cells stably expressing KCNN3

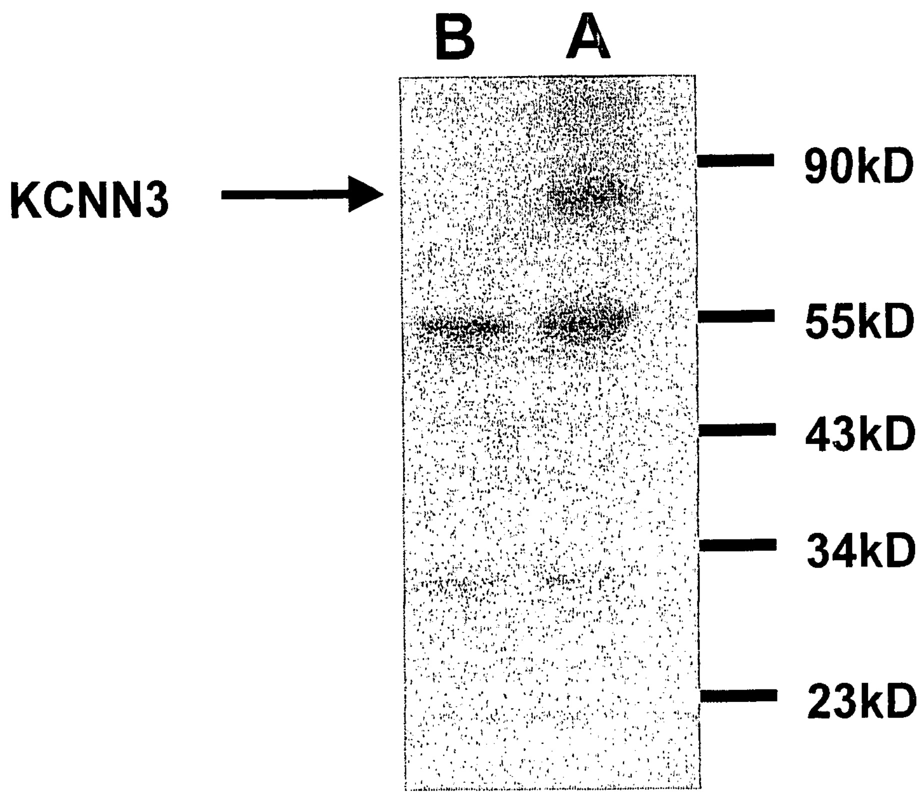


A: CHO–KCNN3-myc
B: CHO wild type (control)

**Figure 13: Immunofluorescence of KCNN3 expression in CHO cells stably expressing KCNN3**

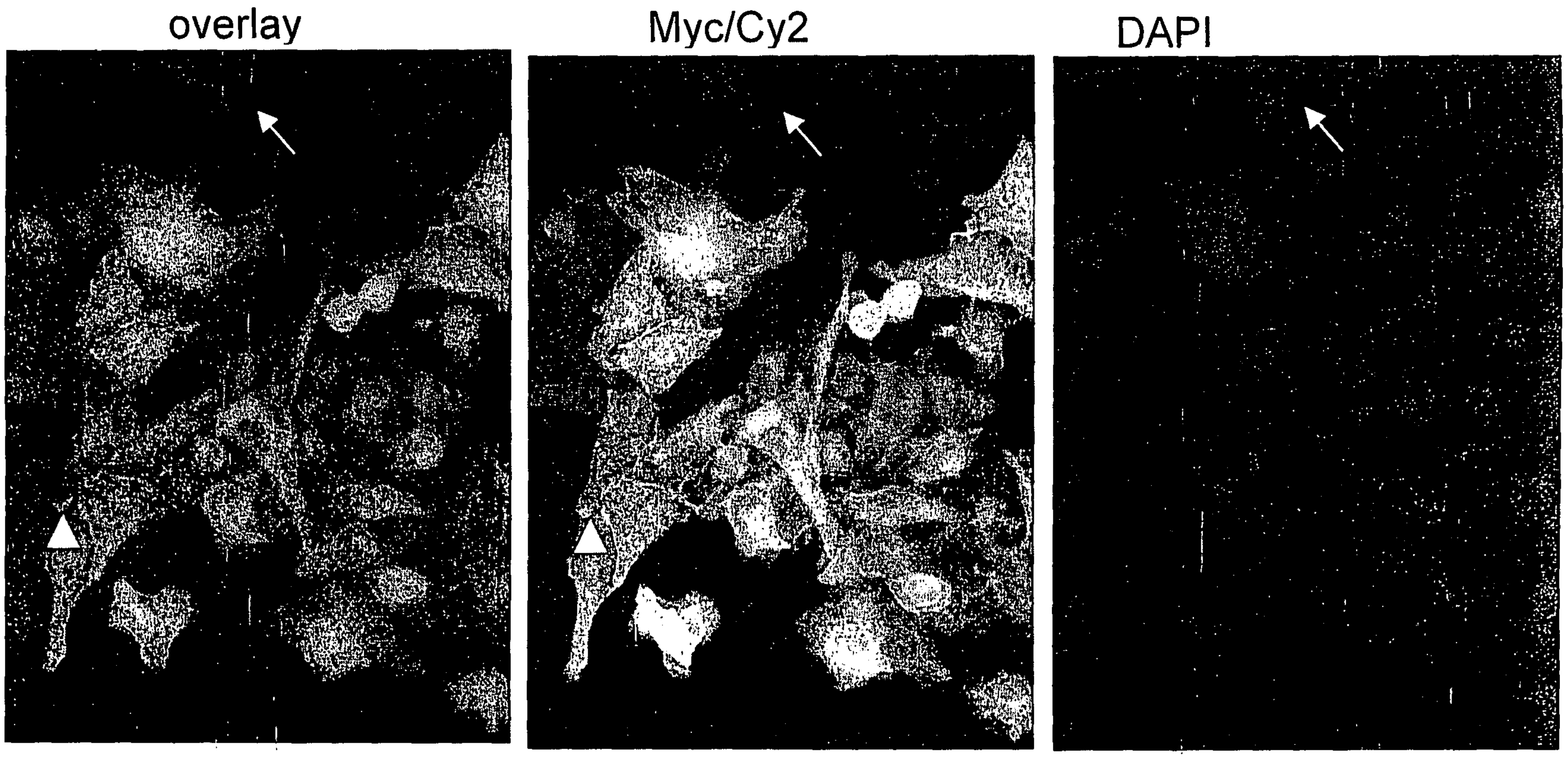


Arrow indicates nucleus of a cell without KCNN3-expression as a negative control.
Arrowhead indicates expression at the plasmamembrane.

# Figure 14: Assay development for screening of KCNN3 ion channel modulators in cellular systems

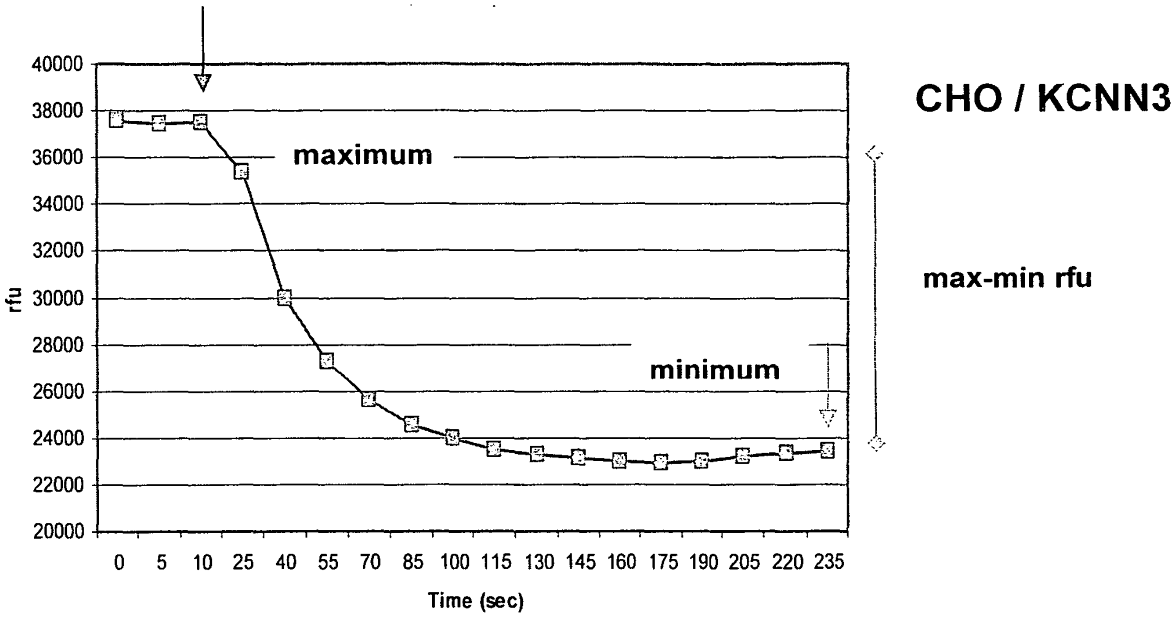


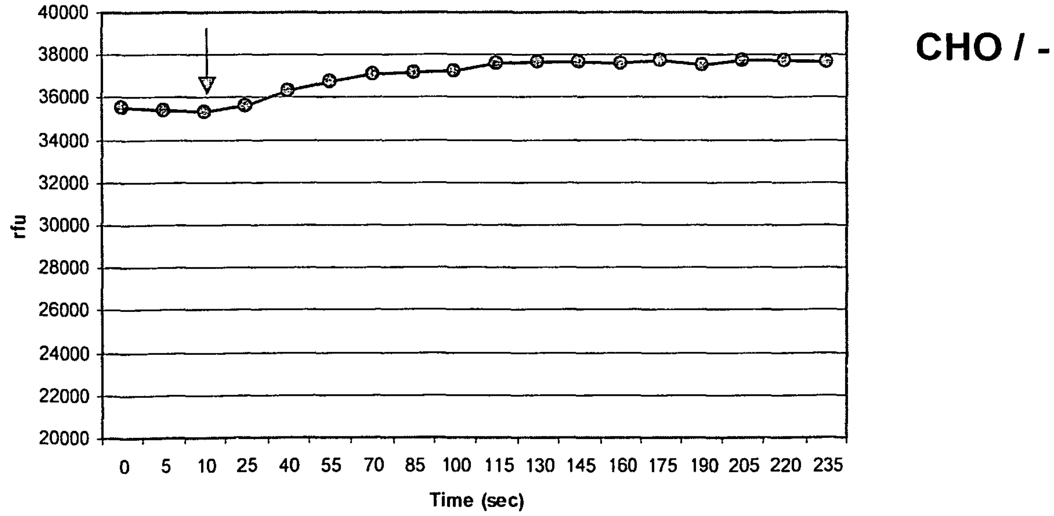

# Figure 15: $IC_{50}$ analysis of the KCNN3 antagonists apamin and trifluoperatine

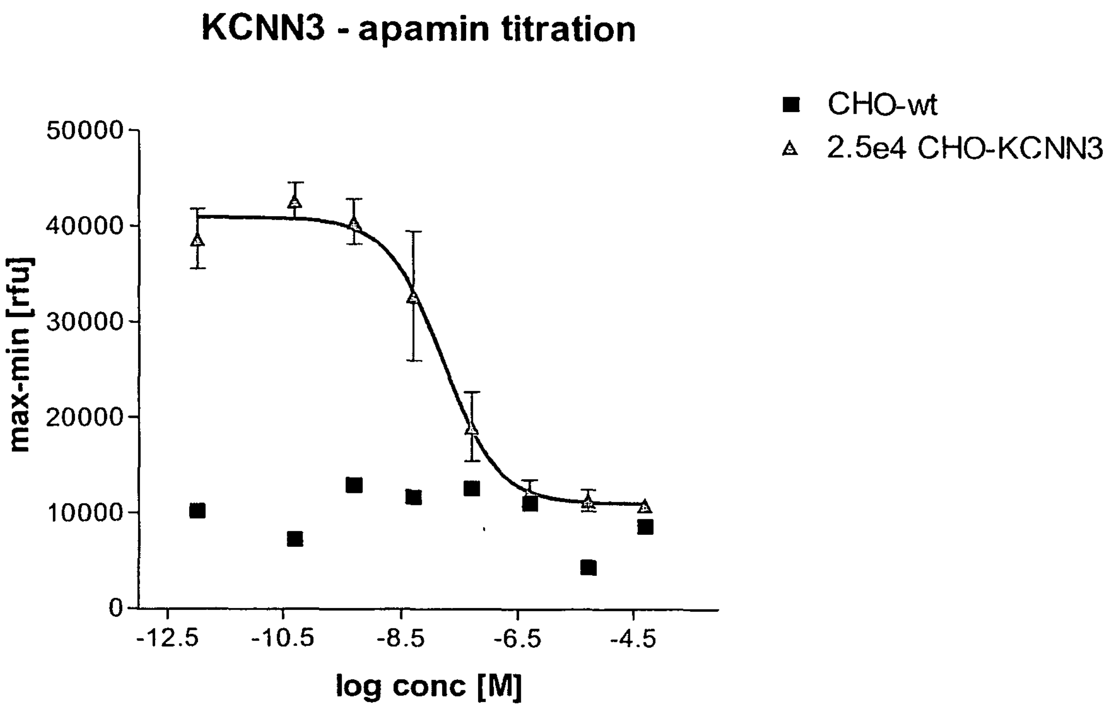


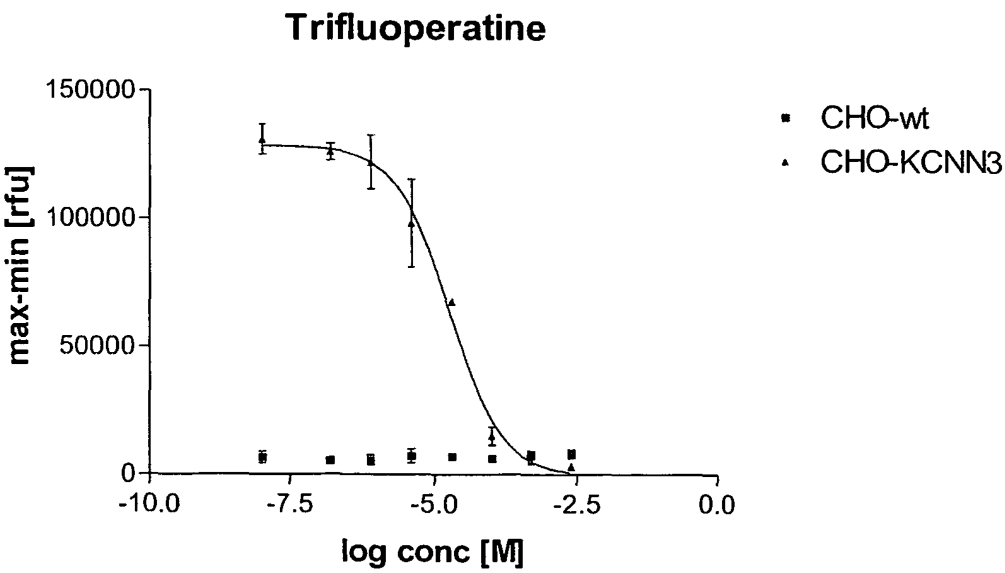

# Figure 16: Z'-value assessment of the cellular KCNN3 screening assay

| Experiment 1 | |
|---|---|
| MW high [rfu] | 124086 |
| MW low [rfu] | 15912 |
| Stabw high [rfu] | 12615 |
| Stabw low [rfu] | 3636 |
| CV high | 10.2 |
| CV low | 22.9 |
| **dyn.range [RFU]** | **108174** |
| **Z'** | **0.55** |

| Experiment 2 | |
|---|---|
| MW high [rfu] | 127968 |
| MW low [rfu] | 16827 |
| Stabw high [rfu] | 11448 |
| Stabw low [rfu] | 3571 |
| CV high | 8.9 |
| CV low | 21.2 |
| **dyn.range [rfu]** | **111141** |
| **Z'** | **0.59** |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004020665 A
- WO 0214543 A
- WO 0052451 A
- WO 0201226 A
- WO 9613744 A
- WO 9816814 A
- WO 9823942 A
- WO 9917086 A
- WO 9934195 A
- WO 0066985 A
- WO 0159436 A
- WO 0159416 A
- US 6150173 A


### Non-patent literature cited in the description


- **VICKERS et al.** *Progress in Neurobiology,* 2000, vol. 60, 139-165
- **WALSH ; SELKOE.** *Neuron,* 2004, vol. 44, 181-193
- **SELKOE ; KOPAN.** *Annu Rev Neurosci,* 2003, vol. 26, 565-597
- **LING et al.** *Int J Biochem Cell Biol,* 2003, vol. 35, 1505-1535
- **BRAAK ; BRAAK.** *J Neural Transm,* 1998, vol. 53, 127-140
- **JOHNSON ; JENKINS.** *J Alzheimers Dis,* 1996, vol. 1, 38-58
- **JOHNSON ; HARTIGAN.** *J Alzheimers Dis,* 1999, vol. 1, 329-351
- **SCHMITT et al.** *Neurology,* 2000, vol. 55, 370-376
- **TERRY et al.** Annals of Neurology. 1981, vol. 10, 184-92
- **STRITTMATTER et al.** *Proc Natl Acad Sci USA,* 1993, vol. 90, 1977-81
- **ROSES.** *Ann NY Acad Sci,* 1998, vol. 855, 738-43
- **KOHLER et al.** *Science,* 1996, vol. 273, 1709-1714
- **ISHII et al.** *Proc. Natl. Acad. Sci. USA,* 1997, vol. 94, 11651
- **CHANDY et al.** *Molec. Psychiat.,* 1998, vol. 3, 32-37
- **FREBOURG et al.** *Am. J. Hum. Genet.,* 1998, vol. 63, A326
- **AUSTIN et al.** *Molec. Psychiat.,* 1999, vol. 4, 261-266
- **WITTEKINDT et al.** *Neurogenetics,* 1998, vol. 1, 259-265
- **SUN et al.** *J. Hum. Genet.,* 2001, vol. 46, 463-470
- **BLANK et al.** *Nature Neurosci.,* 2003, vol. 6, 911-912
- **TACCONI et al.** *Neuroscience,* 2001, vol. 102, 209-215
- **SAILER et al.** *Mol. Cell. Neurosci.,* 2004, vol. 26, 458-469
- **KOLSKI-ANDREACO et al.** *J. Biol. Chem.,* 2004, vol. 279, 6893-6904
- **BOND et al.** *Science,* 2000, vol. 289, 1942-1946
- **TAYLOR et al.** *Circ. Res.,* 2003, vol. 93, 124-131
- **PEDARZANI et al.** *J. Biol. Chem.,* 2001, vol. 276, 9762-9769
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **DEVEREUX et al.** *Nucleic Acids Res.,* 1987, vol. 12, 387
- **PEARSON ; LIPMAN.** *Proc. Natl. Acad. Sci. USA,* 1988, vol. 85, 2444-2448
- **WILBUR ; LIPMAN.** *SIAM J. Appl. Math.,* 1984, vol. 44, 557-567
- **NEEDLEMAN ; WUNSCH.** *J. Mol. Biol.,* 1970, vol. 48, 443-453
- **IQBAL ; SWAAB ; WINBLAD ; WISNIEWSKI.** Alzheimer's Disease and Related Disorders (Etiology, Pathogenesis and Therapeutics). Wiley & Sons, 1999
- **SCINTO ; DAFFNER.** Early Diagnosis of Alzheimer's Disease. Humana Press, 2000
- **MAYEUX ; CHRISTEN.** Epidemiology of Alzheimer's Disease: From Gene to Prevention. Springer Press, 1999
- **YOUNKIN ; TANZI ; CHRISTEN.** Presenilins and Alzheimer's Disease. Springer Press, 1998
- **BRAAK ; BRAAK.** *Acta Neuropathology,* 1991, vol. 82, 239-259

- **BANCHER et al.** *Neuroscience Letters,* 1993, vol. 162, 179-182
- **GOLD et al.** *Acta Neuropathol,* 2000, vol. 99, 579-582
- **RÖSSLER et al.** *Acta Neuropathol,* 2002, vol. 103, 363-369
- **GIANNAKOPOULOS et al.** *Neurology,* 2003, vol. 60, 1495-1500
- **BENNETT et al.** *Arch Neurol,* 2004, vol. 61, 378-384
- **METSAARS et al.** *Neurobiol Aging,* 2003, vol. 24, 563-572
- **TERRY et al.** Annals of Neurology. 1981, vol. 10, 184-192
- **SAMBROOK ; RUSSELL.** Molecular Cloning: A Laboratory Manual. Cold Spring Harbor Laboratory Press, 2001
- **SCHENA M.** Microarray Biochip Technology. Eaton Publishing, 2000
- **HARLOW ; LANE.** Antibodies: A Laboratory Manual. Cold Spring Harbor Laboratory Press, 1999
- **EDWARDS R.** Immunodiagnostics: A Practical Approach. Oxford University Press, 1999
- **BEHR.** *Acc Chem Res,* 1993, vol. 26, 274-278
- **MULLIGAN.** *Science,* 1993, vol. 260, 926-931
- **WOLFF.** *Curr Opin Neurobiol,* 1993, vol. 3, 743-748
- **GILLESPIE.** *DN&P,* 1992, vol. 5, 389-395
- **AGRAWAL ; AKHTAR.** *Trends Biotechnol,* 1995, vol. 13, 197-199
- **CROOKE.** *Biotechnology,* 1992, vol. 10, 882-6
- **BARINAGA.** *Science,* 1993, vol. 262, 1512-1514
- **WICKSTROM.** *Trends Biotechnol,* 1992, vol. 10, 281-287
- **HANNON.** *Nature,* 2002, vol. 418, 244-251
- **MC CELLAND ; PARDEE.** Expression Genetics: Accelerated and High-Throughput Methods. Eaton Publishing, 1999
- **LANZA et al.** *Nature Medicine,* 1999, vol. 9, 975-977
- **PETERSON DA.** *Curr. Opin. Pharmacol.,* 2002, vol. 2, 34-42
- **COLMAN A.** *Drug Discovery World,* 2001, vol. 7, 66-71
- **HAMILL et al.** *Pflügers Arch.,* 1981, vol. 391, 85-100
- **VARNUM et al.** *Pro. Natl. Acad. Sci. USA,* 1993, vol. 90, 11528-11532
- **DART et al.** *J. Physiol.,* 1995, vol. 483, 29-39
- **DINESH ; HABLITZ.** *Brain Res.,* 1990, vol. 535, 318-322
- **NETZER et al.** *Curr Opin Drug Discov Devel,* 2003, vol. 4, 462-469
- **CAPECCHI.** *Science,* 1989, vol. 244, 1288-1292
- **HOGAN et al.** Manipulating the Mouse Embryo: A Laboratory Manual. Cold Spring Harbor Laboratory Press, 1994
- **JACKSON ; ABBOTT.** Mouse Genetics and Transgenics: A Practical Approach. 1999
- **SCHWARZ et al.** *Biochemistry,* 1999, vol. 38, 9456-9464
- **KRIVOSHEEV ; USANOV.** *Biochemistry-Moscow,* 1997, vol. 62, 1064-1073
- **HARLOW et al.** Antibodies, A Laboratory Manual. Cold Spring Harbor Laboratory Press, 1988
- **DUBEL ; BREITLING.** Recombinant Antibodies. Wiley-Liss, 1999
- **HARLOW ; LANE.** Using Antibodies: A Laboratory Manual. Cold Spring Harbor Laboratory Press, 1999
- **EDWARDS R.** Immunodiagnostics: A Practical Approach. Oxford University Press, 1999
- **TERSTAPPEN et al.** *Neuropharmacology,* 2001, vol. 40, 772-783